# EUROPEAN PATENT SPECIFICATION

(11) **EP 4 486 220 B1**
(45) Date of publication and mention of the grant of the patent: **05.11.2025**
(21) Application number: 23709399.2
(22) Date of filing: 03.03.2023
(51) Int. Cl.: A61B 17/04, A61F 2/24, A61M 25/00, A61B 17/00, A61B 90/00

(54) **SOFT TISSUE ANCHOR SYSTEM FOR HEART REPAIR**
WEICHGEWEBEANKERSYSTEM ZUR HERZREPARATUR
SYSTÈME D'ANCRAGE DE TISSU MOU POUR RÉPARATION CARDIAQUE

(30) Priority: 03.03.2022 US 202263316062 P; 24.01.2023 US 202363440766 P
(43) Date of publication of application: 08.01.2025
(73) Proprietor: Cardiomech AS, 7030 Trondheim (NO)
(72) Inventor: HIORTH, Nikolai, 0779 Oslo (NO); HIORTH, Hans Emil, 7030 Trondheim (NO); BLIX, John B., Maple Grove, Minnesota 55311 (US)
(74) Representative: Dehns
(86) International application number: PCT/EP2023/055515
(87) International publication number: WO 2023/166218

(56) References cited:
- EP-A1- 1 884 199
- WO-A1-2007/005394
- WO-A1-2010/132310
- WO-A1-2019/014158
- US-A1- 2005 251 208
- US-A1- 2016 157 845

## Description

The present invention relates to a soft tissue anchor system for implantation in soft body tissue to hold a line.

The chordae tendineae are cord-like tendons that connect the papillary muscles to the tricuspid valve and the mitral valve in the heart. The valves consist of leaflets that open and close with the beating of the heart in order to control blood flow and blood pressure within the heart.

Mitral valve disease presents an important challenge to cardiac surgeons and cardiologists. Mitral regurgitation has become the leading pathophysiological condition of the mitral valve in the developed world. One of the most important causes of regurgitation is prolapse of one of the mitral leaflets. The pathological abnormality that requires repair is rupture or other degenerative changes of the chords, leaflet or other related structures. When the chord(s) remain intact, the mitral leaflets open and close synchronously and in a fashion that prevents leakage of the valve. The normal chords can rupture acutely, causing acute decompensation in the form of heart failure. This usually results in an emergency condition requiring rapid intervention. Damage to the chord(s) can also occur more slowly including rupturing or elongation due to degenerative processes, causing the mitral valve to develop leaks or regurgitation.

Surgical repair of the mitral valve has become relatively standardized, using resection of the prolapsed leaflet and/or implantation of new, artificial chordae lines to control leaflet motion. In addition, a mitral ring is frequently placed to shrink the size of the mitral valve annulus. Surgical replacement of ruptured or elongated chords is highly effective in eliminating or minimizing mitral valve regurgitation. The procedure is presently performed with open heart surgery techniques. This requires use of cardiopulmonary bypass and arresting of the heart. This surgical approach, although working well, is a highly invasive procedure which can cause serious complications, long hospital stays and substantial expense. Consequently, a less invasive approach would be preferable. Similarly, a less invasive approach would also be preferable for treatment of the tricuspid valve which, analogously to the mitral valve, may suffer tricuspid valve disease.

Insertion of mitral leaflet chords has been done using a minimally invasive surgical approach entering the heart through its apex. The technique, was developed by the company Neochord Inc. and is described, for example, in WO2012/167120, but still requires a surgical incision and the chords do not get inserted in the papillary muscles where they normally should be fixed.

WO2008/101113 describes another example of a system for repair of the heart, including implantation of artificial chordae lines. In the described method an anchor can be attached to the papillary muscle and is coupled to the leaflet of the mitral valve by an artificial chordae line, a suture and a clip. The clip allows for adjustment of the length of the artificial chordae line. A complex multi-stage process is required to implant the papillary anchor and the suture and join them together. The papillary anchor is formed of a memory metal such as nitinol and has a 'flowered' shape with sharp 'petals' for hooking the anchor to body tissue. The flowered shape is flattened into a tube shape and held in a tube that is passed into the heart. The tube and anchor are then pressed against the papillary muscle and the anchor is pushed out of the tube so that the petals pierce the muscle and fold outward through the muscle to provide a secure coupling of the anchor to the muscle tissue. In a subsequent surgical procedure, an artificial chordae line may be attached to the anchor. Then in a further step, the suture is attached to the leaflet and this suture is joined to the chord by the clip. The suture is attached to the leaflet by locating a vacuum port near to the leaflet and pulling it into the vacuum port where it can be pierced.

It will be appreciated that this technique, whilst avoiding open heart surgery, still requires a sequence of relatively complex steps. The number of steps required increases the risk. Furthermore, the complexity of the device means that parts implanted within the body are at risk of coming loose and injuring the patient by embolization. In particular, the clip could come loose from the anchors. It is also thought that the use of a suture with an additional clip, as proposed, may not effectively repair the heart valve since it will not closely simulate a natural chord.

In an earlier patent application, WO2016/042022, the present applicant disclosed a catheter device for implanting an artificial chordae line to repair a heart valve. The catheter device of WO2016/042022 includes a mechanical gripper device for grasping the leaflet of the heart valve, with a leaflet anchor housed in the gripper. The leaflet anchor can be formed from a flexible material, such as nitinol, with a grapple hook shape in an unfolded configuration, and being able to deform elastically into the folded configuration, for example when constrained within a leaflet anchor channel in the gripper device. The hooks are straightened out when the leaflet anchor is in the folded configuration. When the leaflet is grasped by the gripper device then the leaflet anchor can be pushed out of the gripper to drive the hooks though the leaflet whilst they return elastically to the unfolded configuration, thereby securing the leaflet anchor in the leaflet.

The device described in WO2016/042022 also uses a papillary anchor with a broadly similar arrangement of foldable hooks. The papillary anchor is held within a tube of the catheter device in a folded configuration and can be pushed out of the tube with the hooks being driven into the heart wall whilst they return elastically to the unfolded configuration, thereby securing the papillary anchor to the muscle. The papillary anchor includes a locking ring acting as a locking mechanism for clamping an artificial chordae line when no force is applied. The locking ring maybe elastically deformed to release the line from the locking mechanism for adjustment of the length of the chordae line.

In another earlier patent application, WO2020/109588, the present applicant disclosed further refinements to the catheter device disclosed in WO2016/042022, and new developments related thereto. One area of refinement focussed on the design of the leaflet anchor. The leaflet anchor disclosed therein was designed to increase the surface area of the leaflet anchor in contact with the leaflet upon implantation, and to minimise trauma experienced by the leaflet during implantation.

Other anchoring systems are also known from US2009/0076547 for example, which discloses a tissue anchor comprising a single elongate strip having folded portions. The elongate strip is delivered from a tubular member passing through the tissue. A suture extends through the elongate strip, and is looped back. Upon the application of tension by the suture, the elongate strip folds around the tissue.

Whilst the devices of WO2016/042022 and WO2020/109588 provided a significant advance in this field it has been found that further refinement of the design may be advantageous. The present disclosure relates to new features building on the design of the device disclosed in WO2016/042022 and WO2020/109588 in various respects.

US 2005/251208 A1 discloses a linear anchor for anchoring to tissue, and adapted to be delivered in an elongate straightened configuration through the tissue before compressing in a longitudinal direction to plicate gastrointestinal tissue therebetween.

US 2016/157845 A1 discloses a tissue anchor having a body, protrusions from the body, and a tissue engaging fin.

The present invention is defined by the features of the independent claim. Embodiments of the invention are defined in the dependent claims.

It is an objective of the present invention to provide an improved anchor system for implantation in soft body tissue, and more preferably in heart tissue.

In accordance with the present invention a soft tissue anchor system as discussed in the second aspect, catheter devices as discussed in the fourth and fifth aspects, and a method of manufacturing a soft tissue anchor system according to the ninth aspect, are herein provided.

Viewed from a first aspect, there is disclosed a soft tissue anchor system for implantation in soft body tissue to secure an artificial line, the anchor system comprising: a line; and a tubular cap member; wherein an end of the line is fixed to the tubular cap member and extends from a central portion thereof such that, in use, the tubular cap member is configured to extend in a plane parallel to a surface of the soft body tissue when implanted in the soft body tissue and under tension of the line when it is passing through the tissue in a direction away from the surface thereof; and wherein the tubular cap member comprises an opening configured to receive a wire guide member for passing the tubular cap member through the soft body tissue during implantation.

By providing a tubular cap member comprising an opening configured to receive the wire guide member for passing the tubular cap member through the soft body tissue during implantation, the tubular cap member may be manipulated such that it can be passed through the soft body tissue in a narrow configuration. For example, the wire guide members can push the tubular cap member in a direction of insertion such that it passes through the soft body tissue in the narrow configuration. In other words, the opening may facilitate engagement between the wire guide member and the tubular cap member, such that the tubular cap member can be held end-on to then be passed through the soft body tissue in a direction parallel to its axis. Passing the tubular cap member through the soft body tissue in this manner may minimise trauma at the site of implantation of the soft tissue anchor system.

Moreover, by using a wire guide member to motivate the tubular cap member through the body tissue, rather than a needle or delivery catheter passed through the body tissue from which the soft tissue anchor is deployed, a hole in the body tissue through which the tubular cap member is passed need not be as large as a hole required by a needle or other conduit containing the soft tissue anchor system to pass through. That is, the size of the implantation site (i.e. opening made during implantation) in the body tissue is constrained by no more than the geometry of the tubular cap member, or other parts of the soft tissue anchor system passing through the soft body tissue. In comparison, the size of openings which are required for implantation via a needle will always be constrained by the size of the needle which is always larger than the member it is to deploy. This may reduce trauma at the site of implantation.

Once the tubular cap member has been passed through the soft body tissue, the wire guide member can be withdrawn. In use, when a tension is then applied via the line passing back through the soft body tissue from where the tubular cap member was passed through the soft body tissue, the tubular cap member is pulled against the soft body tissue such that the tubular cap member extends in a plane parallel to the soft body tissue. The line may then extend through the soft body tissue, with the tubular cap member contacting the soft body tissue with maximal surface area and to prevent withdrawal of the tubular cap member back through the implantation site.

Accordingly, the soft tissue anchor system of this aspect may be implanted in soft body tissue such that an artificial chordae line is secured to the soft body tissue by a resulting contact force between the tubular cap member and the soft body tissue, in a manner that minimises a size of the implantation site, and thus any resulting trauma, during its implantation.

There is hence provided an arrangement which, where a wire guide member engages the tubular cap member, the tubular cap member will straighten and extend collinearly with the line. This may facilitate implantation of the tubular cap member into the soft body tissue. When the line then experiences a force, the line may turn the tubular cap member to lie in parallel with a surface of the body tissue owing to its centralised fixing with the tubular cap member. This may facilitate securement of the tubular cap member to the soft body tissue, since under tension the tubular cap member will be turned perpendicular to the line in a "T" configuration, the line normal to the surface of the body tissue and the length of the tubular cap member parallel to the surface of the body tissue, preventing movement of the tubular cap member through the soft body tissue (similarly to how a treasury tag remains secured in use).

The tubular cap member, in combination with the line, may be considered a 'T-bar' anchor, in view of its deployed "T" configuration.

The tubular cap member may be an elongate, tubular body comprising a hollow for receiving the wire guide member. The opening/hollow may extend entirely along the entire length of the tubular cap member or may alternatively comprise a blind opening/hollow.

The tubular cap member may comprise a first end, a second end distal to the first end, and a side wall extending between the first end and the second end. The opening may be located at a first end of the tubular cap member. The second end of the tubular cap member may define a tip of the tubular cap member, the tip being configured for insertion into the soft body tissue.

The central portion of the tubular cap member may be considered as the portion of the tubular cap member located around the geometric centre of the tubular cap member (i.e. a central point along the longitudinal axis of the tubular cap member), this portion comprising: less than 30% of the length of the tubular cap member; less than 40% of the length of the tubular cap member; less than 50% of the length of the tubular cap member; or less than 60% of the length of the tubular cap member. The central portion of the tubular cap member may be considered to include the 'middle third' of the length of the tubular cap member. In other words, the central portion of the tubular cap member may comprise any point on the tubular cap member that is closer to the geometric centre of the tubular cap member than either the first end or the second end.

The tubular cap member may be formed of a suitable rigid biocompatible material capable of retaining its shape, such as stainless steel, titanium, engineering plastics or nitinol.

The line may be regarded as a tension line since, in use, the line applies a tension force to the tubular cap member upon implantation.

The line may be formed of a suture material. The line may be the artificial line. Alternatively, the line may be fixed to the artificial line through any suitable fastening arrangement, such as a knot or a looped engagement.

As described above, the end of the line is fixed to the tubular cap member, with the line extending from a central portion of the tubular cap member. Having the point from which the line extends from the tubular cap member be located along the central portion of the tubular cap member may provide a stable arrangement for maintaining the "T" configuration upon implantation.

In some examples, the line may extend substantially centrally, i.e. substantially centrally from the centre of the tubular cap member (e.g. in line with the geometric centre of the tubular cap member).

In other examples, the line may extend asymmetrically, and may therefore extend from a point along the tubular cap member more towards the first end or the second end of the tubular cap member (yet still within the central portion of the tubular cap portion).

For example, asymmetric arrangements may locate the point from which the line extends from the tubular cap member distally from the opening (i.e. towards the tip of the tubular cap member). Having the line extend from a point located towards the tip of the tubular cap member may facilitate the application of a torque to the tubular cap member to place it in the "T" configuration. However, in other preferred examples, the point from which the line extends from the tubular cap member may be proximal to the opening of the tubular cap member (i.e. closer towards the opening of the tubular cap member than the tip). Having the line extend from a point located towards the opening of the tubular cap member may better distribute a contact force exerted by the tubular cap member when it extends in the plane parallel to a surface of the body tissue when implanted and under tension of the line. The end cap may have a smaller projected area closer to its tip (e.g. due to a taper and/or bevel of the tip), and the tubular cap member may have a larger projected area closer to the opening (e.g. due to a lack of taper/the tubular cap member being wider towards the opening). Accordingly, placing the point from which the line extends from the tubular cap member proximal to the opening may result in a more balanced distribution of contact force exerted by a profile of the tubular cap member, when it extends in the plane parallel to a surface of the body tissue.

Accordingly, the line may extend from the central portion of the tubular cap member to which it is fixed from: substantially at the centre of the tubular cap member; towards the first end of the tubular cap member; or towards the second end of the tubular cap member, as described above.

The manner in which the end of the line is fixed to the tubular cap portion may cover a length of the tubular cap member. For example, a length of the end of the line may be wrapped circumferentially around the tubular cap member or a portion thereof, with this length of line additionally extending along an axial length of the tubular cap member (i.e. in a spiralled or coiled manner). The line extending from the tubular cap member may extend from this coiled portion of line. Alternatively, a length of the end of the line fixed to the tubular cap member or a portion therefor may extend substantially axially along the tubular cap member or the portion thereof. The line extending from the tubular cap member may extend from this length of line.

The end of the line may be fixed to the central portion of the tubular cap member. However, the end of the line need not be restricted to being fixed to the central portion of the tubular cap member, provided the line extends from the central portion of the tubular cap member. Thus, in an alternative wording, the soft tissue anchor system may be considered to have an end of the line fixed to a central portion of the tubular cap member, with this specific recitation of the end of the line referring to the portion of line initially extending from the tubular cap member (i.e. being an 'end' portion of the line determining the position along the tubular cap member at which, in use, tension can be applied by the line to the tubular cap member).

The wire guide member received by the opening is suitable for passing the tubular cap member through the soft body tissue during implantation. In other words, the wire guide member is suitable for fixing the soft tissue anchor system in the body tissue by passing the tubular cap member through the soft body tissue during implantation. The passing of the tubular cap member through the soft body tissue results in the implantation of the soft tissue anchor system in the body tissue to secure an artificial line.

The tubular cap member may be a single component (e.g. a hollow tube), wherein the line is tied or otherwise secured to the tubular cap member. The tubular cap member may be formed as a single component by laser cutting or machining nitinol or stainless steel.

Alternatively, the tubular cap member may comprise an outer tubular member and an inner tubular member, wherein the outer tubular member is configured to receive the inner tubular member. That is, the inner tubular member may be nested within, and/or concentric with the outer tubular member. The inner tubular member may define the opening configured to receive the wire guide member.

The inner tubular member may comprise a flared inlet defining the opening configured to receive the wire guide member. The flared inlet will be understood to be a portion of the inner tubular member defining the opening, wherein a circumferential extent of the inner tubular member is increased relative to the rest of the inner tubular member.

Preferably, the flared inlet comprises an angled face to facilitate guiding of the wire guide member into the inner tubular member. This arrangement may be preferable where the wire guide member is not received by the opening of the tubular cap member prior to implantation.

The flared inlet may be configured to mate with a corresponding portion of the wire guide member. That is, the flared inlet may be complementary to a shape of e.g. a shoulder region, or bulge portion, of the wire guide member. The angled face of the flared inlet may be complementary to the shoulder region, or bulge portion, of the wire guide member. The flared inlet may improve contact between the wire guide member and the tubular cap member during implantation of the fabric body.

The inner tubular member may be retained within the outer tubular member via a press fit and/or a mating notch. The inner tubular cap member may also or alternatively be retained within the outer tubular member via a crimp operation.

The flared inlet may be configured to locate the inner tubular member within the outer tubular member. An end of the inner tubular member proximal to the opening may mate with the outer tubular member via a mating notch, a press fit and/or a crimp connection. The flared inlet may provide the press fit between the inner tubular member and the outer tubular member. An end of the inner tubular member distal to the opening may also mate with the outer tubular member via a mating notch, a press fit and/or a crimp connection. Providing at least two regions or points of retention may improve the retaining strength of the inner tubular member within the outer tubular member.

The outer tubular member may comprise a shoulder region configured to mate with the inner tubular member, distal to the opening. This may facilitate the locating of the inner tubular member within the outer tubular member, such that the opening defined by the inner tubular member is suitably located at the end of the tubular cap member.

The tubular cap member may be configured to receive the line between the inner tubular member and the outer tubular member. The line may be fixed to the tubular cap member by crimping, swaging, clamping, gluing and/or sandwiching the line between the inner tubular member and the outer tubular member. The line could also be fixed by stitching it or tying it to the tubular member.

The outer tubular member may comprise a side wall and an aperture (i.e. an opening) formed in the side wall, wherein the aperture receives the line. The end of the line may be fixed to the tubular cap member between the outer tubular member and the inner tubular member. The opening can be formed in a central region of the outer tubular member, thereby facilitating the extension of the line from the central portion of the tubular cap member and optionally the fixing of the end of the line to the central portion of the tubular cap member.

The outer tubular member may comprise a groove extending from the aperture towards the opening. The groove may be configured to receive the line during implantation. Accordingly, a profile of the line during implantation may be sunk into the tubular cap member, and thereby be at least partially contained within the profile of the tubular cap member, such that a size of the implantation site is minimised and trauma during implantation may be further reduced.

The tubular cap member may comprise a pointed tip configured to pierce the body tissue, wherein the pointed tip is located at an end of the tubular cap member distal to the opening configured to receive the wire guide member. The pointed tip may be configured to pierce the body tissue when a motive force is applied to the tubular cap member by the wire guide member.

The pointed tip may comprise a conical tip, i.e. a pointed tip having rotational symmetry about a longitudinal axis of the tubular cap member.

Alternatively, the pointed tip may have a rotationally asymmetric geometry about a longitudinal axis of the tubular cap member. Using a rotationally asymmetric tip geometry for the pointed tip may facilitate the pointed tip having a higher draft angle for a plane of its point (i.e. a plane of its shearing edge). Using a pointed tip with a higher draft angle may reduce the force required for the pointed tip to pierce the soft body tissue, and thus the trauma experienced at the implantation site during implantation.

To this end, the pointed tip may comprise a bevel plane. A bevel plane will be understood to define a single plane, i.e. a plane in which a face of the bevel is ground (e.g. a grinding plane) or otherwise formed.

The bevel plane may define a face of the pointed tip. The bevel plane may have a draft angle, relative to a radial plane of the tubular cap member, of: more than 50°, more than 60°, more than 70°, or more than 80°. The bevel plane may have a draft angle, relative to the longitudinal axis of the tubular cap member, of: less than 10°, less than 20°, less than 30°, or less than 40°.

The pointed tip may comprise a single bevel plane, and may be described as a bevel tip, or a chisel tip.

Alternatively, the pointed tip may comprise two lancet planes in addition to the bevel plane. The lancet planes may flank the bevel plane, preferably symmetrically. The bevel plane and each of the lancet planes will be understood to define a respective plane (such as a grinding plane). Such a tip geometry may be described as a lancet point tip. Therefore, the pointed tip may be a lancet point tip.

In arrangements where the opening configured to receive the wire guide member is comprises a blind bore, or a blind hollow, the pointed tip may be considered a pointed tip stylet. In arrangements where the opening configured to receive the wire guide member extends completely through the tubular cap member (where such an arrangement facilitates manufacture of the tubular cap member), the pointed tip may be considered a pointed tip cannula.

The pointed tip may be a retractable pointed tip. The tip of the tubular cap member may have a first position in which the tip is a pointed tip, i.e. in which the pointed tip is deployed, and may have a second position in which the tip is a dulled and/or rounded tip, i.e. in which the pointed tip is retracted. The second position may be a rest position of the retractable pointed tip. The retractable pointed tip may be urged from the second position to the first position via engagement of the wire guide member with the tubular cap member.

The outer tubular member may define the pointed tip. If the pointed tip is permanently present, the inner tubular member may comprise a blind bore which receives the wire guide member. If the pointed tip is a retractable pointed tip, the inner tubular member may be hollow, such that the wire guide member may actuate the retractable pointed tip and/or any suitable deployment mechanism.

Alternatively, the tubular cap member may comprise a dull and/or rounded tip, wherein the dulled and/or rounded tip is located at an end of the tubular cap member distal to the opening configured to receive the wire guide member.

By having a dulled and/or rounded tip, a risk of laceration and/or further trauma to the body tissue caused by the tubular cap member, once implanted or otherwise, is mitigated.

The outer tubular member and the inner tubular member each define openings extending along the entirety of their length, i.e. they are hollow along the entirety of their length. The inner tubular member may be configured to allow passage of a piercing section of the wire guide member therethrough, and may also be configured to abut a shoulder portion, or bulge portion, of the wire guide member. A wire guide member comprising a piercing section, which is configured to pierce the body tissue during implantation of the anchor system, may be regarded as a piercing wire guide member.

By enabling passage of the piercing wire guide member through the inner tubular member, the tubular cap member may comprise a dulled and/or rounded tip whilst still enabling piercing of the body tissue during implantation of the anchor system. Further, by providing an inner tubular member configured to abut a shoulder portion, or bulge portion, of the wire guide member, suitable contact for manipulation of the arm portions via the piercing wire guide member may be realised.

Moreover, the use of tubular cap members which are suitable for use with a piercing wire guide member may further reduce trauma experienced by the site of implantation, because the piercing section of the wire guide member may have a smaller diameter than, for example, a hollow needle member making the incision for implantation.

The inner tubular member may extend out of the outer tubular member, and may define the tip of the tubular cap member. This arrangement results in a step transition located at the tip of the tubular cap member between the inner tubular member and the outer tubular member, which may facilitate smoother passage of the tubular cap member through the body tissue during implantation.

Alternatively, the outer tubular member may comprise a tapered portion located at the tip of the tubular cap member. That is, the tip of the tubular cap member may be defined by the outer tubular member and an end of the inner tubular member may be contained within the outer tubular member. This arrangement results in a continuous transition located at the tip of the tubular cap member between the tip of the tubular cap member and the outer tubular member, which may facilitate smoother passage of the tubular cap member through the body tissue during implantation.

The soft tissue anchor system may comprise a fabric body having a base portion and an arm portion extending from the base portion; wherein the arm portion is configured to collapse in folds towards the base portion such that, in use, the body tissue is sandwiched between the base portion and the arm portion; wherein the tubular cap member is fixed at an end of the arm portion distal to the base portion.

In other words, viewed from another aspect there is disclosed a soft tissue anchor system for implantation in soft body tissue to secure an artificial line, the anchor system comprising: a fabric body comprising a base portion and an arm portion extending from the base portion; wherein the arm portion is configured to collapse in folds towards the base portion such that, in use, the body tissue is sandwiched between the base portion and the arm portion; wherein the arm portion comprises a tubular cap member fixed at an end of the arm portion distal to the base portion; wherein an end of the line is fixed to the tubular cap member and extends from a central portion thereof such that, in use, the tubular cap member is configured to extend in a plane parallel to a surface of the soft body tissue when implanted in the soft body tissue and under tension of the line when it is passing through the tissue in a direction away from the surface thereof; and wherein the tubular cap member is configured to receive a wire guide member for implanting the anchor in body tissue and pull the arm portion through the body tissue during implantation.

By providing a fabric body in combination with the tubular cap member, the anchor system may be able to contact a greater surface area of body tissue when implanted, and hence may have improved stability and/or improved tissue ingrowth upon implantation. Further, by providing the collapsible arm portion attached via a base portion, the base portion itself may further be able to provide a greater lateral surface by which to connect the anchor system and the soft body tissue upon implantation.

Further, the use of a fabric body compared to a rigid body such as a metal body may reduce damage to the body tissue caused by the anchor during and/or after implantation. The fabric may better complement a surface of the body tissue that it contacts, thus spreading force exerted on the body tissue over a greater area and reducing trauma experienced by the body tissue at the site of implantation.

Collapsing of the arm portion in folds towards the base portion may be considered as a concertina motion. Accordingly, the arm portion may also be configured to concertina towards the base portion such that, in use, the body tissue is sandwiched between the base portion and the arm portion.

The fabric body, being formed of a fabric material, may be naturally disposed to fold and/or concertina because of the conformable nature of fabric. However, in some arrangements, the arm portion may comprise fold lines, narrowed sections, and/or weakened portions configured to aid in folding and/or concertinaing the arm portion.

It will be appreciated that, owing to the tubular cap member being fixed at an end of the arm portion distal to the base portion, the arm portion is arranged to be pulled through the soft body tissue as the tubular cap member passes through the soft body tissue during implantation.

The tubular cap member may be regarded as an 'end cap', for example in reference to how the tubular cap member may form a capped end of the arm portion.

The arm portion may be fixed towards and/or at the opening of the tubular cap member, i.e. at an end of the tubular cap member distal to the tip of the tubular cap member (i.e. the end of the tubular cap member in the direction of insertion during implantation).

The outer tubular member and the inner tubular member may be configured to sandwich, crimp and/or clamp the distal end of a respective arm portion therebetween, thereby fixing the tubular cap member to the arm portion. Adhesives may be additionally or alternatively employed, to fix the tubular cap member to the arm portion.

The outer tubular member may comprise a groove, channel, aperture or other suitable feature for guiding the arm portion between the outer tubular member and the inner tubular member. This groove, channel, aperture or other suitable feature may be formed in circumferential alignment with the aperture for the line, and may also be formed at or towards the opening configured to receive the wire guide member. Providing the line and the arm portion in alignment with one another may improve the stability of the soft tissue anchor system when implanted in soft body tissue.

Fixing the arm portion towards the opening of the tubular cap member and the line at the central portion of the tubular cap member may facilitate the line applying a torque to the tubular cap member when under tension, thereby causing the tubular cap member to lie flat against the soft body tissue when implanted.

Fixing the arm portion towards and/or at the opening of the tubular cap member may also facilitate retrieval of the tubular cap member. For example, upon retrieval of the tubular cap member, tension in the line may be relaxed. The arm portion/fabric body may then be pulled so as to apply tension. This tension may result in a torque being applied to the tubular cap member, causing the tubular cap member to rotate such that it extends in a plane perpendicular to the surface of the soft body tissue (e.g. such that it extends collinearly with the line). The tubular cap member may then be pulled and/or guided back through the soft body tissue. This arrangement may therefore facilitate retrieval of the tubular cap member, in a manner that also minimises any trauma experienced by the body tissue during retrieval.

Alternatively, the aperture formed in the side wall of the outer tubular member receiving the line may also receive the arm portion. Accordingly, the fixing of the arm portion and the line to the tubular cap member may be coincidental.

Alternatively, and as may be preferable in arrangements where the wire guide member is received by the opening of the end cap prior to implantation, the outer tubular member may not comprise a channel for guiding the arm portion between the outer tubular member and the inner tubular member and/or a flared inlet defining the opening configured to receive the wire guide member. This may result in the tubular cap member being made shorter and/or thinner (i.e. narrower in diameter), and hence more compact, for arrangements where the wire guide member need not be guided into engagement with the tubular cap member during implantation.

The fabric body may be formed of a single layer of fabric or multiple layers of fabric. In this context, a fabric may be considered as a material formed as a weave made of threads or threadlike forms. One possible material is a polyester fabric, other materials include fabrics of PET, UHMPE, EPTFE, PTFE and similar. The fabric body may be formed of any suitable flexible, conformable and biocompatible material. The fabric body may be formed by laser cutting an existing material or any other suitable manufacturing technique.

Where the line is fixed to the artificial line, the line will be fixed to the artificial line behind the base portion, i.e. distally to the tubular cap member and not between the tubular cap portion and the base portion. In other words, the line may have a length at least greater than the length of the fabric body, with a first end joined to the tubular cap member and a second end extending from the base portion. This second end, located past the base portion, may then be fixed to the artificial line.

The arm portion may be configured to collapse in folds towards the base portion by action of the line threaded through the arm portion and the base portion. Thus, the line may be configured to collapse the arm portion in folds towards the base portion when a tension force is applied to the line.

When a tensile force is applied to the line the entirety of the arm portion may collapse in folds towards the base portion under tension of the line, owing to the line being fixed to the tubular cap member and being threaded through the arm portion.

The location of the threading of the line may facilitate the collapse of the arm portion in folds. For example, as the line is pulled, the line may draw holes in the arm portion through which the tension line is threaded together. Accordingly, the arm portion may be biased to fold in relation to each location the tension line is threaded through the arm portion.

Preferably, the anchor system is configured so that the tensile force is applied to a portion of the line threaded through the base portion. This arrangement may facilitate the entire collapse of the arm portion.

The line may be threaded no more than three or four times through the arm portion and the base portion. This arrangement may facilitate the holes in the fabric body through which the line is threaded being further spaced apart, and/or a shorter fabric body being employed. Relative to the size of the anchor, each of these arrangements may provide wider folding portions of the fabric body that provide improved stability for the anchor system upon implantation. Using a shorter fabric body may also reduce the size of the overall soft tissue anchor system, thus improving its packaging within a delivery device/system for implantation.

Additionally, or alternatively, the arm portion may be configured to collapse in folds towards the base portion due to the presence of a backbone formed of elastic material. The elastic material may be a shape memory metal, such as nitinol. The backbone may be in contact with at least each of the arm portions, and may further be in contact with the base portion. The backbone may be embedded or interwoven with the fabric body, and/or may be sandwiched between layers of the fabric body.

The backbone may comprise a folded configuration and an unfolded configuration. The backbone may be held in the unfolded configuration by application of a constraining force. In the unfolded configuration, the arm portions may extend away from the base portion. Upon removal of the constraining force, the backbone may return to the folded portion in which the backbone comprises one or more folds. The arm portions will occupy the same shape as the backbone, owing to the contact therebetween. Accordingly, the backbone may be a resiliently biased backbone configured to urge the arm portions in folds towards the base portion.

Where a backbone is employed the line may be threaded through the arm portion only, or may extend adjacent to the folds formed in the arm portion upon its collapse.

Each of the arrangements of (i) the line being threaded through the arm portion; and (ii) the base portion and the backbone formed of an elastic material; as described above, may be considered as various means for collapsing the arm portion in folds towards the base portion.

In other words, viewed from another aspect there is disclosed a soft tissue anchor system for implantation in soft body tissue to secure an artificial line, the anchor system comprising: a fabric body comprising a base portion and an arm portion extending from the base portion; and a means for collapsing the arm portion in folds towards the base portion such that, in use, the body tissue is sandwiched between the base portion and the arm portion; wherein the arm portion comprises a tubular cap member fixed at an end of the arm portion distal to the base portion; wherein an end of the line is fixed to the tubular cap member and extends from a central portion thereof such that, in use, the tubular cap member is configured to extend in a plane parallel to a surface of the soft body tissue when implanted in the soft body tissue and under tension of the line when it is passing through the tissue in a direction away from the surface thereof; and wherein the tubular cap member is configured to receive a wire guide member for implanting the anchor in body tissue and pull the arm portion through the body tissue during implantation.

Under an alternative wording, the means for collapsing the arm portion in folds towards the base portion may be equally considered as a mechanism for collapsing the arm portion in folds towards the base portion and/or a collapsing mechanism configured to collapse the arm portion in folds towards the base portion comprising at least one of the arrangements of (i) the line being threaded through the arm portion; and (ii) the base portion and the backbone formed of an elastic material; without departing from the aspect described above.

The tubular cap member may be configured to extend collinearly with the arm portion during implantation and/or retrieval of the fabric body. The tubular cap member may also be configured to extend parallel to a plane of each fold of the arm portion when a tensile force is applied to the line.

As described above, the arm portion may be fixed at an end of the tubular cap member proximal to the arm portion (e.g. where the end where the opening is located), and the line extends from a central region of the tubular cap member. Thus, tension forces applied due to the arm portion and/or the line during implantation may cause a torque to act on the tubular cap member, facilitating the transition of the tubular cap member from an arrangement in which it extends collinearly with the arm portion to one in which it extends in a plane parallel to each fold of the arm portion and/or the surface of the soft body tissue.

Accordingly, when the wire guide member engages the tubular cap member, the tubular cap member will straighten and extend collinearly with the arm portion. This may facilitate implantation of the arm portion of the fabric body in the body tissue. When the line experiences a tensile force and thus collapses the arm portion in folds, the tension may turn the tubular cap member to lie parallel with the folds of the arm portion, and hence parallel with the surface of the body tissue in which the arm portion fold against. This may facilitate securement of the arm portion to the body tissue, and may also minimise a profile of the tubular cap member protruding from the body tissue during implantation. For example, the tubular cap member may turn perpendicular to the tension line in the aforementioned "T" configuration, with the line normal to the surface of the body tissue and the length of tubular cap member parallel to the surface of the body tissue, preventing movement of the tubular cap member through the body tissue. When the fabric body portion is retrieved from the body tissue, e.g. by being pulled from a direction distal to the tubular cap members, a tensile force acting through the arm portion may self-right the tubular cap member from the parallel position, such that it extends collinearly with the arm portion during retrieval. Thus, by coupling the arm portion to the tubular cap member at an end thereof, the tension from the arm portion will tend to straighten out the tubular cap member (e.g. from the "T" configuration mentioned above) allowing it to align with the arm portion and hence align with the hole through the body tissue. This may facilitate retrieval and minimise any trauma experienced by the body tissue during retrieval.

The fabric body may optionally comprise a stiffening member defining a plurality of holes through which the tension line is threaded. The stiffening member may for example be implemented as a tensile member, giving added tensile strength to the fabric body. The stiffening member may be regarded as a backbone to the fabric body, and may preferably comprise a complementary shape to the fabric body. The tension line is preferably threaded through the stiffening member in addition to the fabric body. The stiffening member may define holes through which the tension line is threaded.

The stiffening member may be embedded in the fabric body. The stiffening member may be interwoven with the fabric body. The stiffening member may be sandwiched between layers of the fabric body.

The stiffening member may be at least one high tensile strength line, interwoven in the fabric, to take excessive tensile loads that may be exerted on the fabric during implantation, retraction or along its lifecycle.

The stiffening member may reinforce the fabric body, and provide additional support at the points through which the tension line is threaded through the fabric body as well as optionally where the tubular cap members are attached and at any point where there is narrowing of the fabric along its length. Accordingly, the stiffening member may improve the structural integrity of the fabric body.

The fabric body may be a U-shaped fabric body comprising the base portion and at least two arm portions extending from the base portion; wherein each arm portion comprises a tubular cap member fixed at an end of the arm portion distal to the base portion.

In other words, viewed from another aspect there is disclosed a soft tissue anchor system for implantation in soft body tissue to hold an artificial line, the anchor system comprising: a line; a U-shaped fabric body comprising a base portion and at least two arm portions extending from the base portion; wherein each arm portion is configured to collapse in folds towards the base portion such that, in use, the body tissue is sandwiched between the base portion and each of the arm portions; and a tubular cap member fixed at an end of each arm portion distal to the base portion; wherein an end of the line is fixed to each tubular cap member and extends from a central portion thereof such that, in use, each tubular cap member is configured to extend in a plane parallel to a surface of the soft body tissue when implanted in the soft body tissue under and under tension of the line when it is passing through the tissue in a direction away from the surface thereof; and wherein each tubular cap member comprises an opening configured to engage a wire guide member for implanting the U-shaped fabric body in the body tissue.

By providing a fabric body comprising at least two arm portions extending from the same base, the anchor system may be able to contact a greater surface area of body tissue when implanted, and hence may have improved stability and/or improved tissue ingrowth upon implantation. Further, by providing the collapsible arm portions attached via a base portion, the base portion itself may further be able to provide a greater lateral surface by which to connect the anchor system and the leaflet upon implantation. A U-shaped anchor system, i.e. with the option of at least two arm portions, may provide enhanced performance compared to the single arm portion.

Accordingly, a soft tissue anchor system as described above may have an improved securement strength compared to known anchor systems comprising a single arm portion and/or fixing member and/or using different design features.

In the example herein using at least two arm portions the U-shaped body is so-called due to the U-shape formed by the base portion and two arm portions. However, it will be appreciated that this merely refers to the unit structure of the fabric body with two arm portions and the shape will differ if more arm portions are present. For example, a fabric body comprising three arm portions may be regarded as a W-shaped body, wherein the W-shaped body comprises two U-shaped units. Accordingly, the anchor system may comprise a fabric body comprising multiple U-shaped units such that a fabric body of suitable size is arrived at for the desired purpose of the soft tissue anchor system.

The base portion may define a maximum width of the U-shaped fabric body. That is, the base portion may be the widest part of the fabric body.

The base portion may comprise a pair of wing portions. The wing portions may extend wider than an outermost edge of the arm portions.

By providing a base portion of increased width, or at least greater width than compared to the arm portions, the base portion may be configured to provide greater lateral support to the fabric body upon implantation in the body tissue.

When the anchor system is in use then the base portion may be implanted on the atrial side of the leaflet, where the retention is required during a heartbeat. In this context an increased lateral support for the atrial side location can be beneficial to achieve a wide area of support of the leaflet. If the base portion is at the atrial side then the arm portion(s) are on the ventricle side. They act to hold the anchor system in place and provide some support, while the atrial side portion (base portion) is more active in carrying forces exerted by blood flow on the leaflets during heart contraction (systole).

The base portion may comprise a shape retention member. The shape retention member may be configured to increase a lateral stiffness of the base portion. The shape retention member may be configured to maintain a planar extent of the base portion, or a 3D shape that conforms with the shape of the leaflet/anatomy.

By stiffening the base portion using a shape retention member, the base portion may be able to provide greater lateral support to the body tissue in which the fabric body is implanted. Further, tensile forces experienced by the fabric body during implantation when holding the artificial line, may be more evenly distributed across the base portion when a shape retention member is provided. This may improve the stability of the fabric body upon implantation.

The shape retention member may be formed of an elastic material. The base portion may comprise a folded configuration and an unfolded configuration, wherein the shape retention member is configured to urge the base portion from the folded configuration to the unfolded configuration upon removal of a constraining force. The base portion may be substantially planar in the unfolded configuration.

The shape retention member may be embedded, interwoven with, and/or sandwiched between layers of, the base portion.

The base portion may be formed by folding a portion of the fabric body back over on itself, and sandwiching the shape retention member therebetween. The shape retention member and/or the folded portion of the fabric body may be fixed in place by an adhesive, ultrasonic welding, stiches or other suitable fixing means.

The shape retention member may comprise a fluorescent marker. The fluorescent marker may assist in locating the fabric body via imaging during implantation and/or retrieval.

The shape retention member may be formed of a shape retention metal such as stainless steel, titanium or nitinol. The shape retention member may be formed by laser cutting, or by shaping a wire formed of a similar material.

The base portion may provide a surface for retrieving the fabric body following implantation. The fabric body may be retrieved by pulling the arm portions out of the body tissue by grasping the base portion, and pulling the base portion away from the body tissue. The shape retention member may improve ease of retrieval, as the shape retention member may help distribute tensile forces experience across the base portion during retrieval.

The shape retention member in the base portion may also prevent the arm portion(s) from over extending through the leaflet when being deployed, as it can act as a stopper while the arms are being pulled through the tissue by the guide wires - in that way the base portion combined with the shape retention member may act to place a limitation to how far the deployment wires can go during deployment.

The base portion may be configured to be grasped by a snare, and optionally the base portion may be located using the fluorescent marker. For example, the snare may encircle the arm portions where they initially extend from the base portion, on the same side of the body tissue as the base portion when implanted. Then, the snare may be tightened such that, upon retraction, the snare grasps the base portion. The pair of wing portions may provide a widened surface of the base portion by which the base portion may be grasped by the snare.

The base portion combined with the shape retention member may assist with placing the soft tissue anchor system in locations over at a wider area (narrow and deep) of the leaflet while still achieving leaflet support.

The fabric body may comprise a narrow waist portion extending between the base portion and the/each arm portion. Upon implantation, the narrow waist portion may be aligned with the soft body tissue such that the narrow waist portion is encircled by the soft body tissue at the implantation site. The narrow waist portion may be configured to be longer than a thickness of the soft body tissue in which the soft tissue anchor system is to be implanted. For example, a length of the narrow waist portion may be: greater than at least 1mm; greater than at least 2mm; or greater than at least 3mm. The length of the narrow waist portion may be between 1 and 3 mm. The narrow waist portion may equally be considered as part of the arm portion, such that the arm portion(s) comprise a narrow waist portion at an end of the arm portion(s) proximal to the base portion, or alternatively may be considered its own respective portion as described above. The narrow waist portion may stabilise the implantation of the base portion adjacent to the body tissue. This feature also may aid in holding the arm portion(s) in place prior to tensioning the line.

Each arm portion may be provided with its own respective line. Each line may be an artificial line, i.e. a line acting as an implantable cord. The line may be fixed to the central region of each tubular cap member as described above.

For example, in some arrangements where the soft tissue anchor comprises at least two arm portions, the artificial line may be the line for one of the arm portions, and for the remaining arm portions additional lines may be employed, each fixed to the artificial line (in sliding engagement or a steadfast engagement). Additionally, or alternatively, two of the arm portions may comprise a mutual line, this line joined to the artificial line/the line of another arm portion adjacent to the base portion. A first end of the line may be joined to the tubular cap member of the first arm portion, and a second end of the line may be joined to the tubular cap member of the second arm portion. A central portion of the line may then be fixed to the artificial line/any other lines.

Where the line is threaded through the arm portion(s), for each arm portion(s) there may be provided: a hole in the base portion; a hole in the narrow waist portion; and at least one hole in the arm portion. The hole in the narrow waist portion may be located closer to the base portion than the arm portion (i.e. the hole may be located proximal to the base portion). This arrangement may improve compression of the fabric body around the soft body tissue, since the placement of a hole in the narrow waist portion proximal to the base portion encourages the collapse of the base portion towards the soft body tissue/towards the collapsing arm portions. Accordingly, the stability of the anchor system upon implantation may be improved.

Where a narrow waist portion is not provided, for each arm portion(s) there may be provided: a hole in the base portion; and at least two holes in the arm portion.

It will be appreciated that the above-described features of the arm portion(s) and the base portion may be applicable to soft tissue anchor systems comprising one arm portion as well as soft tissue anchor systems comprising at least two arm portions.

As described above, the artificial line may be joined to a portion of the line distal to the end fixed to the central portion. In some arrangements, the artificial line may be slidably joined to the portion of the line threaded through the base portion. The artificial line may be arranged to apply the tensile force to the line.

The artificial line may be slidably joined to the line by any suitable knot, for example a bridle knot. Alternatively, the artificial line may be slidably joined to the line by an intermediate member, such as an eyelet connected to the artificial line through which the tension line passes.

By enabling the artificial line to slide relative to the tension line, the point at which the tensile force is applied to the tension line may naturally adjust as the tensile force is applied. This may help spread the distribution of the tensile force across each arm portion, such that each arm portion is suitably retracted. Further, such an arrangement may facilitate implanting the fabric body in the body tissue at an angle with respect to the artificial line.

Alternatively, the artificial line may be fixedly joined to the line such that the relative positions of the line and the artificial line does not change.

Alternative to comprising the U-shaped fabric body, the soft tissue anchor system may comprise a plurality of the 'T-bar' anchor as described above. For example, the soft tissue anchor system may comprise at least two T-bar anchors, each configured to receive a wire guide member. The T-bar anchors may be joined to a mutual line such that they are thereby connected.

In other words, viewed from another aspect there is disclosed a soft tissue anchor system for implantation in soft body tissue to secure an artificial line, the anchor system comprising: a line; and a plurality of tubular cap members; wherein an end of the line is fixed to each tubular cap member and extends from a central portion thereof such that, in use, each tubular cap member is configured to extend in a plane parallel to a surface of the soft body tissue when implanted in the soft body tissue and under tension of the line when it is passing through the tissue in a direction away from the surface thereof; and wherein each tubular cap member comprises an opening configured to receive a wire guide member for passing the tubular cap member through the soft body tissue during implantation.

In use, each opening may be configured to receive a respective wire guide member such that each tubular cap member is simultaneously implanted in the body tissue. Alternatively, in use, each opening may be configured to receive a mutual wire guide member in turn such that each tubular cap member is sequentially implanted in the body tissue.

Each T-bar anchor may be provided with its own respective line. Each line may be an artificial line, i.e. a line acting as an implantable cord. Alternatively, the line may be fixed to each tubular cap member/T-bar anchor.

For example, in some arrangements where the soft tissue anchor comprises at least T-bar anchors, the artificial line may be the line for one of the T-bar anchors, and for the remaining T-bar anchors additional lines may be employed, each fixed to the artificial line (in sliding engagement or a steadfast engagement). Additionally, or alternatively, two of the T-bar anchors may comprise a mutual line, this line joined to the artificial line/the line of another T-bar anchor. A first end of this mutual line may be joined to the tubular cap member of the first T-bar anchor, and a second end of the mutual line may be joined to the tubular cap member of the second T-bar anchor. A central portion of the line may then be fixed to the artificial line/any other lines. The mutual line may also be regarded as the fixing between any lines also connected to a tubular cap member.

The tension line may comprise a plurality of bridle lines, with each bridle line being associated with (i.e. an end of each bridle line being fixed to) a respective tubular cap member.

Each bridle line is preferably connected to a common bridle point, wherein the bridle point is configured to place each bridle line under tension when the soft tissue anchor system is implanted in the soft body tissue and the bridle point itself is under tension. That is, the bridle point is configured to place each bridle line under tension when each bridle line is passing through the tissue in a direction away from the surface of the soft body tissue and when the bridle point itself is under tension.

By providing an arrangement comprising a plurality of bridle lines, each placed under tension due to a common bridle point, the tubular cap members of the soft tissue anchor may each experience a tensile force pulling them towards a plane or axis intersecting the bridle point and in the direction of the tensile force applied to the bridle point. This may result in each of the tubular cap members being motivated towards one another, and in turn capturing, gathering and/or pinching together any excess body tissue located between the tubular cap members upon implantation. Accordingly, this arrangement may restore the shape of the soft body tissue and/or provide additional structural support to the body tissue.

The arrangement comprising a plurality of bridle lines each connected to a common bridle point may be particularly advantageous when the soft body tissue is a heart valve leaflet. The reshaping of the tissue of the leaflet resulting from capturing excess leaflet tissue as described above has been found to provide an outcome similar to resection of the leaflet. Resection is a common surgical step in existing methods of heart valve repair, in which a surgeon resects a damaged section of the heart valve and stitches the edges of the remaining tissue together. Resection is often performed where there is excessive leaflet tissue present. However, by providing a line arrangement comprising a bridle point and a plurality of bridle lines, the need for a resection step may be obviated. This could simplify the overall surgical procedure.

In the described embodiment, the line may be considered to be a bridle arrangement, wherein the bridle arrangement comprises a plurality of bridle lines. An end (i.e. a first end) of each bridle line will be fixed to a respective tubular cap member and extend from a central portion thereof such that, in use, each tubular cap member is configured to extend in a plane parallel to a surface of the soft body tissue when implanted in the soft body tissue and under tension of the bridle line when it is passing through the tissue in a direction away from the surface thereof. Each bridle line will be further fixed to the bridle point at a second end, or to another tubular cap member with a central portion of the bridle line instead connected to the common bridle point.

The bridle point may be a fixed bridle point such that the bridle lines cannot move relative to the bridle point at the connection. The bridle point may be a fixed knot, an eye plate or other suitable fastening.

Alternatively, the bridle point may be a slidable bridle point such that the bridle lines can move relative to the bridle point at the connection. The bridle lines may self-adjust, in a manner as described above. The bridle point may be a bridle knot, with a single, mutual line providing multiple bridle lines.

The artificial line may be connected to the bridle point, and may be configured to apply a tension force to the bridle point, i.e. place the bridle point under tension.

The artificial line may form one of the bridle lines. The remaining bridle lines may be joined to the artificial line at the bridle point, located along the artificial line.

The bridle point is preferably arranged to provide each of the plurality of bridle lines on a first side of the bridle point, with a single tension and/or artificial line located on a second side of the bridle point opposite the first side. Such a configuration may provide a more stable distribution of tensile force from the bridle point to each of the bridle lines.

The plurality of bridle lines may consist of two bridle lines, in which case the bridle arrangement may comprise a Y-shaped configuration. The plurality of bridle lines may consist of three bridle lines, in which case the bridle arrangement may comprise a trident-shaped configuration.

Viewed from a second aspect of the present invention, there is provided a soft tissue anchor system for implantation in soft body tissue to hold an artificial line. The anchor system comprises: a U-shaped fabric body comprising a base portion and at least two arm portions extending from the base portion; wherein each arm portion is configured to collapse in folds towards the base portion such that, in use, the body tissue is sandwiched between the base portion and each of the arm portions.

By providing a fabric body comprising two or more arm portions extending from the same base, the anchor system may be able to contact a greater surface area of body tissue when implanted, and hence may have improved stability and/or improved tissue ingrowth upon implantation. Further, by providing the collapsible arm portion(s) attached via a base portion, the base portion itself may further be able to provide a greater lateral surface by which to connect the anchor system and the leaflet upon implantation. A U-shaped anchor system, i.e. with at least two arm portions, may provide enhanced performance compared to the single arm portion.

Accordingly, a soft tissue anchor system as described above may have an improved securement strength compared to known anchor systems comprising a single arm portion and/or fixing member and/or using different design features.

Further, the use of a fabric body compared to a rigid body such as a metal body may reduce damage to the body tissue caused by the anchor during and/or after implantation. The fabric may better complement a surface of the body tissue that it contacts, thus spreading force exerted on the body tissue over a greater area and reducing trauma experienced by the body tissue at the site of implantation.

Collapsing of the arm portions in folds towards the base portion may be considered as a concertina motion. Accordingly, the arm portions may also be configured to concertina towards the base portion such that, in use, the body tissue is sandwiched between the base portion and the arm portions.

The fabric body, being formed of a fabric material, may be naturally disposed to fold and/or concertina because of the conformable nature of fabric. However, in some arrangements, the arm portions may comprise fold lines, narrowed sections, and/or weakened portions configured to aid in folding and/or concertinaing the arm portions.

In one arrangement, each arm portions may be configured to collapse in folds towards the base portion by action of a tension line threaded through the arm portions and the base portion. Where two arm portions are used then each can have its own tension line. Thus, the tension line may be advantageously configured to collapse the arm portion in folds towards the base portion when a tensile force is applied to the tension line. It will be appreciated that each tension line could act as implantable chords (i.e. the tension line may be the artificial line).

The tension line is preferably fixed to the arm portion at an end of the arm portion distal to the base portion. Thus, when a tensile force is applied to the tension line, the entirety of the arm portion may be configured to collapse in folds towards the base portion.

The location of the threading of the tensile line may facilitate the collapse of the arm portion in folds. For example, as the tension line is pulled, the tension line may draw holes in the arm portion through which the tension line is threaded together. Accordingly, the arm portion may be biased to fold in relation to each location the tension line is threaded through the arm portion.

Preferably, the anchor system is configured so that the tensile force is applied to a portion of the tension line(s) threaded through the base portion. This arrangement may facilitate the entire collapse of the arm portions.

The line may be threaded no more than three or four times through the arm portion and the base portion. This arrangement may facilitate the holes in the fabric body through which the line is threaded being further spaced apart, and/or a shorter fabric body being employed. Relative to the size of the anchor, each of these arrangements may provide wider folding portions of the fabric body that provide improved stability for the anchor system upon implantation. Using a shorter fabric body may also reduce the size of the overall soft tissue anchor system, thus improving its packaging within a delivery device/system for implantation.

The soft tissue anchor system may comprise the artificial line. That is, the fabric body may be in combination with the artificial line.

The artificial line may be fixedly joined to the line such that the relative positions of the line and the artificial line does not change.

The artificial line may be slidably joined to the portion of the tension line threaded through the base portion. The artificial line may be arranged to apply the tensile force to the tension line.

Alternatively, the artificial line may be slidably joined to the tension line by any suitable knot, for example a bridle knot. Alternatively, the artificial line may be slidably joined to the tension line by an intermediate member, such as an eyelet connected to the artificial line through which the tension line passes.

By enabling the artificial line to slide relative to the tension line, the point at which the tensile force is applied to the tension line may naturally adjust as the tensile force is applied. This may help spread the distribution of the tensile force across each arm portion, such that each arm portion is suitably retracted. Further, such an arrangement may facilitate implanting the fabric body in the body tissue at an angle with respect to the artificial line.

The fabric body may optionally comprise a stiffening member defining a plurality of holes through which the tension line is threaded. The stiffening member may for example be implemented as a tensile member, giving added tensile strength to the fabric body. The stiffening member may be regarded as a backbone to the fabric body, and may preferably comprise a complementary shape to the fabric body. The tension line is preferably threaded through the stiffening member in addition to the fabric body. The stiffening member may define holes through which the tension line is threaded.

The stiffening member may be embedded in the fabric body. The stiffening member may be interwoven with the fabric body. The stiffening member may be sandwiched between layers of the fabric body.

The stiffening member may be at least one high tensile strength line, interwoven in the fabric, to take excessive tensile loads that may be exerted on the fabric during implantation, retraction or along its lifecycle.

The stiffening member may reinforce the fabric body, and provide additional support at the points through which the tension line is threaded through the fabric body as well as optionally where the end caps are attached and at any point where there is narrowing of the fabric along its length. Accordingly, the stiffening member may improve the structural integrity of the fabric body.

Additionally or alternatively, the arm portions may be configured to collapse in folds towards the base portion due to the presence of a backbone formed of elastic material. The elastic material may be a shape memory metal, such as nitinol. The backbone may be in contact with at least each of the arm portions, and may further be in contact with the base portion. The backbone may be embedded or interwoven with the fabric body, and/or may be sandwiched between layers of the fabric body.

The backbone may comprise a folded configuration and an unfolded configuration. The backbone may be held in the unfolded configuration by application of a constraining force. In the unfolded configuration, the arm portions may extend away from the base portion. Upon removal of the constraining force, the backbone may return to the folded portion in which the backbone comprises one or more folds. The arm portions will occupy the same shape as the backbone, owing to the contact therebetween. Accordingly, the backbone may be a resiliently biased backbone configured to urge the arm portions in folds towards the base portion.

Where a backbone is employed, the line may be threaded through the arm portion.

Each of the arrangements of (i) the line being threaded through the arm portion; and (ii) the base portion and the backbone formed of an elastic material; as described above, may be considered as various means for collapsing the arm portion in folds towards the base portion.

In other words, viewed from another aspect of the present invention there is provided a soft tissue anchor system for implantation in soft body tissue to hold a line. The anchor system comprises: a U-shaped fabric body comprising a base portion and at least two arm portions extending from the base portion; and a means for collapsing each arm portion in folds towards the base portion such that, in use, the body tissue is sandwiched between the base portion and each of the arm portions.

Under an alternative wording, the means for collapsing the arm portion in folds towards the base portion may be equally considered as a mechanism for collapsing the arm portion in folds towards the base portion and/or a collapsing mechanism configured to collapse the arm portion in folds towards the base portion comprising at least one of the arrangements of (i) the line being threaded through the arm portion; and (ii) the base portion and the backbone formed of an elastic material; without departing from the aspect described above.

The artificial line may be joined to the fabric body via the base portion, using any suitable fastening arrangement.

The tension line and/or the artificial line may be formed of a suture material.

Each arm portion may comprise an end cap fixed at an end of each arm portion distal to the base portion. Each end cap may comprise an opening configured to engage a wire guide member for implanting the fabric body in the body tissue. The end cap may be regarded as a tubular cap member.

By providing end caps comprising openings configured to engage a wire guide member for implanting the fabric body, the arm portions may be manipulated such that they can be implanted through the body tissue. For example, the wire guide members can push the arm portions in a direction of their engagement such that the arm portions pass through the body tissue.

Further, by using wire guide members which guide the arm portions through the body tissue, rather than a needle from which the arm portions are deployed, a hole in the body tissue through which each arm portion is passed need not be as large as a hole required by a needle or other conduit containing the arm portions to pass through. That is, the size of the openings is constrained by no more than the geometry of the fabric body. In comparison, the size of openings which are required for implantation via a needle will always be constrained by the size of the needle which is always larger than the member it is to deploy. This may reduce trauma at the site of implantation.

The anchor system may comprise a tension line (e.g. as described above). The tension line may be fixed to the end cap and extends from a central portion thereof such that, in use, the end cap is configured to extend in a plane parallel to a surface of the body tissue when implanted in the soft body tissue and under tension of the line when it is passing through the tissue in a direction away from the surface thereof.

The end cap may comprise a first end, a second end distal to the first end, and a side wall extending between the first end and the second end. The opening may be located at a first end of the end cap. The second end of the end cap may define a tip of the tubular cap member, the tip being configured for insertion into the soft body tissue.

The central portion of the end cap may be considered as the portion of the end cap located around the geometric centre of the end cap (i.e. a central point along the longitudinal axis of the end cap), this portion comprising: less than 30% of the length of the end cap; less than 40% of the length of the end cap; less than 50% of the length of the end cap; or less than 60% of the length of the end cap. The central portion of the end cap may be considered to include the 'middle third' of the length of the end cap. In other words, the central portion of the end cap may comprise any point on the end cap that is closer to the geometric centre of the end cap than either the first end or the second end.

In some examples, the line may extend substantially centrally, i.e. substantially centrally from the centre of the end cap (e.g. in line with the geometric centre of the end cap).

In other examples, the line may extend asymmetrically, and may therefore extend from a point along the end cap more towards the first end or the second end of the end cap (yet still within the central portion of the end cap).

For example, asymmetric arrangements may locate the point from which the line extends from the end cap distally from the opening (i.e. towards the tip of the end cap). Having the line extend from a point located towards the tip of the end cap may facilitate the application of a torque to the end cap to place it in the "T" configuration. However, in other preferred examples, the point from which the line extends from the end cap proximally to the opening (i.e. towards the opening of the end cap). Having the line extend from a point located towards the opening of the end cap may better distribute a contact force exerted by the end cap when it extends in a plane parallel to the folds/surface of the body tissue when under tension of the line. The end cap may have a smaller projected area closer to the tip (e.g. due to a taper and/or bevel of the tip). The end cap may have a larger projected area closer towards the opening (e.g. due to a lack of taper/the end cap being wider towards the opening). Accordingly, placing the point from which the line extends from the end cap proximal to the opening may result in a more balanced distribution of contact force exerted by a profile of the tubular cap member, when it extends in a plane parallel to the folds/a surface of the body tissue.

Accordingly, the line may extend from the central portion of the end cap to which it is fixed from: substantially at the centre of the end cap; towards the first end of the end cap; or towards the second end of the end cap, as described above.

The manner in which the end of the line is fixed to the end cap may cover a length of the end cap. For example, a length of the end of the line may be wrapped circumferentially around the end cap or a portion thereof, with this length of line additionally extending along an axial length of the end cap (i.e. in a spiralled or coiled manner). The line extending from the end cap may extend from this coiled portion of line. Alternatively, a length of the end of the line fixed to the end cap or a portion therefor may extend substantially axially along the end cap or the portion thereof. The line extending from the end cap may extend from this length of line.

The end of the line may be fixed to the central portion of the end cap. However, the end of the line need not be restricted to being fixed to the central portion of the end cap, provided the line extends from the central portion of the end cap. Thus, in an alternative wording, the soft tissue anchor system may be considered to have an end of the line fixed to a central portion of the end cap, with this specific recitation of the end of the line referring to the portion of line initially extending from the end cap (i.e. being an 'end' portion of the line determining the position along the end cap at which, in use, tension can be applied by the line to the end cap).

Each end cap may comprise an outer tubular member and an inner tubular member, wherein the inner tubular member is configured to be received by the outer tubular member. That is, the inner tubular member may be nested within, and/or concentric with the outer tubular member. The inner tubular member may define the opening configured to engage the wire guide member.

The outer tubular member and the inner tubular member may be configured to sandwich, crimp and/or clamp the distal end of a respective arm portion therebetween, thereby fixing the end cap to the arm portion. Adhesives may be additionally or alternatively employed, to fix the end cap to the arm portion.

In one arrangement, the outer tubular member and the inner tubular member each define openings extending along the entirety of their length, i.e. they are hollow along the entirety of their length. The inner tubular member may be configured to allow passage of a piercing section of the wire guide member therethrough, and may also be configured to abut a shoulder portion, or bulge portion, of the wire guide member. A wire guide member comprising a piercing section, which is configured to pierce the body tissue during implantation of the anchor system, may be regarded as a piercing wire guide member.

By enabling passage of the piercing wire guide member through the inner tubular member, the end cap may comprise a dulled and/or rounded tip whilst still enabling piercing of the body tissue during implantation of the anchor system. Further, by providing an inner tubular member configured to abut a shoulder portion, or bulge portion, of the wire guide member, suitable contact for manipulation of the arm portions via the piercing wire guide member may be realised.

Moreover, the use of end caps which are suitable for use with a piercing wire guide member may further reduce trauma experienced by the site of implantation, because the piercing section of the wire guide member may have a smaller diameter than, for example, a hollow needle member making the incision for implantation.

The inner tubular member may extend out of the outer tubular member, and may define the tip of the end cap. This arrangement results in a step transition located at the tip of the end cap between the inner tubular member and the outer tubular member, which may facilitate smoother passage of the end cap through the body tissue during implantation.

Alternatively, the outer tubular member may comprise a tapered portion located at the tip of the end cap. That is, the tip of the end cap may be defined by the outer tubular member and an end of the inner tubular member may be contained within the outer tubular member. This arrangement results in a continuous transition located at the tip of the end cap between the tip of the end cap and the outer tubular member, which may facilitate smoother passage of the end cap through the body tissue during implantation.

The inner tubular member may comprise a flared inlet defining the opening configured to engage the wire guide member. The flared inlet will be understood to be a portion of the inner tubular member defining the opening, wherein a circumferential extent of the inner tubular member is increased relative to the rest of the inner tubular member.

Preferably, the flared inlet comprises an angled face to facilitate guiding of the wire guide member into the inner tubular member. This arrangement may be preferable where the wire guide member is not received by the opening of the tubular cap member prior to implantation.

The flared inlet may be configured to mate with a corresponding portion of the wire guide member. That is, the flared inlet may be complementary to a shape of the shoulder region, or bulge portion, of the piercing wire guide member. The angled face of the flared inlet may be complementary to the shoulder region, or bulge portion, of the wire guide member. The flared inlet may improve contact between the wire guide member and the end cap during implantation of the fabric body.

The inner tubular member may be retained within the outer tubular member via a press fit and/or a mating notch. The inner tubular cap member may also or alternatively be retained within the outer tubular member via a crimp operation.

The flared inlet may be configured to locate the inner tubular member within the outer tubular member. An end of the inner tubular member proximal to the opening may mate with the outer tubular member via a mating notch, a press fit and/or a crimp connection. The flared inlet may provide the press fit between the inner tubular member and the outer tubular member. An end of the inner tubular member distal to the opening may also mate with the outer tubular member via a mating notch, a press fit and/or a crimp connection. Providing at least two regions or points of retention may improve the retaining strength of the inner tubular member within the outer tubular member.

The outer tubular member may comprise a shoulder region configured to mate with the inner tubular member, distal to the opening. This may facilitate the locating of the inner tubular member within the outer tubular member, such that the opening defined by the inner tubular member is suitably located at the end of the tubular cap member.

A tip of the end cap distal to the base portion may be a dulled and/or rounded tip. By having a dulled and/or rounded tip, a risk of laceration and/or further trauma to the body tissue caused by the end cap, once implanted or otherwise, is mitigated.

The tip of the end cap may be located at and end of the end cap distal to the arm portion (i.e. at an end of the end cap distal to the opening configured to engage the wire guide member).

In an alternative arrangement, each end cap may comprise a pointed tip. The pointed tip may be configured to pierce the body tissue and, for example, the pointed tip may pierce the body tissue when a motive force is applied to the end cap by the wire guide member.

The tip of the end cap may be located at and end of the end cap distal to the arm portion (i.e. at an end of the end cap distal to the opening configured to engage the wire guide member).

The pointed tip may comprise a conical tip, i.e. a pointed tip having rotational symmetry about a longitudinal axis of the tubular cap member.

Alternatively, the pointed tip may have a rotationally asymmetric geometry about a longitudinal axis of the tubular cap member. Using a rotationally asymmetric tip geometry for the pointed tip may facilitate the pointed tip having a higher draft angle for a plane of its point (i.e. a plane of its shearing edge). Using a pointed tip with a higher draft angle may reduce the force required for the pointed tip to pierce the soft body tissue, and thus the trauma experienced at the implantation site during implantation.

To this end, the pointed tip may comprise a bevel plane. A bevel plane will be understood to define a single plane, i.e. a plane in which a face of the bevel is ground (e.g. a grinding plane) or otherwise formed.

The bevel plane may define a face of the pointed tip. The bevel plane may have a draft angle, relative to a radial plane of the tubular cap member, of: more than 50°, more than 60°, more than 70°, or more than 80°. The bevel plane may have a draft angle, relative to the longitudinal axis of the tubular cap member, of: less than 10°, less than 20°, less than 30°, or less than 40°.

The pointed tip may comprise a single bevel plane, and may be described as a bevel tip, or a chisel tip.

Alternatively, the pointed tip may comprise two lancet planes in addition to the bevel plane. The lancet planes may flank the bevel plane, preferably symmetrically. The bevel plane and each of the lancet planes will be understood to define a respective plane (such as a grinding plane). Such a tip geometry may be described as a lancet point tip. Therefore, the pointed tip may be a lancet point tip.

In arrangements where the opening configured to receive the wire guide member is comprises a blind bore, or a blind hollow, the pointed tip may be considered a pointed tip stylet. In arrangements where the opening configured to receive the wire guide member extends completely through the tubular cap member (where such an arrangement facilitates manufacture of the tubular cap member), the pointed tip may be considered a pointed tip cannula.

The pointed tip may be a retractable pointed tip. The tip of the end cap may have a first position in which the tip is a pointed tip, i.e. in which the pointed tip is deployed, and may have a second position in which the tip is a dulled and/or rounded tip, i.e. in which the pointed tip is retracted. The second position may be a rest position of the retractable pointed tip. The retractable pointed tip may be urged from the second position to the first position via engagement of the wire guide member with the end cap.

The outer tubular member may define the pointed tip. If the pointed tip is permanently present, the inner tubular member may comprise a blind bore which receives the wire guide member. If the pointed tip is a retractable pointed tip, the inner tubular member may be hollow, such that the wire guide member may actuate the retractable pointed tip and/or any suitable deployment mechanism.

The tension line may be fixed to the end caps. Each end cap may be configured to receive the tension line between the inner tubular member and the outer tubular member. The tension line may be fixed to each end cap by crimping, swaging, clamping, gluing and/or sandwiching the tension line between the inner tubular member and the outer tubular member. The tension line could also be fixed by stitching it or tying it to the tubular member.

The outer tubular member may comprise an opening formed in a side wall, the opening configured to receive the tension line. The opening may be formed in a central region of the end cap, and more preferably the opening may be formed towards the tip of the end cap.

Each end cap may be configured to extend collinearly with a respective arm portion during implantation and/or retrieval of the U-shaped fabric body. Each end cap may be configured to extend parallel to a plane of each fold of the respective arm portion when a tensile force is applied to the tension line.

As the arm portion may be fixed at an end of the end caps proximal to the arm portions, and as the tension line may be fixed at a central region and/or towards a tip of the end cap distal to the arm portion, tensile forces applied due to the arm portions and/or the tension lines may cause a torque to act on the end caps.

Accordingly, when the wire guide member engages the end cap, the end cap will straighten and extend collinearly with the arm portions. This may facilitate implantation of the arm portions of the fabric body in the body tissue. When the tension line experiences a tensile force and thus collapses the arm portions in folds, the tension line may turn the end caps to lie parallel with the folds of the arm portion, and hence parallel with a surface of the body tissue in which the arm portions fold against. This may facilitate securement of the arm portions to the body tissue, and may also minimise a profile of the end caps protruding from the body tissue during implantation. For example, the end cap may turn perpendicular to the tension line in a "T" configuration, with the tension line normal to the surface of the body tissue and the length of end cap parallel to the surface of the body tissue, preventing movement of the end cap through the body tissue. When the fabric body portion is retrieved from the body tissue, e.g. by being pulled from a direction distal to the end caps, a tensile force acting through the arm portions may self-right the end caps from the parallel position, such that they extend collinearly with the arm portions during retrieval. Thus, by coupling the arm portion to the end cap at an end thereof then tension from the arm portion will tend to straighten out the end cap (e.g. from the "T" configuration mentioned above) allowing it to align with the arm portion and hence align with the hole through the body tissue. This may facilitate retrieval and minimise any trauma experience by the body tissue during retrieval.

The end caps may be formed of a suitable rigid biocompatible material capable of retaining its shape, such as stainless steel, titanium, engineering plastics or nitinol. The end caps may be a single component (e.g. a hollow tube), where the tension line is tied or secured to the tube. The end caps may be formed as a single component by laser cutting or machining nitinol or stainless steel.

The outer tubular member of each end cap may comprise a groove, channel, aperture or other suitable feature for guiding the arm portions between the outer tubular member and the inner tubular member. This groove, channel, aperture or other suitable feature may be formed in circumferential alignment with the aperture, or opening, for the line, and may also be formed at or towards the opening configured to receive the wire guide member. Providing the line and the arm portion in alignment with one another may improve the stability of the soft tissue anchor system when implanted in soft body tissue.

Fixing the arm portion towards the opening of the end caps and the line at the central portion of the tubular cap member may facilitate the line applying a torque to the end caps when under tension, thereby causing the end caps to lie flat against the soft body tissue when implanted.

Fixing the arm portion towards and/or at the opening of the end caps may also facilitate retrieval of the end caps. For example, upon retrieval of the soft tissue anchor system, tension in the line may be relaxed. The arm portion/fabric body may then be pulled so as to apply tension. This tension may result in a torque being applied to the end caps, causing the end caps to rotate such that the extend in a plane perpendicular to the surface of the soft body tissue (e.g. such that it extends collinearly with the line). The tubular cap member may then be pulled and/or guided back through the soft body tissue. This arrangement may therefore facilitate retrieval of the end caps during retrieval of the soft tissue anchor system, in a manner that also minimises any trauma experienced by the body tissue during retrieval.

Alternatively, the aperture formed in the side wall of the outer tubular member receiving the line may also receive the arm portion. Accordingly, the fixing of the arm portion and the line to the end caps may be coincidental.

Alternatively, and as may be preferable in arrangements where the wire guide members are received by the openings of the end caps prior to implantation, the outer tubular member may not comprise a channel for guiding the arm portion between the outer tubular member and the inner tubular member and/or a flared inlet defining the opening configured to receive the wire guide member. This may result in the end caps being made shorter and/or thinner (i.e. narrower in diameter), and hence more compact, for arrangements where the wire guide member need not be guided into engagement with the end caps during implantation.

The fabric body may be formed of a single layer of fabric or multiple layers of fabric. In this context, a fabric may be considered as a material formed as a weave made of threads or threadlike forms. One possible material is a polyester fabric, other materials include fabrics of PET, UHMPE, EPTFE, PTFE and similar. The fabric body may be formed of any suitable flexible, conformable and biocompatible material. The fabric body may be formed by laser cutting an existing material or any other suitable manufacturing technique.

In the example herein using at least two arm portions the U-shaped body is so-called due to the U-shape formed by the base portion and two arm portions. However, it will be appreciated that this merely refers to the unit structure of the fabric body with two arm portions and the shape will differ if more arm portions are present. For example, a fabric body comprising three arm portions may be regarded as a W-shaped body, wherein the W-shaped body comprises two U-shaped units. Accordingly, the anchor system may comprise a fabric body comprising multiple U-shaped units such that a fabric body of suitable size is arrived at for the desired purpose of the soft tissue anchor system.

The base portion may be the widest part of the fabric body. That is, the base portion may define a maximum width of the fabric body.

The base portion may comprise a pair of wing portions. The wing portions may extend wider than an outermost edge of the arm portions.

By providing a base portion of increased width, or at least greater width than compared to the arm portions, the base portion may be configured to provide greater lateral support to the fabric body upon implantation in the body tissue.

When the anchor is in use then the base portion may be implanted on the atrial side of the leaflet, where the retention is required during a heartbeat. In this context an increased lateral support for the atrial side location can be beneficial to achieve a wide area of support of the leaflet. If the base portion is at the atrial side then the arm portions are on the ventricle side. They act to hold the anchor system in place and provide some support, while the atrial side portion (base portion) is more active in carrying forces exerted by blood flow on the leaflets during heart contraction (systole).

The base portion may comprise a shape retention member. The shape retention member may be configured to increase a stiffness of the base portion. The shape retention member may be configured to maintain a planar extent of the base portion, or a 3D shape that conforms with the shape of the leaflet/anatomy.

By stiffening the base portion using a shape retention member, the base portion may be able to provide greater lateral support to the body tissue in which the fabric body is implanted. Further, tensile forces experienced by the fabric body during implantation when holding the artificial line, may be more evenly distributed across the base portion when a shape retention member is provided. This may improve the stability of the fabric body upon implantation.

The shape retention member may be formed of an elastic material. The base portion may comprise a folded configuration and an unfolded configuration, wherein the shape retention member is configured to urge the base portion from the folded configuration to the unfolded configuration upon removal of a constraining force. The base portion may be substantially planar in the unfolded configuration.

The shape retention member may be embedded, interwoven with, and/or sandwiched between layers of, the base portion.

The base portion may be formed by folding a portion of the fabric body back over on itself, and sandwiching the shape retention member therebetween. The shape retention member and/or the folded portion of the fabric body may be fixed in place by an adhesive, ultrasonic welding, stiches or other suitable fixing means.

The shape retention member may comprise a fluorescent marker. The fluorescent marker may assist in locating the fabric body via imaging during implantation and/or retrieval.

The shape retention member may be formed of a shape retention metal such as stainless steel, titanium or nitinol. The shape retention member may be formed by laser cutting, or by shaping a wire formed of a similar material.

The base portion may provide a surface for retrieving the fabric body following implantation. The U-shaped fabric body may be retrieved by pulling the arm portions out of the body tissue by grasping the base portion, and pulling the base portion away from the body tissue. The shape retention member may improve ease of retrieval, as the shape retention member may help distribute tensile forces experience across the base portion during retrieval.

The shape retention member in the base portion may also prevent the arm portions from over extending through the leaflet when being deployed, as it can act as a stopper while the arms are being pulled through the tissue by the guide wires - in that way the base portion combined with the shape retention member may act to place a limitation to how far the deployment wires can go during deployment.

The base portion may be configured to be grasped by a snare, and optionally the base portion may be located using the fluorescent marker. For example, the snare may encircle the arm portions where they initially extend from the base portion, on the same side of the body tissue as the base portion when implanted. Then, the snare may be tightened such that, upon retraction, the snare grasps the base portion. The pair of wing portions may provide a widened surface of the base portion by which the base portion may be grasped by the snare.

The arm portions may comprise narrow waist portions extending between the base portion and each arm portion. Upon implantation, the narrow waist portion may be aligned with the soft body tissue such that the narrow waist portion is encircled by the soft body tissue at the implantation site. The narrow waist portion may be configured to be longer than a thickness of the soft body tissue in which the soft tissue anchor system is to be implanted. For example, a length of the narrow waist portion may be: greater than at least 1mm; greater than at least 2mm; or greater than at least 3mm. The length of the narrow waist portion may be between 1 and 3 mm. Each narrow waist portion may equally be considered as part of a respective arm portion, such that the arm portions each comprise a narrow waist portion at an end of the arm portion proximal to the base portion, or alternatively may be considered its own respective portion as described above. The narrow waist portions may stabilise the implantation of the base portion adjacent to the body tissue. This feature also may aid in holding the arm portions in place prior to tensioning the tension line.

Each arm portion may be provided with its own respective line. For example, in some arrangements where the soft tissue anchor comprises at least two arm portions, the artificial line may be the line for one of the arm portions, and for the remaining arm portions additional lines may be employed, each fixed to the artificial line (in sliding engagement or a steadfast engagement). Additionally, or alternatively, two of the arm portions may comprise a mutual line, this line joined to the artificial line/the line of another arm portion adjacent to the base portion. A first end of the line may be joined to the end cap of the first arm portion, and a second end of the line may be joined to the end cap of the second arm portion. A central portion of the line may then be fixed to the artificial line/any other lines.

Where the line is threaded through the arm portions, for each arm portion there may be provided: a hole in the base portion; a hole in the narrow waist portion; and at least one hole in the arm portion. The hole in the narrow waist portion may be located closer to the base portion that the respective arm portion (i.e. the hole may be located proximal to the base portion). This arrangement may improve compression of the fabric body around the soft body tissue, since the placement of a hole in the narrow waist portion proximal to the base portion encourages the collapse of the base portion towards the soft body tissue/towards the collapsing arm portions. Accordingly, the stability of the anchor system upon implantation may be improved.

Where a narrow waist portion is not provided, for each arm portion(s) there may be provided: a hole in the base portion; and at least two holes in the arm portion.

The base portion combined with the shape retention member may assist with placing the soft tissue anchor system in locations over at a wider area (narrow and deep) of the leaflet while still achieving leaflet support.

The tension line may comprise a plurality of bridle lines, with each bridle line being associated with (i.e. threaded through the base portion and) a respective arm portion.

Each bridle line is preferably connected to a common bridle point, wherein the bridle point is configured to place each bridle line under tension when a tensile force is applied to the bridle point. That is, the bridle point is configured to apply a tensile force to each of the bridle lines when the bridle point itself is under tension.

By providing an arrangement comprising a plurality of bridle lines, each placed under tension due to a common bridle point, the arm portions (and/or end caps, if present) of the soft tissue anchor may each experience a tensile force pulling them towards a plane or axis intersecting the bridle point and in the direction of the tensile force applied to the bridle point. This may result in each of the arm portions being motivated towards one another, and in turn capturing, gathering and/or pinching together any excess body tissue located between the arm portions upon implantation. Accordingly, this arrangement may restore the shape of the soft body tissue and/or provide additional structural support to the body tissue.

The arrangement comprising a plurality of bridle lines each connected to a common bridle point may be particularly advantageous when the soft body tissue is a heart valve leaflet. The reshaping of the tissue of the leaflet resulting from capturing excess leaflet tissue as described above has been found to provide an outcome similar to resection of the leaflet. Resection is a common surgical step in existing methods of heart valve repair, in which a surgeon resects a damaged section of the heart valve and stitches the edges of the remaining tissue together. Resection is often performed where there is excessive leaflet tissue present. However, by providing a line arrangement comprising a bridle point and a plurality of bridle lines, the need for a resection step may be obviated. This could simplify the overall surgical procedure.

In the described embodiment, the line may be considered to be a bridle arrangement, wherein the bridle arrangement comprises a plurality of bridle lines. Each bridle line will be threaded through each of the arm portions and the base portion (and an end of each bridle line may be fixed to a respective end cap), with each bridle line configured to collapse each arm portion in folds towards the base portion when a tensile force is applied to the bridle line by the bridle point. Each bridle line will be connected to the bridle point at a location on an opposite side of the base portion to the arm portions.

The bridle point may be a fixed bridle point such that the bridle lines cannot move relative to the bridle point at the connection. The bridle point may be a fixed knot, an eye plate or other suitable fastening.

Alternatively, the bridle point may be a slidable bridle point such that the bridle lines can move relative to the bridle point at the connection. The bridle lines may self-adjust, in a manner as described above. The bridle point may be a bridle knot, with a single, mutual line providing multiple bridle lines.

The artificial line may be connected to the bridle point, and may be configured to apply a tension force to the bridle point, i.e. place the bridle point under tension.

The artificial line may form one of the bridle lines. The remaining bridle lines may be joined to the artificial line at the bridle point, located along the artificial line.

The bridle point is preferably arranged to provide each of the plurality of bridle lines on a first side of the bridle point, with a single tension and/or artificial line located on a second side of the bridle point opposite the first side. Such a configuration may provide a more stable distribution of tensile force from the bridle point to each of the bridle lines.

The plurality of bridle lines may consist of two bridle lines, in which case the bridle arrangement may comprise a Y-shaped configuration. The plurality of bridle lines may consist of three bridle lines, in which case the bridle arrangement may comprise a trident-shaped configuration.

The soft tissue anchor systems of any of the above-discussed aspects may be a valve anchor system for implantation in a heart valve leaflet to secure an artificial chordae line. The heart valve leaflet may be a mitral valve leaflet or a tricuspid valve leaflet.

Viewed from a third aspect, there is disclosed a soft tissue anchor system for implantation in soft body tissue to secure an artificial line, the anchor system comprising: a plurality of anchor members; and a bridle arrangement comprising a plurality of bridle lines and a common bridle point; wherein each bridle line is associated with a respective anchor member and connected to the common bridle point; wherein the bridle point is configured to place each bridle line under tension when the soft tissue anchor system is implanted in the soft body tissue and the bridle point itself is under tension, such that each anchor member is motivated towards an axis intersecting the bridle point and a direction in which the tensile force is being applied to the bridle point.

By providing a bridle arrangement comprising a plurality of bridle lines, each placed under tension due to a common bridle point, the anchor members of the soft tissue anchor system may each experience a tensile force pulling them towards a plane or axis intersecting the bridle point and in the direction of the tensile force applied to the bridle point. This may result in each of the anchor members being motivated towards one another, and in turn capturing, gathering and/or pinching together any excess body tissue located between the anchor members upon implantation. Accordingly, this arrangement may restore the shape of the soft body tissue and/or provide additional structural support to the body tissue.

The bridle arrangement comprising a plurality of bridle lines each connected to a common bridle point may be particularly advantageous when the soft body tissue is a heart valve leaflet. The reshaping of the tissue of the leaflet resulting from capturing excess leaflet tissue as described above has been found to provide an outcome similar to resection of the leaflet. Resection is a common surgical step in existing methods of heart valve repair, in which a surgeon resects a damaged section of the heart valve and stitches the edges of the remaining tissue together. Resection is often performed where there is excessive leaflet tissue present. However, by providing a line arrangement comprising a bridle point and a plurality of bridle lines, the need for a resection step may be obviated. This could simplify the overall surgical procedure.

The soft tissue anchor system may be a leaflet anchor system. The soft body tissue may be a leaflet heart valve. The artificial line may be an artificial chordae line.

Each anchor member may be configured to be passed through the soft body tissue, with the bridle line passing through the soft body tissue. As such, the bridle point may be configured to place each bridle line under tension when each bridle line is passing through the tissue in a direction away from the surface of the body tissue and when the bridle point itself is under tension.

The bridle point may be a fixed bridle point such that the bridle lines cannot move relative to the bridle point at the connection. The bridle point may be a fixed knot, an eye plate or other suitable fastening.

Alternatively, the bridle point may be a slidable bridle point such that the bridle lines can move relative to the bridle point at the connection. The bridle lines may self-adjust, in a manner as described above. The bridle point may be a bridle knot, with a single, mutual line providing multiple bridle lines.

The artificial line may be connected to the bridle point, and may be configured to apply a tension force to the bridle point, i.e. place the bridle point under tension.

The artificial line may form one of the bridle lines. The remaining bridle lines may be joined to the artificial line at the bridle point, located along the artificial line.

The bridle point is preferably arranged to provide each of the plurality of bridle lines on a first side of the bridle point, with a single tension and/or artificial line located on a second side of the bridle point opposite the first side. Such a configuration may provide a more stable distribution of tensile force from the bridle point to each of the bridle lines.

The plurality of bridle lines may consist of two bridle lines, in which case the bridle arrangement may comprise a Y-shaped configuration. The plurality of bridle lines may consist of three bridle lines, in which case the bridle arrangement may comprise a trident-shaped configuration.

Each anchor member may be a tubular cap member. The tubular cap member may be of the form as described above, as according to the first aspect for example.

Each anchor member may be an arm portion and/or an end cap. The arm portion and/or end cap may be of the form as described above, as according to the second aspect for example.

The plurality of anchor members may comprise tubular cap members and arm portions and/or end caps.

Whilst the soft tissue anchor systems of the above-discussed aspects may have particular advantage and application in the treatment of the heart, unless stated otherwise their application should not be considered limited to the treatment of soft heart tissue.

The soft tissue anchor system may be deployed via a suitable deployment device, advantageously a catheter device. The deployment device may comprise an anchor deployment mechanism that holds and/or guides the soft tissue anchor system during deployment, e.g. during piercing of the body tissue and implantation of the arm portion(s) of the fabric body. The anchor deployment mechanism, or another part of a catheter device that holds the anchor deployment mechanism, may also act to remove the wire guide member(s) and/or tension the tension line in order to place the arm portion(s) and end cap(s) into their final position, e.g. with the arm portion(s) folded and the end cap(s) turned to sit along the surface of the body tissue.

Thus, viewed from a fourth aspect of the present invention, there is provided a catheter device for implanting a soft tissue anchor system in heart tissue, the catheter device comprising: a housing section, wherein the housing section extends from a distal end of the catheter device along the length of the catheter device toward a proximal end of the catheter device; and a soft tissue anchor system as described in any of the preceding aspects located within the housing section. In accordance with the present invention, the soft tissue anchor system is as described according to the second aspect. In variations of the fourth aspect, the soft tissue anchor system is as described according to any of the other preceding aspects.

The catheter device of the fourth aspect may have one or more features corresponding to those of the soft tissue anchor systems of the second aspect of the invention, or of the remaining aspects as disclosed. Thus, the above-description of the soft tissue anchor systems of the preceding aspects, including but not limited to all technical advantages and alternative embodiments, may be equally applicable to the catheter device of the fourth aspect.

The catheter device may comprise a wire guide member(s) for deploying the soft tissue anchor system from the housing section. Where the soft tissue anchor system comprises end cap(s), the catheter device may comprise a wire guide member(s) in engagement with the opening.

The wire guide member may comprise a shoulder portion. The housing section may comprise a stopping portion configured to mate with the shoulder portion and thereby limit a distal translation of the wire guide member in the housing section.

By limiting a distal translation of the wire guide member, an overextension of the wire guide member may be prevented from occurring during implantation of the soft tissue anchor system. For example, where the catheter device experiences curvature during delivery to the implantation site the path of the wire guide member may be shortened or lengthened relative to the longitudinal axis of the catheter device. Accordingly, by limiting the distal translation of the wire guide member in the housing section, a final deployment length for the wire guide member may be better controlled. This may be particularly advantageous in ensuring that, where multiple wire guide members are used, the wire guide members extend to the same final length to facilitate a stable and symmetric implantation of the end caps and/or arm portions in the body tissue.

Thus, the wire guide member may comprise the shoulder portion for limiting a distal translational range of the wire guide member in the housing section (i.e. a first shoulder portion), and the corresponding portion/shoulder region configured to mate with/engage the end cap(s) for implanting the anchor system in the body tissue (i.e. a second shoulder portion).

Viewed from a fifth aspect of the present invention, there is provided a catheter device for implanting a soft tissue anchor system in heart tissue, the catheter device comprising: a housing section, wherein the housing section extends from a distal end of the catheter device along the length of the catheter device toward a proximal end of the catheter device; a soft tissue anchor system comprising a plurality of anchor members; and
a deployment system configured to simultaneously implant each of the anchor members in the heart tissue; wherein the deployment system comprises a plurality of wire guide members, wherein each wire guide member comprises a guide portion located at a distal end of the wire guide members and configured to engage a respective anchor member; and wherein the deployment system is configured to maintain a coplanar alignment between the guide portions of the plurality of wire guide members during implantation of the anchor members.

In accordance with the present invention, the soft tissue anchor system is a soft tissue anchor system according to the second aspect. Accordingly, the catheter device of the fifth aspect may have one or more features corresponding to those of the soft tissue anchor systems of the second aspect and other related aspects of the invention.

When implanting a soft tissue anchor system comprising a plurality of anchor members in heart tissue, the inventors have recognised that it can be desirable to implant each of the anchor members simultaneously in the heart tissue. Simultaneously implanting the anchor members may improve the stability of the implanted system, since the anchor members may be deployed in an identical and better controlled manner. Additionally, simultaneously implanting the anchor members may reduce the complexity of the implantation procedure.

However, the inventors have further recognised that during delivery of a soft tissue anchor system to the heart via a catheter device, the delivery shaft of the catheter device may experience a curvature due to the curvature of the blood vessels through which the catheter device traverses. Such curvature can result in various components of the catheter device having to traverse varying distances, according to the radius of curvature they experience during delivery to the heart. This may pose a problem for wire guide members used to implant anchor members of the soft tissue anchor system in the heart tissue. If one wire guide member traverses a greater distance than the other, anchor members each deployed by a respective wire guide member may not be implanted in the heart tissue at the same time, since the wire guide members may become asymmetrically tensioned or positioned during their delivery to the heart. This may affect the ability of the deployment system to simultaneously implant the anchor members in the heart tissue.

Accordingly, the deployment system is configured to maintain a coplanar alignment between the guide portions of the plurality of wire guide members during implantation of the anchor members. As such, regardless of the curvature the wire guide members each independently experience during delivery, the end portions of the wire guide members that ultimately manipulate the anchor members during implantation will remain aligned such that the anchor members will be simultaneously implanted. By controlling the coplanar alignment of the guide portions of the wire guide members, any possible deflection of the wire guide members due to differing curvatures can be restored prior to their engagement with the respective anchor members.

By being implanted simultaneously, it will be understood that the anchor members themselves are implanted in the heart tissue coincidentally, further to being implanted in the heart tissue during the same action or motion.

The guide portions will be understood to be in coplanar alignment when equivalent points or portions thereof, such as the ends of the wire guide members themselves, are located in substantially the same plane as one another, said plane defined perpendicularly to a longitudinal axis of the catheter device or the wire guide members themselves. The guide members may be considered to be in coplanar alignment when they are coincident with the same plane, or when they are within the same plane with a tolerance of: less than 0.5mm; less than 1.0mm or less than 1.5mm.

Each wire guide member comprises a proximal end and a distal end. The portion located at the distal end is the guide portion configured to engage a respective anchor member, as discussed above. Each wire guide member may also comprise a control portion, extending from the guide portion and located towards and/or at the proximal end.

The guide portion may comprise a pointed tip configured to pierce the heart tissue during implantation. Alternatively, the guide portion may be configured to be received by an opening of the anchor member, such that the guide portion provides a motive force to the anchor member which itself pierces the heart tissue.

The control portion may be thicker, i.e. have a greater diameter, than the guide portion. The control portion will generally extend from the housing of the catheter device and through a delivery shaft of the catheter device. The control portion may extend to a delivery handle of the catheter device, the delivery handle located at the proximal end of the catheter device.

The guide portion may have a diameter (i.e. an outer diameter) of approximately 0.3mm. The control portion may have a diameter (i.e. an outer diameter) of approximately 0.5mm.

Each wire guide member may comprise a bulge portion.

The bulge portion is preferably located between the guide portion and the control portion, and has a diameter greater than both the guide portion and the control portion.

The bulge portion may have a diameter (i.e. an outer diameter) of approximately 0.8mm. The bulge portion may have a diameter between 0.6 and 0.9mm.

The bulge portion may have a length approximately equal to its diameter. The bulge portion may have a length of approximately 0.8mm.

The bulge portion has a bulbous shape. The bulbous shape preferably comprises a central portion defining the maximum diameter of the bulge portion, and two tapered/transition portions located either side of the central portion.

Each wire guide member may be movable between a first configuration and a second configuration, wherein in the first configuration the bulge portion is configured to engage a first obstruction configured to limit a translation of the wire guide member in the proximal direction, and wherein in the second configuration the wire guide member is configured to engage a respective anchor member.

By limiting the translation of the wire guide members in the proximal direction, the wire guide members are prevented from being asymmetrically withdrawn into a delivery shaft of the catheter device during deployment (e.g. due to extension of tension experienced due to curvature during delivery). The first obstruction also provides a reference point by which the coplanar alignment of the wire guide members may be restored, or maintained, during delivery of the catheter device to the heart. For example, the guide portions of the wire guide members may each be in coplanar alignment with one another when the bulge portions abut the first obstructions. Coplanar alignment of the guide portions of the wire guide members may be restored by automatically or manually moving each of the wire guide members to the first configuration. Thus, the first obstructions will themselves understandably be in coplanar alignment with one another.

The first obstruction may define a channel or groove through which the control portion may pass, but not the guide portion.

The first obstruction may be an abutment portion located in a proximal part of the housing section.

By placing the first obstruction in the proximal part of the housing, the point at which coplanar alignment may be maintained or restored is located distally to any portion of the wire guide member that may experience curvature during delivery.

The guide portions of the plurality of wire guide members are configured to be in coplanar alignment when the bulge portions abut the first obstructions.

The abutment portion may be complementary to a shape of the bulge portion, such that the bulge portion is configured to mate with the abutment portion in the first configuration.

In the second configuration, the bulge portion may be configured to abut a complementary portion of the anchor member.

The complementary portions may be the tapered/transition portions of the bulge portion.

The bulge portion may therefore provide a surface or portion by which the wire guide members may provide a motive force to the anchor members.

The bulge portion itself may act as a shoulder portion configured to engage a flared inlet of a tubular cap member or an end cap, as described in the first and second aspects respectively, as well as any related aspects.

Alternatively, in the second configuration, the bulge portion may be configured to abut a second abutment portion located in a distal part of the housing section, wherein the second abutment portion is configured to limit a translation of the wire guide member in the distal direction.

Providing a second abutment portion in the distal part of the housing section may prevent an overextension of the wire guide members during implantation of the soft tissue anchor system.

The second abutment portion may comprise a channel or groove through which the control section and the guide portion may pass, but not the bulge portion.

In such an arrangement, the bulge portion may be located within the control portion, rather than between the control portion and the guide portion.

The wire guide member may comprise a shoulder portion extending between the control portion and the guide portion. The shoulder portion may define a sloping transition between the control portion and the guide portion. The shoulder portion may be configured to abut, or engage, a complementary portion of the anchor member to thereby provide a motive force to the anchor member during implantation.

The deployment system may be configured to passively maintain coplanar alignment between the guide portions by manually restoring the guide portions to be in a common plane. For example, a user may pull each of the wire guide members upon delivery of the catheter device to the heart tissue, with each of the first obstructions providing a surface by which to restore coplanar alignment.

Alternatively, the deployment system may be configured to actively maintain coplanar alignment between the guide portions by automatically restoring the guide portions to be in a common plane.

The deployment system may comprise a spring arrangement. Each wire guide member may be connected to the spring arrangement. The spring arrangement may be configured to automatically restore the coplanar alignment between the guide portions of the plurality of wire guide members during delivery of the catheter device to the heart. The spring arrangement may be an example of an arrangement configured to actively maintain coplanar alignment between the guide portions.

The spring arrangement may provide one or more control inputs to the plurality of wire guide members.

Preferably, the spring arrangement configured to bias each wire guide member towards being in the first configuration.

By providing a spring arrangement configured to bias each wire guide member towards being in the first configuration, the deployment system may automatically restore coplanar alignment between the guide portions of the wire guide members during delivery of the catheter device to the heart.

The spring arrangement may comprise a plurality of spring members each selectively connected to a respective wire guide member. Each spring member may be configured to provide a restoring force to a respective wire guide member.

The spring arrangement may be configured to adjust a relative tension between each wire guide member. Adjusting the relative tension between each of the wire guide members may maintain a coplanar alignment of the guide portions, despite any deflection or misalignment of the control portions or otherwise during delivery of the catheter device.

The spring arrangement may comprise a tension adjustment mechanism for relaxing tension in each of the wire guide members, and a tension restorer for relaxing, or balancing, the relative between each of the wire guide members. The tension adjustment mechanism may relax a tensioning force experienced by any wire guide members experiencing tension due to deflection or curvature, such tension potentially experienced as a pulling force due to the first obstruction preventing withdrawal of the wire guide members into delivery shaft. The tension restorer may relieve tension by adjusting a lateral force on each of the wire guide members, i.e. a force applied in a direction that extends perpendicular to the longitudinal extent of the wires in the spring arrangement. The tension restorer may hence relieve any tension by the principle of bow tension, where variation in a lateral displacement of the wire increases or decreases the longitudinal tension in the wire guide members.

The spring arrangement may be located in a delivery handle of the catheter device.

The plurality of wire guide members may be configured to be simultaneously translated by a single control input. The single control input may be configured to selectively engage the plurality of wire guide members. Once a coplanar alignment of the wire guide members is restored (or provided a coplanar alignment of the wire guide members is maintained) during delivery of the catheter device, an equal and/or symmetrical longitudinal translation will effect an equal, and thus simultaneous, manipulation of the wire guide members to simultaneously implant the anchor members.

The single control input may comprise a first set of gears configured to transmit a translational input, and a second set of gears configured to translate each of the wire guide members. The first set of gears are complementary to the second set of gears, and are selectively engageable. The gears will preferably engage one another once the catheter device is delivered to the heart tissue, prior to implantation. The first set of gears are simultaneously operable, such that each of the wire guide members is simultaneously translated by the single control input.

The spring arrangement may be configured to be selectively disconnected from the plurality of wire guide members prior to controlling and/or engagement the single control input. The spring arrangement may be configured to be selectively reconnected, e.g. prior to retraction of the catheter device.

The spring arrangement may be provided as a part of the single control input.

Each anchor member may be an end cap of a soft tissue anchor system.

The following features may be features of the catheter device of the fourth aspect and/or the fifth aspect.

The catheter device may comprise an anchor deployment mechanism that holds and/or guides the soft tissue anchor system during deployment, e.g. during piercing of the body tissue and implantation of the arm portion(s) of the fabric body. The anchor deployment mechanism, or another part of a catheter device that holds the anchor deployment mechanism, may also act to remove the wire guide member(s) and/or tension the tension line in order to place the arm portion(s) and/or end cap(s) into their final position, e.g. with the arm portion(s) folded and/or the end cap(s) turned to sit along the surface of the body tissue.

The soft tissue anchor system may be held within a curtain or sheath in the catheter device. The sheath may be configured to deflect, or crumple, during deployment to aid deployment of the soft tissue anchor system from the catheter device. The sheath may reduce friction between the soft tissue anchor system and the catheter device during deployment of the soft tissue anchor system from the catheter device.

The sheath may be a thin tubular sheath.

Where the soft issue anchor system comprises a fabric body, the sheath may be used to hold the fabric body. Where the soft tissue anchor system comprises tubular cap members/end caps, said members/caps may be held outside or within the sheath.

The sheath may be housed within the anchor deployment mechanism.

The sheath may be attached or secured to the anchor deployment mechanism, such that it does not release from the catheter device during deployment of the soft tissue anchor system from the catheter device.

The anchor deployment mechanism may comprise an anchor deployment tube that holds and guides part(s) of the soft tissue anchor system, such as the arm portion(s) and/or end cap(s), and wire guide member(s).

In the case where multiple arm portions are used, such as with a U-shaped fabric body and two arm portions, then the anchor deployment tube may comprises a pair of tubes, one for each arm portion and its respective wire guide member, with a join between the tubes allowing for the connective bridge of fabric from the base portion between the arm portions to span between the tubes. For example, there may be slots along the tubes for the connective bridge to slide along.

Similarly, in cases where soft tissue anchor systems comprising two tubular cap members/T-bar anchors are used, each connected via a mutual line (e.g. a single line extending between the respective central portions of two tubular cap members, joined to an artificial line in a central portion of the line; or two lines each connected to the central portion of a respective tubular cap member and fixed to one another distally to the ends thereof, for example), then the anchor deployment tube can similarly comprise a pair of tubes, one for each tubular cap member (i.e. T-bar anchor) and its respective wire guide member. There may also be a join between the tubes allowing for the mutual line/fixing between the two lines between the two T-bar anchors to span between the tubes. For example, there may be slots along the tubs for the mutual line/fixing to slide along.

The sheath may be held within the anchor deployment tube, and may extend to the tubes housing the end caps/tubular cap members.

The soft tissue anchor system can be a leaflet anchor deployed via the catheter device. One possible catheter device is a device configured for repair of the heart by implanting an artificial chordae line, for which the soft tissue anchor system acts as a leaflet anchor for joining the artificial chordae line to a leaflet of the heart. In an example, the catheter device comprises: a housing section extending from a distal end of the catheter device along the length of the catheter device toward a proximal end of the catheter device; a leaflet anchor (comprising the soft tissue anchor system) for placement in a leaflet of a heart valve, wherein the leaflet anchor is arranged to be coupled to the artificial chordae line; and a leaflet anchor deployment mechanism for deploying the leaflet anchor to attach it to the leaflet of the heart.

The leaflet anchor deployment mechanism may comprise a mechanical gripper device for grasping the leaflet of the heart valve, and a leaflet anchor tube for housing the leaflet anchor before deployment into the body tissue; the gripper device and leaflet anchor being arranged such that when, in use, the gripper device grasps the leaflet, the soft tissue anchor system can be pushed out of a leaflet anchor tube to pierce the leaflet and deploy the anchor as described elsewhere herein, so that it will secure the fabric body in the leaflet.

The mechanical gripper device may include a gripper arm rotatably coupled to a main body of the catheter device so that the gripper arm can rotate relative to the catheter device to move an outer end of the gripper arm away from the main body of the catheter device.

The gripper arm may comprise a slot formed in a base of the gripper arm. The gripper arm may be rotatably coupled to the main body via the slot, such that the gripper arm is configured to translate away from the main body of the catheter device. This translation will be comprised in/in addition to the rotational motion of the gripper arm described above.

By allowing the gripper arm to translate away from the main body, a space between the main body of the catheter device and the gripper arm may be provided and adjusted. This may provide an improved grip on the grasped leaflet. For example, a leaflet may have a thickness of between 1 to 3 mm in humans, and thus being able to translate the gripper arm away from the main body of the catheter device to accommodate leaflets of varying thickness may improve contact of the leaflet between the main body of the catheter device and the gripper arm.

Additionally, by providing a means by which the gripper arm may translate away from the main body of the catheter device, the leaflet/soft body tissue may be more smoothly disengaged by the gripper arm since the entire gripper arm face may ungrasp the grasped leaflet in one translational motion; before then being rotated to release the leaflet from between the main body of the catheter device and the gripper arm altogether.

The leaflet anchor tube may be formed in the main body of the catheter device. The leaflet anchor may thus be configured to be deployed from the main body of the catheter device and towards the gripper arm grasping the leaflet.

The gripper arm may comprise a plurality of serrations configured to increase an area of contact between the leaflet and the gripper arm. The serrations may be located on a first portion of the gripper arm configured to face the opening of the leaflet anchor tube, and on a second portion of the gripper arm configured to face the main body of the catheter device.

By providing serrations surrounding the gripper arm in this manner, the gripper arm may provide an improved grasping contact between itself and the leaflet. For example, the serrations may effectively circumferentially surround the face of the gripper arm configured to contact the leaflet. Accordingly, the leaflet may be better supported by the gripper arm.

The main body may also comprise a plurality of serrations, formed on a surface configured to face the gripper arm. Providing serrations on both gripper arm and the main body of the catheter device may further improve the force with which the gripper arm grasps the leaflet.

The serrations are preferably dulled so as to minimise trauma experienced by the leaflet when grasped. The serrations may be unevenly and/or evenly distributed undulations in the surface of the gripper arm and/or the main body of the catheter device that increase a surface area thereof, compared to e.g. a flat/planar surface.

The gripper arm may comprise an internal space configured to receive the arm portion(s) and/or end cap(s) and the wire guide member(s) deployed from the leaflet anchor tube formed in the main body of the catheter device. The internal space may be open to a face of the gripper arm configured to contact the leaflet, and bounded by side walls of the gripper arm.

The internal space may facilitate a complete extension of the wire guide member(s), such that the arm portion(s) and/or end cap(s) are completely passed through the leaflet and thus reliably implanted in the soft body tissue.

The internal space may comprise a first internal space configured to receive a first wire guide member and a second internal space configured to receive a second wire guide member. The openings of the first and second internal spaces may be separated by a gripping portion configured to contact the leaflet. Providing a gripping portion may increase the surface area of the leaflet in contact with the gripper arm, and thus the stability of the leaflet during implantation of multiple arm portions and/or end caps into the leaflet.

In example embodiments the leaflet anchor tube is arranged to implant the leaflet anchor in the leaflet of the heart by piercing the leaflet from an atrial side of the leaflet. The leaflet may be a mitral valve leaflet or a tricuspid valve leaflet.

This device allows a leaflet to be easily gripped and a new chord securely attached to the leaflet. There is no need for a complex procedure involving the use of vacuum and sutures as in WO 2008/101113. The mechanical gripper device can be opened and closed several times if required to release and re-engage the leaflet until it is in the desired position for the anchor to be placed.

The previous catheter devices of WO2016/042022 and WO2020/109596 generally contemplated the implantation of the leaflet anchor in the leaflet of the heart by piercing the leaflet from a ventricular side of the leaflet, rather than an atrial side of the leaflet. However, the present applicant recognises that a catheter device which implants a leaflet anchor from an atrial side of the leaflet provides a number of advantages which may not be present when a leaflet anchor is implanted from a ventricular side of the leaflet of the heart.

When implanted from a ventricular side of the leaflet, the leaflet anchor may need to be located towards an edge of the leaflet in order to provide adequate support to the flailing leaflet. In contrast, when implanted from an atrial side of the leaflet, the leaflet anchor may provide adequate support to the edge of the leaflet when the leaflet anchor is implanted towards the edge of the leaflet, or towards an annulus of the leaflet.

The chosen location of implantation of the leaflet anchor may depend on a number of factors, and may be patient-specific. Implanting the leaflet anchor in an atrial side of the leaflet may provide a surgeon with greater flexibility in choosing where to implant the leaflet anchor, i.e. by being able to implant the leaflet anchor towards an edge of the leaflet, towards an annulus of the leaflet, or in between.

The tissue of the leaflet closer to the annulus, rather than towards an edge of the leaflet, may be less prone to experiencing trauma associated with the implantation of the leaflet anchor. The tissue towards the annulus of the leaflet may be thicker than that closer to the leaflet edge, for example. The tissue towards the annulus of the leaflet may be more able to withstand the tension associated with the artificial chordae line during the cardiac cycle, when the line is taut.

By implanting the leaflet anchor from the atrial side, the leaflet anchor may be able to be implanted closer towards an annulus of the leaflet of the heart whilst still providing adequate support to the edge of the leaflet.

When the artificial chordae line is used to prevent leaflet regurgitation (i.e. mitral regurgitation or tricuspid regurgitation) the line will generally be fixed at two ends, with one end located at/in the papillary muscle of the heart, and the other located at the leaflet anchor. If the leaflet anchor (soft tissue anchor system) is therefore implanted in the leaflet from the ventricular side, the line will extend to the papillary muscle without providing any support to the edge of the leaflet, i.e. at a flailing end of the leaflet. However, when implanted in the leaflet from the atrial side, the line may extend along an atrial-side surface of the leaflet, and extend over the edge of the leaflet before descending into the ventricle to the location of implantation in the papillary muscle. As such, the line may provide support to the flailing edge of the leaflet with the anchor implanted towards the leaflet annulus. This may also better replicate the action of chordae tendineae located towards the edge of the leaflet of the heart valve.

The leaflet anchor (soft tissue anchor system) may be arranged to be deployed such that the artificial chordae line will be in contact with an atrial side of the leaflet of the heart between the leaflet anchor and an edge of the leaflet of the heart valve.

When the leaflet anchor is implanted from the atrial side, it will be understood that the artificial chordae line may provide support to the flailing edge when implanted in the atrial side of the leaflet, given that the line will descend from the atrial side to the ventricular side through the leaflet valve over an edge of the leaflet, when the line is implanted. This may be particularly beneficial when treating flailing leaflets.

The previous catheter devices of WO2016/042022 and WO2020/109596 may generally require precise implantation of the leaflet anchor (soft tissue anchor system) in the leaflet to provide adequate support to the edge of the leaflet. As the leaflet anchor does not provide any additional support to the edge of the leaflet other than its own implantation, the location of implantation of the anchor determines how much support is provided to the edge of the leaflet. Accordingly, implantation of the leaflet anchor may need to be more precise to ensure adequate support to the edge of the leaflet.

However, due to the contact of the artificial chordae line with the edge of the leaflet when the leaflet anchor is implanted in the atrial side of the leaflet, the location of implantation of the leaflet anchor of the present invention need not be so precise, as additional support is provided to the edge of the leaflet regardless of whether the leaflet anchor is implanted towards the edge of the leaflet or towards the atrial annulus of the leaflet. This may result in more efficient implantation of the leaflet anchor, as movement of the leaflet during the cardiac cycle which may alter the location of implantation of the leaflet anchor will be of lesser detriment to the overall support provided by the leaflet anchor.

The artificial chordae line may have a varying cross-sectional area. The artificial chordae line may have a first cross-sectional area at an end distal to the leaflet anchor and/or configured to be located at/in the papillary muscle. The artificial chordae line may have a second cross-sectional area at an end proximal to and/or attached to the leaflet anchor (soft tissue anchor system).

The first cross-sectional area and the second cross-sectional area may be different. The second cross-sectional area may be a rectangular cross-sectional area. The second cross-sectional area may be an oval cross-sectional area. A major axis of the second cross-sectional area may be configured to be parallel to an atrial surface of the leaflet anchor. The second cross-sectional area may be greater than the first cross-sectional area. The first cross-sectional area may be circular.

By providing an artificial chordae line comprising a second cross-sectional area as discussed, the surface area of the artificial line which is in contact with the atrial side of the leaflet may be increased. The artificial chordae line may therefore provide a greater degree of support to the flailing leaflet when implanted.

The artificial chordae line may comprise a plurality of sutures. The plurality of sutures may increase an area of contact between the artificial chordae line and the atrial side of the leaflet, such that a greater degree of support is provided to the flailing leaflet by the artificial chordae line. Additionally, if one of the plurality of sutures fails, there is a redundancy introduced such that one or more of the plurality of sutures may still be successfully implanted.

The catheter device comprises a housing section extending from a distal end towards a proximal end of the catheter device. The most distal end of the catheter device may be where the artificial chordae line is generally implanted from, whilst the proximal end may be located at an opposite end of the catheter device. The catheter device may generally be inserted into the body in a direction aligned with the direction of extension from the proximal end to the distal end of the catheter device.

The leaflet anchor (soft tissue anchor system) may be arranged to be deployed by pushing it out of an opening at the end of the leaflet anchor tube, wherein the opening is arranged to be in contact with an atrial side of the leaflet of the heart during deployment.

Placing the opening of the leaflet anchor tube in contact with the atrial side of the leaflet of the heart during deployment may facilitate the implantation of the leaflet anchor from the atrial side of the leaflet. For example, placing the opening in contact with the atrial side may ensure proper placement, location and deployment of the anchor as it assumes its unfolded configuration from its elastically folded configuration.

The leaflet anchor (soft tissue anchor system) may be arranged to be pushed out of the leaflet anchor deployment mechanism from the proximal end of the catheter device toward the distal end of the catheter device.

The catheter device may comprise a linear-shaped rod for deployment of the leaflet anchor. The linear-shaped rod may be configured to push the leaflet anchor out of the leaflet anchor deployment mechanism. The linear-shaped rod may be the wire guide member(s).

The previous devices disclosed in WO2016/042022 and WO2020/109596 each taught a U-shaped rod for pushing the leaflet anchor out of the leaflet anchor deployment mechanism. The U-shaped rod was required in each of these prior art devices due to the leaflet anchor being deployed in a ventricular side of the leaflet, i.e. beneath the leaflet. However, for the present device, the leaflet anchor (soft tissue anchor system) may be configured to be implanted in an atrial side of the leaflet. Accordingly, the leaflet is approached from above, and as such the leaflet anchor is deployed in a distal direction of the catheter device. In other words, the leaflet anchor is deployed in the same direction by which the catheter device approaches the leaflet of the heart.

As such, a linear rod may be used. The linear rod, being linear, may generally be easier to manufacture. The linear rod may also be easier to arrange in the catheter device, along any other number of wires, rods and the like for operating other components located in the distal end of the catheter device. As such the linear rod may ease manufacture of the catheter device, particularly when compared to previous U-shaped rod designs. The wire guide member(s) may function as the linear rod.

The catheter device may generally approach the location of implantation from an atrial side of the heart rather than a ventricular side of the heart due to known techniques of insertion of the catheter device into the heart. That is, on approach a distal end of the catheter device will be oriented towards the atrial side of the leaflet of the heart. As such, to facilitate deployment of the leaflet anchor in the atrial side of the leaflet (the surface of which will be oriented towards the proximal end of the catheter device), the leaflet anchor may be pushed out of the leaflet anchor deployment mechanism from the proximal end of the catheter device toward the distal end of the catheter device.

The leaflet anchor tube may be formed in the main body of the catheter device. The leaflet anchor tube may not be formed in the gripper device. An opening of the leaflet anchor tube may be located on a surface of the main body of the catheter device, such that the leaflet anchor may be deployed from the main body of the catheter device. The opening may be located such that the leaflet anchor is deployed from a circumferential surface and/or side of the catheter device.

As also described above, the gripper arm may comprise an internal space configured to receive the arm portion(s) and/or end cap(s) and the wire guide member(s) deployed from the leaflet anchor tube formed in the main body of the catheter device. The internal space may be open to a face of the gripper arm configured to contact the leaflet, and bounded by side walls of the gripper arm.

The leaflet anchor tube may extend generally along a length of the main body of the catheter device. The leaflet anchor tube may also have a component of extension along a radius of the catheter device such that the leaflet anchor may be deployed from the catheter device from a position on the circumferential surface of the catheter device.

The present applicants have realised that forming the leaflet anchor tube in the main body of the catheter device, rather than in the gripper device, may provide a number of advantages not previously known.

In WO2020/109596 and WO2016/042022, a catheter device comprising a gripper arm, with the leaflet anchor tube formed in the gripper arm of the device, is contemplated. To be able to house the leaflet anchor tube within the gripper arm, the gripper arm is hence longer than the leaflet anchor in the prior art device. The extended length of the gripper arm to accommodate the leaflet anchor, and the location of deployment from the gripper arm, may effectively reduce the range of depth at which the leaflet anchor may be implanted in the leaflet.

When the leaflet anchor tube is formed in the main body of the catheter device, the gripper arm may be shorter in length than the leaflet anchor when in the folded configuration. This is possible as the leaflet anchor (soft tissue anchor system) is not housed within the gripper arm. A shorter gripper arm may be able to grab the leaflet harder than a longer gripper arm when the same force is applied to the gripper arm. When the leaflet anchor is deployed in the leaflet at a location adjacent to a generally distal end of the gripper arm, the moment of the force associated with deploying the anchor may be reduced when using the shorter gripper arm. Thus, the leaflet may be more firmly held in place during implantation of the anchor.

Additionally, or alternatively, the length of the leaflet anchor may not be constrained by the length of the gripper arm. As such, when the leaflet anchor tube is formed in the main body of the catheter device, leaflet anchors longer than the length of the gripper arm may be utilised. Longer leaflet anchors may be capable of securing and/or supporting a larger portion of the leaflet of the heart, when implanted, compared to a shorter anchor.

The gripper arm may be arranged to meet an opening of the leaflet anchor tube. The leaflet anchor tube may be formed in the main body such that a surface of the gripper arm meets the opening of the leaflet anchor tube when it is held against the main body of the catheter. The opening of the leaflet anchor tube may meet a distal end of the gripper arm. The opening of the leaflet anchor tube may meet a surface of the gripper arm located towards the distal end of the gripper arm.

By arranging the gripper arm to meet an opening of the leaflet anchor tube, the leaflet anchor may be more reliably deployed at the desired location in the heart. For example, when the gripper arm grasps the leaflet, the leaflet may be held between the gripper arm and the leaflet anchor tube such that the leaflet anchor (soft tissue anchor system) may be deployed into the leaflet at the grasped location. The gripper arm, located against a proximal side of the leaflet, may provide resistance against the leaflet as the anchor is deployed, such that the leaflet is suitably constrained during deployment.

Alternatively, the leaflet anchor tube may be housed within the gripper arm. An opening of the leaflet anchor tube may be at the end of the gripper arm and oriented towards a distal end of the catheter.

In this arrangement, it will be understood that the gripper arm may be hinged towards a proximal end of the catheter device, with a distal end of the gripper arm oriented towards a distal end of the catheter device. Thus, contrary to the gripper arms disclosed in WO2016/042022 and WO2020/109596, the gripper arm may be oriented such that the leaflet anchor (soft tissue anchor system) is configured to be deployed in a distal direction of the catheter, rather than a proximal direction.

The opening of the leaflet anchor tube, located in the gripper arm, may be configured to meet a complementary surface of the main body of the catheter device. As such, when the leaflet is grasped by the gripper arm, the main body of the catheter device may provide resistance against the leaflet as the anchor (soft tissue anchor system) is deployed, such that the leaflet is suitable constrained during deployment.

The gripper arm may be configured to grasp the leaflet such that the leaflet anchor tube is arranged to implant the leaflet anchor toward an annulus of the leaflet. The gripper arm may be configured in this way when the leaflet anchor tube is formed in the main body of the catheter device or the gripper arm.

For example, the gripper arm may grasp the leaflet such that an opening of the leaflet anchor tube is located towards the annulus of the leaflet. When the leaflet anchor tube is located in the main body of the catheter device, the gripper arm may grasp the leaflet from a ventricular side such that the distal end of the gripper arm is in contact with the leaflet at or towards a ventricular annulus of the leaflet, and the opening of the leaflet anchor tube is located at or towards the annulus of the leaflet on the atrial side. When the leaflet anchor tube is located in the gripper arm, the gripper arm may grasp the leaflet from an atrial side such that the distal end of the gripper arm is in contact with the leaflet at or towards the atrial annulus of the leaflet, and the opening of the leaflet anchor tube is hence located at or towards the atrial annulus of the leaflet.

The gripper arm may be additionally or alternatively configured to grasp the leaflet such that the leaflet anchor tube is arranged to implant the leaflet anchor toward an edge of the leaflet. The gripper arm may be configured in this way when the leaflet anchor tube is formed in the main body of the catheter device or the gripper arm.

The catheter device may comprise a hinge mechanism for the gripper arm, wherein the hinge mechanism is formed integrally with the material of the main body and rotates away from the main body by elastic deformation of that material.

A single wire may be provided to actuate the gripper arm by bending the hinge mechanism to rotate the end of the gripper arm away from the main body, with the gripper arm returning elastically to its rest position if no force is applied to the wire.

The gripper arm may be actuated with a single wire or with multiple wires. Advantages can be obtained if a hinge mechanism for the gripper arm is formed integrally with the material of the main body and rotates away from the main body by elastic deformation of that material. The gripper arm as well as the hinge mechanism may be formed integrally with the material of the main body. Alternatively, the gripper arm may include a separately formed arm section, such as a milled piece or a laser cut piece, with the separate arm section being attached to a hinge mechanism of the main body, for example by gluing or welding.

In some examples, the main body of the catheter device may be formed from an elastic metal such as nitinol with a hinge being provided by an elastic joint formed in the elastic metal. In that case a single wire can be used to elastically deform the gripper arm by bending an elastic joint with the main body to rotate the end of the gripper arm away from the main body, with the gripper arm returning elastically to its at rest position once no force is applied to the wire. An advantage of this is that the elastic force of the gripper arm can hold it in place against the main body of the catheter device when the force is released from the wire, without the need for a separate wire to be pulled to keep the grip on the leaflet secure. A second wire may however be implemented as a backup if it may be needed.

In other examples, the main body of the catheter device may be formed from a composite material, such as carbon or glass reinforced PEEK. The gripper arm may then be joined to the main body of the catheter device using a pin joint, the pin forming the axis of rotation of the gripper arm. The pin joint mentioned herein may be a revolute joint or a hinge joint, i.e. comprising intermeshing features with a pin or cylindrical member joining said members, the pin forming the axis of rotation for the joint.

Alternatively, or in addition, the gripper arm can be heat set in a "more than closed" configuration. This would allow the gripper arm to grasp tissue towards the main body of the device.

To form the gripper arm and the hinge integrally with the main body of the catheter device, the main body of the catheter may comprise an outer tube, with the gripper arm being formed as an articulated section of the outer tube. Several forms of slits and/or patterns can be formed in the tubing in order to provide a weakened hinge section allowing for bending without plastic deformation of the gripper arm.

In alternative arrangements a hinged gripper arm may be used. In that case the gripper arm may be milled, actuation in that case could be done with a spring for closing, and wire for opening, or vice versa, or with two wires (one for opening and one for closing). A pulley cut in the device can be used to redirect the pulling force from the pull wire.

The gripping surface of the gripper arm may be arranged to hold the leaflet with friction. For example, the gripping surface may use a material with a high coefficient of friction and/or the gripping surface may have a texture or surface profile for increasing friction, such as a ridged or saw-toothed profile.

The housing section may be a two-part housing section. The catheter device may comprise: the two-part housing section extending from the distal end of the catheter device along the length of the catheter device toward the proximal end of the catheter device, the two-part housing section comprising a distal part at the distal end of the catheter device and a proximal part located on the proximal side of the distal part; the leaflet anchor deployment mechanism being at the proximal part of the housing section; a papillary anchor deployment mechanism at the distal part of the housing section for deployment of a papillary anchor for attachment to the papillary muscle, wherein the papillary anchor deployment mechanism is arranged for deployment of the papillary anchor by moving it outward in the distal direction relative to the distal part; and a flexible joint located between the proximal part and the distal part of the two-part housing section, wherein the flexible joint allows a centreline of the distal part to be angled relative to a centreline of the proximal part.

The two-part housing section may be arranged to be coincidentally placed between the papillary muscle and a leaflet of the heart during use of the catheter device.

The gripper arm may be provided in the proximal part of the two-part housing section and may be rotatably coupled to the catheter device. The gripper arm may be rotatably coupled via any of the above-discussed mechanisms.

The two-part housing section may be formed from two tubular sections in any suitable material, i.e. a medically appropriate material. Stainless steel or nitinol may be used. In the alternative, composite materials such as carbon-fibre or glass-fibre reinforced PEEK may be used. The catheter device may be formed via a combination of such materials with the materials for different parts of the device being selected dependent on the required characteristics of those parts. A material that allows Ultrasound to pass through and at the same time have sufficient strength is preferred, Carbon reinforced PEEK meets these demands well, and would also allow Injection moulding of the components which lowers manufacturing cost. Fibre reinforced plastic are normally not visible on X-ray, so strategically placed radiopaque markers in all components may be used to determine device component(s) position and orientation on X-ray relative to each other, as complementary information to ultrasound imaging.

The flexible joint may include a hinge element, for example with the distal part of the two-part housing section coupled to the proximal part via a pivoting mechanism or via an elastically deformable element. For example, the two parts of the housing section may be composite or metal parts coupled together by the hinge element.

The papillary anchor may be housed within the distal part of the housing section before its deployment. The papillary anchor may have a similar cross-section as the distal part of the housing section. For example, both may have a tubular form when the anchor is held in the distal part. As noted above the anchor may have a folded and an unfolded configuration allowing pins of the anchor to form into hooks within the body tissue during deployment of the papillary anchor. The papillary anchor deployment mechanism may take a similar form to that of WO2016/042022 or WO2020/109596.

In one example the papillary anchor deployment mechanism includes a first wire or rod for pushing the papillary anchor in the distal direction relative to the distal part of the two-part housing section. There may additionally be a second wire or rod for releasing the papillary anchor from the papillary anchor deployment mechanism in order to disengage the papillary anchor from the catheter device after it is implanted in the body tissue, i.e. the tissue of the papillary muscle and/or tissue adjacent to the papillary muscle.

The papillary anchor may have a chordae line attached to it, and may include a locking mechanism, such as a locking ring as in WO2016/042022 or in WO2020/109596, the locking mechanism being for clamping the chordae line when no force is applied to the locking mechanism. The locking ring may be able to be elastically deformed to release the line from the locking mechanism for adjustment of the length of the chordae line. The papillary anchor deployment mechanism may include a locking ring holder for holding the locking ring in its elastically deformed position, with the papillary anchor deployment mechanism being arranged to selectively withdraw the locking ring holder from the locking ring so that the chordae line can be locked in place after deployment of the papillary anchor and after any required adjustment of the length of the chordae line.

The leaflet anchor deployment mechanism may allow for retraction and repositioning of the leaflet anchor (soft tissue anchor system) after deployment of the anchor into the leaflet via an ejector unit having a grasping device with a first configuration arranged to permit deployment of the leaflet anchor into the leaflet without disengagement of the leaflet anchor from the ejector unit, and a second configuration in which the leaflet anchor is reversibly released from the ejector unit; wherein in the first configuration the grasping device of the ejector unit grasps a proximal end of the leaflet anchor, whilst a distal end of the leaflet anchor is unimpeded by the grasping device to enable it to be implanted in the leaflet; and wherein in the second configuration the grasping device of the ejector unit is disengaged from the leaflet anchor.

The leaflet anchor (soft tissue anchor system) may be retracted within the retraction tube/catheter, by pulling the chordae so the leaflet anchor folds inside the retraction tube. The retraction tube may be placed on top of a chordae only attached to the leaflet (with device removed) or a leaflet anchor placed in a poor location (partly engaged, free floating, entangled etc.). The retraction tube may be a deflectable shaft, with or without a flexible section on the tip (that allows the tip to find the leaflet anchor base, to allow retraction). Alternatively, the retraction shaft may be a flexible tube that is arranged to engage with the base of the leaflet anchor.

Viewed from a sixth aspect, there is disclosed a method of implanting the soft tissue anchor system according to the first aspect, the method comprising: engaging the tubular cap member using a wire guide member; pushing the tubular cap member through the body tissue using the wire guide member; withdrawing the wire guide member; and applying tension to the line when it is passing through the tissue in a direction away from the surface thereof, such that the tubular cap member sits against the soft body tissue.

The method of the sixth aspect may have one or more features corresponding to those of the soft tissue anchor system of the first aspect. Thus, the above-mentioned description of the soft tissue anchor system of the first aspect, including but not limited to all technical advantages and alternative embodiments, may be equally applicable to the method of the sixth aspect.

Viewed from a seventh aspect, there is disclosed a method of manufacturing a soft tissue anchor system according to the first aspect, the method comprising: fabricating the tubular cap member; and fixing the line to the tubular cap member with the line extending from the central portion thereof.

Fabricating the tubular cap member may comprise machining an outer tubular member; machining an inner tubular member; fixing the line between the outer tubular member and the inner tubular member; and fitting the inner tubular member in the outer tubular member.

Alternatively, where the tubular cap member is a single, monolithic component, fabricating the tubular cap member may comprise laser cutting or machining the tubular cap member from a single piece of nitinol or stainless steel.

The method of the seventh aspect may have one or more features corresponding to those of the soft tissue anchor system of the first aspect. Thus, the above-mentioned description of the soft tissue anchor system of the first aspect, including but not limited to all technical advantages and alternative embodiments, may be equally applicable to the method of the seventh aspect.

Viewed from an eighth aspect, there is disclosed a method of implanting a soft tissue anchor system in soft body tissue according to the second aspect, the method comprising: engaging each arm portion using a respective wire guide member; pushing each arm portion through the body tissue using the respective wire guide member; withdrawing each wire guide member; and collapsing each arm portion in folds towards the base portion such that the body tissue is sandwiched between the base portion and each of the arm portions.

The method of the eighth aspect may have one or more features corresponding to those of the soft tissue anchor system of the second aspect. Thus, the above-mentioned description of the soft tissue anchor system of the second aspect, including but not limited to all technical advantages and alternative embodiments, may be equally applicable to the method of the eighth aspect.

Viewed from a ninth aspect of the present invention, there is provided a method of manufacturing a soft tissue anchor system according to the second aspect, the method comprising: fabricating the U-shaped fabric body; and configuring each arm portion to collapse in folds.

Fabricating the U-shaped fabric body may comprise laser cutting a fabric. The fabric may be selected from the group consisting of: polyester; PET; UHMPE; EPTFE; PTFE or the like.

The method of the ninth aspect of the invention may have one or more features corresponding to those of the soft tissue anchor system of the second aspect of the invention. Thus, the above-mentioned description of the soft tissue anchor system of the second aspect, including but not limited to all technical advantages and alternative embodiments, may be equally applicable to the method of the ninth aspect.

Viewed from a tenth aspect, there is disclosed a method of implanting a soft tissue anchor system in soft body tissue according to the third aspect, the method comprising: implanting each of the anchor members in the soft body tissue; and applying a tensile force to each of the bridle lines by application of a tensile force to the common bridle point.

Viewed from an eleventh aspect, there is disclosed a method of manufacturing a soft tissue anchor system according to the third aspect, the method comprising: connecting each bridle line to a respective anchor member; and connecting each bridle line to a common bridle point.

Viewed from a twelfth aspect, there is disclosed a method of repairing the heart by implanting an artificial chordae line, the method comprising: using the catheter device of the third or fourth aspect to implant the soft tissue anchor system.

The method of the twelfth aspect may have one or more features corresponding to those of the catheter device of the third or fourth aspect. Thus, the above-mentioned description of the catheter device of the third and/or fourth aspects, including but not limited to all technical advantages and alternative embodiments, may be equally applicable to the method of the twelfth aspect.

Certain example embodiments of the invention will now be described by way of example only and with reference to the accompanying drawings, in which:
Figure 1 illustrates the procedure for insertion of a catheter device through a mitral valve;
Figures 2 to 6 show the action of a mechanical gripping mechanism using two gripper arms;
Figure 7 illustrates gripping of a leaflet of the mitral valve with one gripper arm;
Figures 8 to 12 show deployment of a leaflet anchor in a device using an ejector device;
Figure 13 shows a close up view of the valve during placement of a leaflet anchor, which is coupled to an artificial chordae line;
Figure 14 shows movement of the distal end of the catheter device to the papillary muscle for placement of a papillary anchor;
Figure 15 illustrates withdrawal of a treatment catheter part of the device and adjustment of the chord length with an optional adjustment catheter;
Figures 16 and 17 show an example of a hook for an anchor which is threaded with a suture;
Figures 18 and 19 show the folded and unfolded configuration of an example of a papillary anchor;
Figure 20 illustrates withdrawal of a catheter device following implantation of a leaflet anchor in an atrial surface of a leaflet of a heart valve;
Figure 21 shows the catheter device arranged to implant the leaflet anchor in the atrial surface of the leaflet of the heart valve;
Figure 22 shows an alternative arrangement of the catheter device arranged to implant the leaflet anchor in the atrial surface of the leaflet of the heart valve;
Figure 23 shows a modified gripper device for grasping the leaflet of the heart valve in an undeployed configuration;
Figure 24 shows the modified gripper device for grasping the leaflet of the heart valve in a deployed configuration;
Figure 25 shows a fabric body anchor system;
Figure 26 shows a detail view of an end cap of a fabric body anchor system;
Figure 27A shows a fabric body anchor system during implantation in a mitral valve leaflet;
Figure 27B shows a fabric body anchor system following implantation in a mitral valve leaflet from a ventricular-side view;
Figure 27C shows a fabric body anchor system following implantation in a mitral valve leaflet from an atrial-side view;
Figure 28 shows end caps of a fabric body anchor system each engaged with a wire guide member;
Figure 29A shows an alternative end cap arrangement of a fabric body anchor system during implantation in a mitral valve leaflet;
Figure 29B shows a detail view of the end cap arrangement of the fabric body anchor system illustrated in figure 29A;
Figures 30A and 30B show an alternative end cap arrangement of a fabric body anchor system;
Figures 31A and 31B show an alternative end cap arrangement of a fabric body anchor system;
Figures 32A and 32B show an alternative end cap arrangement of a fabric body anchor system;
Figure 33A shows an alternative end cap arrangement of a fabric body anchor system in a first configuration;
Figure 33B shows the end cap arrangement of figure 33A in a second configuration;
Figure 34 shows a wire guide member engaged with an end cap of a fabric body anchor system;
Figures 35A to 35I show a prototype fabric body anchor system at various stages during implantation;
Figures 36A shows a schematic illustration of a U-shaped fabric body anchor system;
Figures 36B and 36C show prototypes of a fabric body anchor system as shown in figure 36A, in plan view;
Figures 37A to 37C show various schematic illustrations of a soft tissue anchor system housed within a delivery shaft of a catheter device;
Figure 38 shows a retrieval mechanism for a fabric body anchor system;
Figures 39A to 39D show the steps of retrieval of a fabric body anchor system;
Figure 40 illustrates an alternative end cap arrangement;
Figure 41 illustrates a prototype soft tissue anchor system in a plan view;
Figure 42 illustrates an alternative end cap arrangement;
Figures 43A to 43D illustrate an alternative soft tissue anchor system during steps of its implantation in soft body tissue;
Figure 44 shows a wire guide member having a bulge portion being received by a tubular cap member;
Figure 45 schematically represents a deployment system for implanting a pair of anchor members;
Figure 46A shows a soft tissue anchor system implanted in a mitral valve leaflet; and
Figure 46B shows the soft tissue anchor system of figure 46A under tension of a line.

The following description details one or more features consistent with, and combinable with, the aforementioned description of the anchor system. The following embodiments herein discussed are not to be viewed in isolation and are not intended to be restrictive, but are to be viewed in the context of the present disclosure as a whole, also considering the appended figures.

The catheter devices presented here are proposed for non-surgical (endovascular) insertion of mitral chords to address mitral regurgitation caused by prolapse of a leaflet 12 of the valve. The figures show different forms of catheter device 2 for this purpose, but it will be understood that the general principles are the same for each device in terms of implantation of a leaflet anchor 10, which can be substituted with any of the soft tissue anchor systems described further below and in accordance with the present invention, and a papillary anchor 9 in order to insert one or more artificial chordae lines 14 into the heart. The artificial chordae line(s) 14 are fixed to the prolapsing leaflet 12 and to the papillary muscle 26, thereby recreating a normal anatomy. A single catheter device 2 is used to place both a leaflet anchor 10 (or a soft tissue anchor system as described further below) and a papillary anchor 9. The length of the chord 14 can be adjusted, again using the same catheter device 2, to eliminate the mitral regurgitation. Thus, such a catheter device enables a single minimally invasive endovascular procedure to be used to repair the mitral valve, providing significant advantages compared to earlier systems requiring more invasive procedures and/or multiple operations.

It should be noted that although an endovascular approach is preferred and the device is hence capable of using this approach, the device could of course be used in different procedures, including more invasive procedures. Many of the advantages will remain, and it could be beneficial to use this device in situations where a more invasive procedure is merited. In addition, it is contemplated that, as discussed above, aspects of the design of the papillary anchor 9 or the soft tissue anchor system described below could be used for an anchor for other purposes and this disclosure is not intended to be limited in this regard.

The catheter device 2 described in the following can be used to insert mitral chords through the venous system, starting in the femoral vein in the groin. A catheter is advanced to the right atrium. Approach to the left atrium is then gained by a so-called transseptal puncture whereafter a larger guidance catheter is advanced into the left atrium. The catheter device 2 for the heart repair is then introduced through the guiding catheter and into the left atrium.

X-ray and ultrasound guidance is used to position the device and, as explained in more detail below, the mitral leaflet 12 is grabbed and an artificial chordae line 14 is attached using a self-expandable leaflet anchor 10, or any of the soft tissue anchor systems described below. The artificial chordae line 14 is then attached to the papillary muscle 26, using a papillary anchor 9. The chord length can now be adjusted to eliminate any mitral regurgitation. Excess chord is then cut and all catheters are withdrawn. Echo and Doppler imaging is used to perform the procedure and monitor the result. The successful use of this endovascular technique will drastically reduce the invasiveness, complications and cost of mitral valve repair.

More detail on the structure and function of the device is set out below with reference to the figures. The procedure of using one form of the device can be summarised as follows:
1) The femoral vein is entered using standard Seldinger technique and the guiding catheter introduced.
2) The guiding catheter is advanced to the right atrium under x-ray guidance.
3) The left atrium is entered after penetration of the atrial septum, guided by x-ray and transesophageal echo.
4) Correct position of the entrance site in the left atrium is verified to assure proper alignment for insertion of the guiding and treatment catheters. The entrance hole in the atrial septum is dilated and the guiding catheter is advanced into the left atrium.
5) A treatment catheter device 2 is advanced through the guiding catheter and positioned in the left atrium above the mitral valve.
6) The prolapsing segment of the mitral leaflet 12 is located with ultrasound and the treatment catheter device 2 is advanced into the left ventricle placing a gripper 6 of the treatment catheter device 2 in position to grip the prolapsing segment.
7) The prolapsing segment is gripped and after assuring correct position either the leaflet anchor 10 is pushed through the leaflet 12 allowing it to open and fix the leaflet 12, or the soft tissue anchor system described below, is deployed.
8) The connection of the leaflet to the anchor may be tested whilst it remains attached to the catheter device 2 via an ejector unit 36, and if the connection is sufficient then the distal end of catheter is advanced further into the left ventricle.
9) The papillary anchor 9 is pushed into the papillary muscle 26 area and out of its housing 8 thereby letting the papillary anchor 9 open inside the papillary muscle 26.
10) If the gripper 6 is still grasping the leaflet 12 then it is released, such as by releasing the leaflet anchor 12 from the ejector unit 36.
11) The length of the artificial chordae line 14 is adjusted until mitral regurgitation is eliminated.
12) The catheter device 2 is pulled back from the papillary anchor 9, and elimination of mitral regurgitation is again confirmed by echocardiography.
13) The position of the artificial chordae line 14 is locked at the papillary anchor 9.
14) The excess chordae line 14 is cut.
15) Additional artificial chordae lines may be placed if necessary.
16) The catheter device is fully withdrawn and removed from the vascular system.

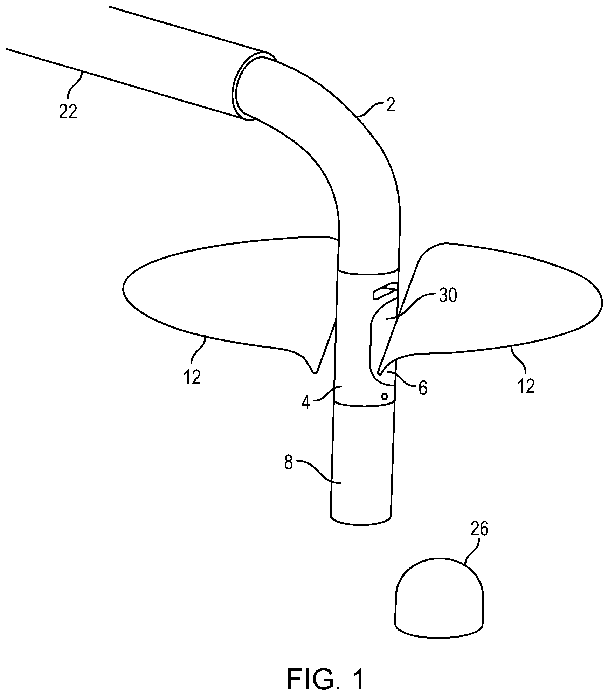

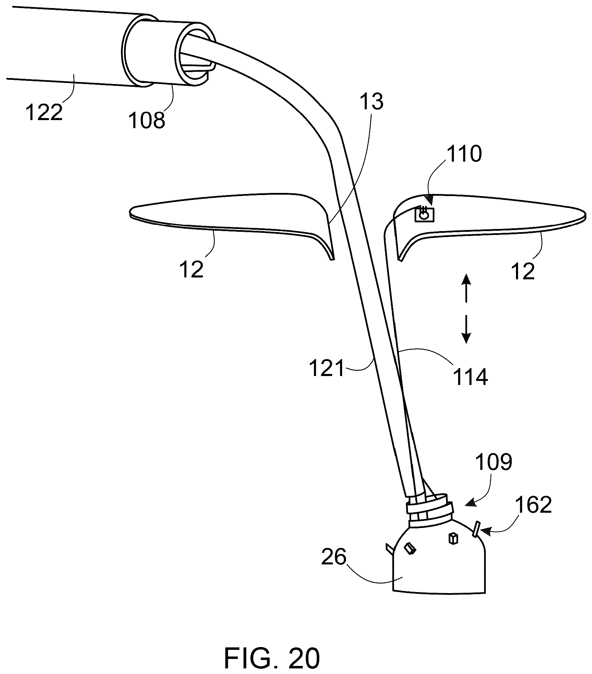

Figures 1 to 19 display an exemplary catheter device 2 as disclosed by WO2020/109596. Whilst the catheter device 2 disclosed in WO2020/109596 is used to implant a leaflet anchor 9 in combination with an artificial chordae line 14 from a ventricular side of a mitral valve leaflet 12, many of the features and/or components of the exemplary catheter device 12 may be compatible with the catheter device 102 of the present invention, or may be modified in accordance with the teachings of the present invention such that a leaflet anchor 110 in combination with an artificial chordae line 114 can be implanted in a leaflet 12 from an atrial side of the leaflet 12, as shown in figures 20 to 22.

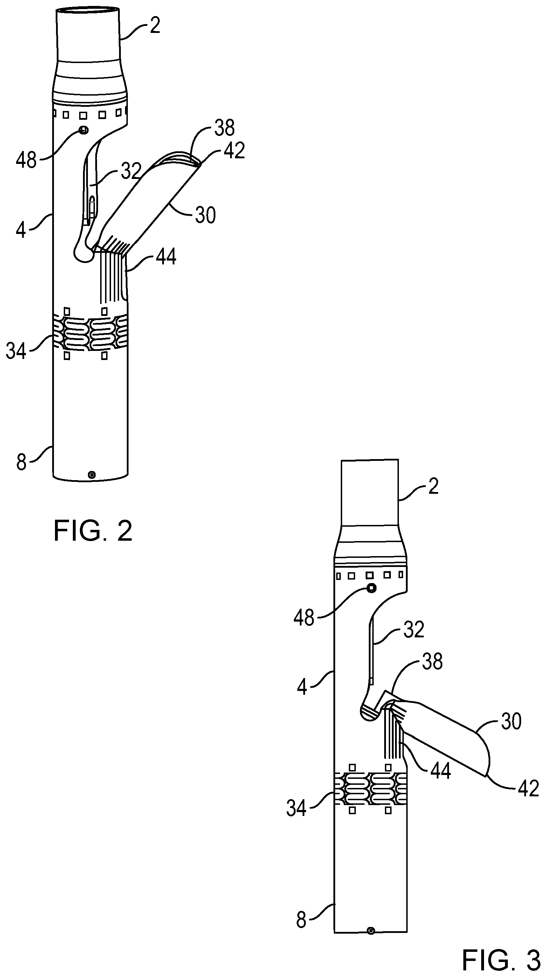

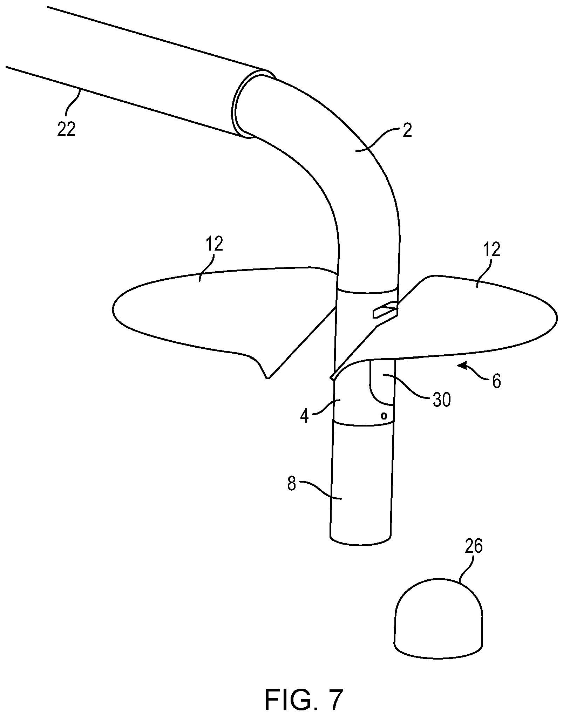

Figure 1 shows guide catheter 22 that has been used to steer a catheter device 2 to a required position within the heart adjacent extending through the mitral valve and hence being between two leaflets 12. The catheter device 2 is composed of four different main parts; a steerable catheter, a gripper housing 4, a gripper device 6 and a papillary anchor housing 8, which holds a papillary anchor 9. The gripper housing 4 and the papillary anchor housing 8 may form a proximal part 4 and a distal part 8 of a two part housing section with a central flexible and extendable joint 34 as shown in figures 2 to 6, 14 and 20 to 22. Thus, it should be understood that the procedure shown in figure 1 (and likewise in figures 7, 13 and 15) may use this arrangement for the gripper housing (proximal part) 4 and papillary anchor housing (distal part) 8. The steerable catheter could be replaced with an alternative arrangement using a steerable sheath about a steerable catheter and flexible tubing within the steerable catheter.

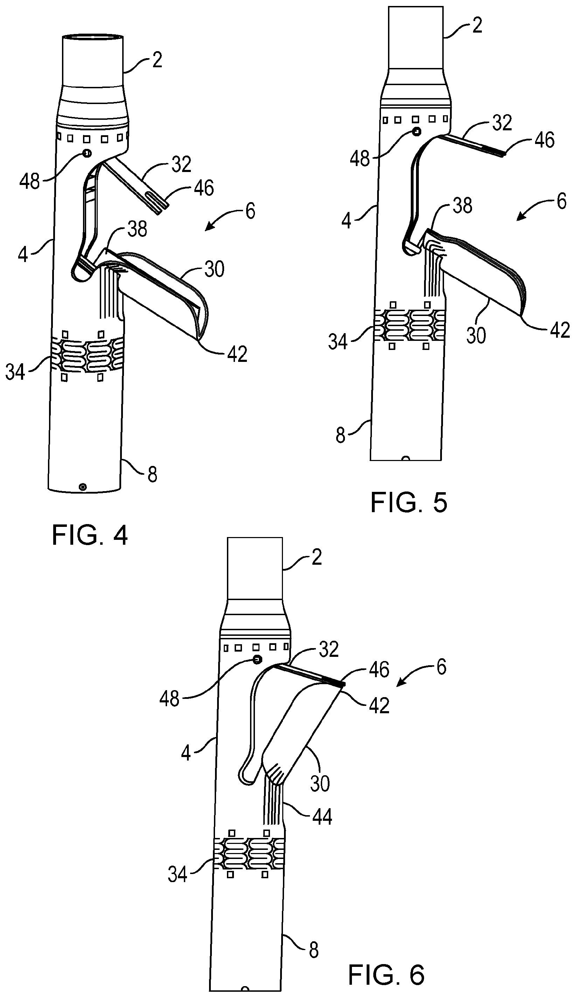

Figure 1 shows a front view of one example catheter device with the gripper device 6 closed. The gripper device 6 of some arrangements uses a single gripper arm 30 that grips the leaflet 12 against the gripper housing part 4 as shown in figure 7. In other arrangements the gripper device 6 uses two gripper arms 30, 32 as shown in figures 2 to 6 in order to allow the leaflet 12 to be grasped between the two gripper arms 30, 32 at a point spaced apart from the main body of the catheter device. The gripper device 6 is a part of a leaflet anchor deployment mechanism for deploying the leaflet anchor 10 or the soft tissue anchor system discussed below to attach it to the leaflet 12 of the heart. In the example of the figures the gripper device 6 includes a leaflet anchor tube 38 for housing the leaflet anchor 10 in a folded configuration prior to deployment. In example embodiments the leaflet anchor tube 38 is in the (first) gripper arm 30, as seen in figures 2 and 4, for example. When the gripper device 6 grasps the leaflet 12, the leaflet anchor 10 can be pushed out of the leaflet anchor tube 38 to pierce the leaflet 12 and form the leaflet anchor 10 into an unfolded configuration so that hooked formations 40 of the leaflet anchor 10 secure it in the leaflet 12. Alternatively, a soft tissue anchor system as discussed below can be implanted by piercing the leaflet as shown later in the figures.

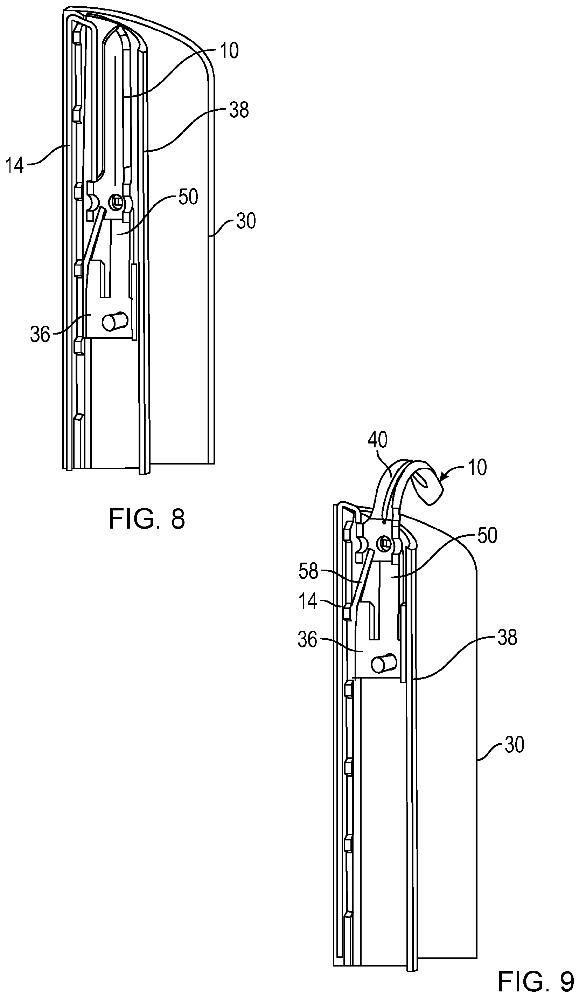

The leaflet anchor 10 or the soft tissue anchor system discussed below is connected to an artificial chordae line 14, which can sit inside a narrow channel that goes along the surface of the first gripper arm 30 (as shown in figures 8 to 12, for example) and via the papillary anchor housing 8 to the papillary anchor 9 (as shown in figures 20 to 22, for example). The channel can be slightly smaller than the diameter of the artificial chordae line 14 and/or have a thin shielding structure (not shown). This makes the artificial chordae line 14 sit in place due to a friction fit. The artificial chordae line 14 goes into the papillary anchor housing 8 and through a papillary anchor locking section, through a locking and cutting piece. The artificial chordae line 14 can be attached to a wire which passes back along the catheter all the way to the outside (to make the adjustment smoother). The wire allows for a shortening of the chord during the procedure, by pulling, or a lengthening of the chord, since the wire can be pushed through the catheter.

The two-part housing section, with the gripper housing (proximal part) 4 and papillary anchor housing (distal part) 8 might be approximately 6-7 mm in diameter, and approximately 30 mm in length.

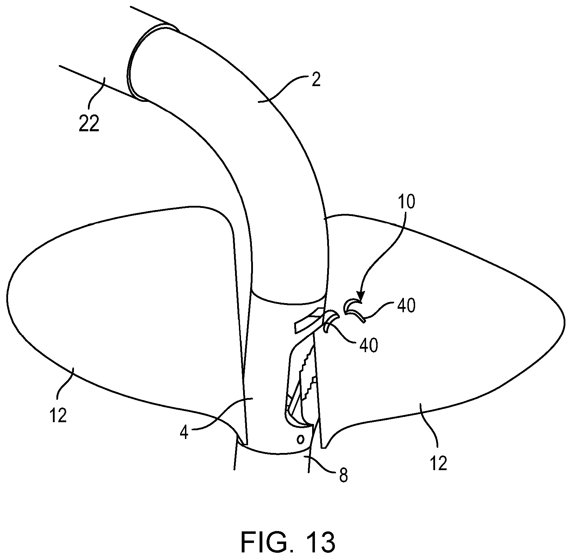

Figures 2 to 6 show steps in movement of the gripper mechanism 6 in an example with two gripper arms 30, 32 as discussed above. This gripper mechanism 6 is a part of a housing section that also includes a flexible and extendable joint allowing the papillary anchor housing 8 (distal part) to be moved toward the papillary muscle 26 after the leaflet 12 has been grabbed by the gripper mechanism 6. In this example, in order to grasp the leaflet 12, the first gripper arm 30 is rotated to move its end 42 away from the main body of the catheter device, with this rotation being enabled via a weakened area 44 of the tubular form of the main body. It can be seen that the leaflet anchor tube 38 sits inside the first gripper arm 30, with the end of the leaflet anchor tube 38 having an opening at the end 42 of the first gripper arm 30. With the first gripper arm 30 open, the second gripper arm 32 is free to rotate to move its end 46 outward of the main body. In this example the second gripper arm 32 rotates around a hinge formed by pins 48 placed in holes in the proximal part 4 of the two-part housing section, but it will be appreciated that a similar final placement of its end 46 may be achieved via a sliding movement. With the second gripper arm 32 folded outward the first gripper arm 30 can close so that the two ends 42, 46 come into contact at a point spaced apart from the main body of the device. This allows the leaflet 12 to be grasped. With the leaflet 12 in place the leaflet anchor 10 can be moved out of the leaflet anchor tube 38 to implant it, such as via a mechanism with an ejector unit 36 as described below in relation to figures 8 to 12, with the final positioning of the leaflet anchor 10 being similar to that shown in figure 13.

Figure 7 shows an alternative form of gripper mechanism 6 that grasps the leaflet 12 with a single gripper arm that holds it against the gripper housing 4. This could also use the ejector unit 36 mechanism of figures 8 to 12.

A ridged surface on the gripper arm(s) 30, 32 may be provided to help it grip the leaflet 12. 3D ultrasound and/or other available sources can be used to confirm that the gripper mechanism 6 has grasped the correct part of the leaflet 12.

The gripper mechanism 6 can be opened and closed as many times as needed to grasp the right part of the leaflet 12. The opening and closing may be facilitated by a system allowing for one wire to pull the gripper mechanism 6 open, and one to pull it closed. Different arrangements of wires and/or rods may be used to control the example with two gripper arms 30, 32, as discussed above. Once the position of the gripper mechanism 6 is confirmed then the leaflet anchor 10 can be pushed out of the end of the leaflet anchor tube 38, such as by pulling a wire in the other end of the catheter. Figure 13 shows a close up view of the leaflet anchor 10 placed in the leaflet 12 with the hooked formations 40 engaging with the leaflet 12.

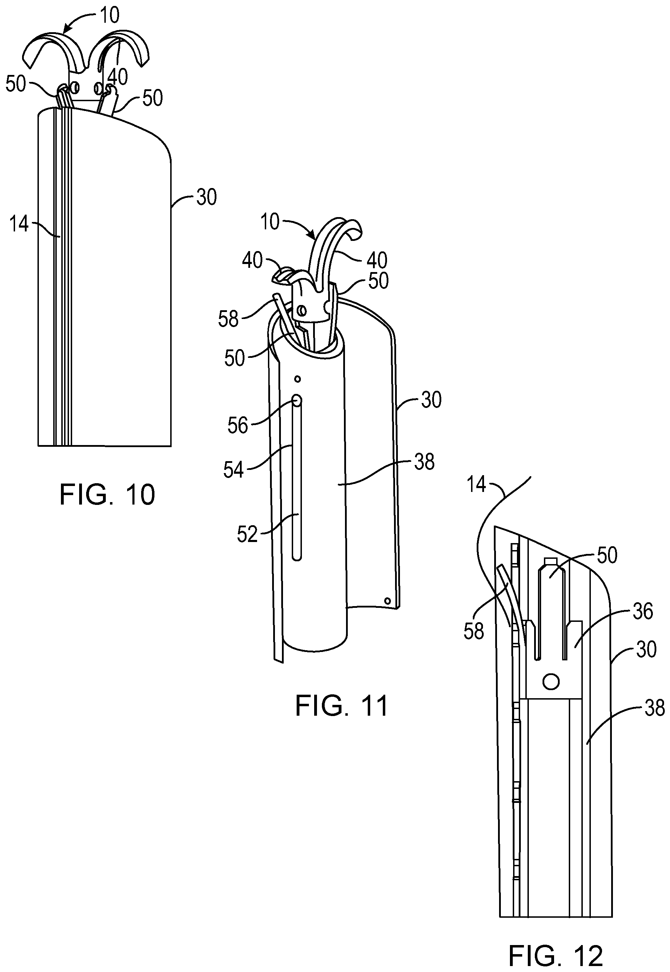

As noted above, an ejector unit 36 may be used as shown in figures 8 to 12. With the use of the ejector unit 36 the leaflet anchor deployment mechanism allows for retraction and repositioning of the leaflet anchor 10 after deployment of the anchor 10 into the leaflet 12. This is achieved via the ejector unit 36, which includes a grasping device 50 with a first configuration, as shown in figure 8 and figure 9 and a second configuration as shown in figure 10 and figure 11.

If the physician is not satisfied by the connection during the testing (for example, if there is too much movement of the anchor 10 and/or not enough resistance to force on the line) then the leaflet anchor 10 can be retracted and placed in another location.

A groove 52 is provided in a wall of the leaflet anchor tube 38 for guiding the ejector unit 36. The groove 52 ensures that the ejector unit 36 remains a single orientation relative to the tube 38 while it is moved along the tube. The groove 52 can set maximum limits on the range of movement of the ejector unit 36 and thus may prevent it from going too far in either direction, out of or into the leaflet anchor tube 38. The ejector unit 36 has a guide pin 56 for engagement with the groove 52. A narrowing 54 in the groove 52 is provided to act as an indicator to let the operator know when the ejector unit 36 has reached a certain position. The size of the guide pin 56 and the width of the narrowing 54 are set so that engagement of the pin 56 with the narrowing 54 in the groove 52 will require an increased force before further movement can be made, thus providing tactile feedback to the operating physician.

The leaflet anchor deployment mechanism of figures 8 to 12 also includes a line pusher 58 for directing the artificial chordae line 14 out of and away from the leaflet anchor tube 38 during deployment of the anchor 10. The line pusher 58 directs the artificial chordae line away from the leaflet anchor tube 38 so that it can be more readily accessed for later manipulation, such as for tightening the line 14 or for pulling on the implanted leaflet anchor 10 for testing of the connection. The line pusher 58 is actuated during the action of deployment of the leaflet anchor 10, with this actuation being triggered when the leaflet anchor 10 is released from the ejector unit 36. Thus, the line pusher 50 is released when the ejector unit 36 withdraws away from the implanted leaflet anchor 10.

In the example shown, the line pusher 58 transitions from a constrained state to a nonconstrained state and moves radially outward to push the line 14 out, with this radially outward movement being permitted and the line pusher released once a constraint from the leaflet anchor 10 is removed. The line pusher 58 is an arm that extends axially forward from the ejector unit toward the leaflet anchor 10 and radially outward of the leaflet anchor tube 38 when the arm is at rest with no forces applied.

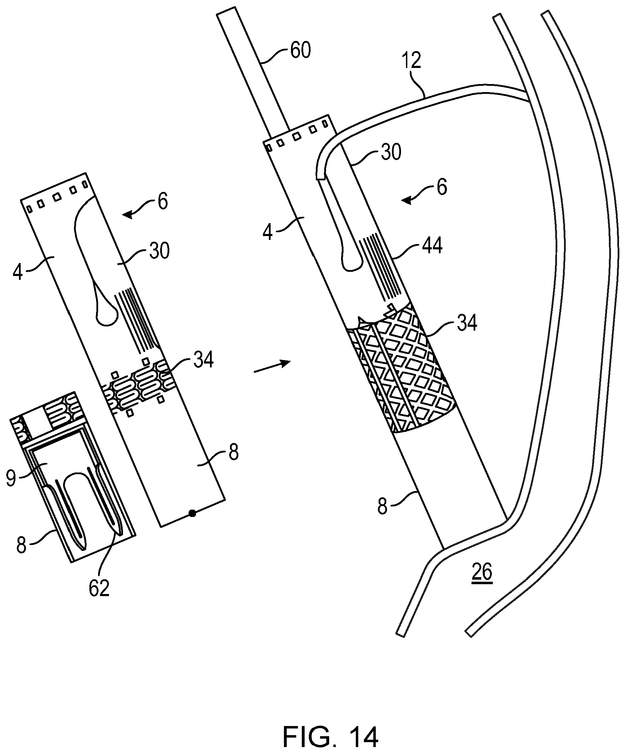

With the leaflet anchor 10 or the soft tissue anchor system discussed below implanted in the leaflet 12 the papillary anchor housing 8 at the end of the treatment catheter is then placed onto the papillary muscle 26. With the use of a flexible and extendable joint 34 this may be done as shown in figure 14. In this example, the flexible and extendable joint 34 is formed by flexible meandering sections cut into a tubular form of the main body. The flexible and extendable joint 36 is formed integrally with a tubular distal part 8, which provides the papillary anchor housing 8 and with a tubular proximal part 4, which provides the gripper housing 4. Further the tubular form of the gripper housing 4 may include an integrally formed gripper arm 30, with a weakened section 44 of the tube providing a hinge. The flexible and extendable joint 34 can be extended by means of wires and/or rods 60 (or via an adjustment catheter 21, that also may push out the papillary anchor 9), which may apply a force to stretch elastic elements of the joint 34. This extension is used to move the papillary anchor 9, within its housing part 8, to place it against the papillary muscle 26, or close to it, since the wires/rods along with the papillary anchor 8 within the distal housing part 8 move with the housing 8 as the joint 34 extends. This can be due to friction between the papillary anchor 9 (or a papillary anchor push tube) and the internal surface of the distal part 8 of the housing section. The position can be confirmed by 3D ultrasound and/or other available sources.

When the distal end of the distal part 8 meets the body tissue, and as further force is applied the counterforce from the body tissue eventually surpasses the forces holding the papillary anchor 9 in place, at this point tissue is pushed flat below the base of the distal part 8 giving a maximal chance of placing all pins 62 of the papillary anchor 9 correctly in tissue, and force can be applied to the papillary anchor 9 so that the ends of the pins 62 then move beyond the distal end of the distal part 8 to meet the body tissue. This may be done via additional force on the papillary anchor 9 from rods or wires 60 or extending the adjustment catheter 21, or it may be done through a pre-tension on the papillary anchor 9 (or friction between the adjustment catheter 21 and the distal part 8) that is held by friction with the distal part until the forces from the body tissue on the distal part 8 changes the balance of forces with the friction sufficiently so that the papillary anchor 9 ejects in a way similar to a paper stapler. As the papillary anchor 9 is ejected the pins 62 fold out and form into the hook shape of the unconstrained papillary anchor 9 to thereby engage with the body tissue 26. At this point the connection can be pull tested by operator, and/or visually confirmed on x-ray and/or ultrasound. If the connection is not satisfactory, the papillary anchor 9 can be pulled back into the distal part 8 and re-placed to attempt an improved coupling of the anchor 9 with the body tissue 26.

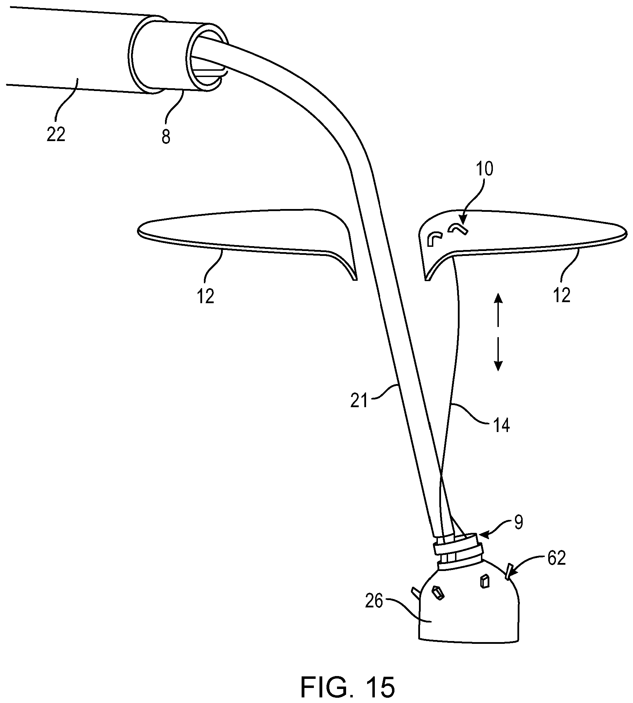

Figure 15 shows the possible next steps. The main part 4, 8 of the device is retracted to minimize influence on the moving leaflets 12. An adjustment catheter 21 can remain at the papillary anchor 9. The length of the artificial chordae line 14 can be adjusted with a wire from the outside. The length is continuously adjusted and the functioning of the leaflet 12 is monitored. The length of the artificial chordae line 14 can be reduced by pulling the chord wire back through the catheter. The length can also be increased by pushing the chord wire, which will slacken the artificial chordae line 14 and allow the movement of the leaflet 12 to pull it out of the adjustment catheter 21. The small size of the adjustment catheter 21 means that the effect of the device on the functioning of the leaflet 12 is minimised. The right length for the artificial chordae line 14 is confirmed with 3D ultrasound and/or other available sources.

When the correct length is confirmed then the device is disengaged from the papillary anchor 9. This process also locks the artificial chordae line 14 in place and cuts off any excess, which is retained in the catheter and withdrawn from the body when the catheter is removed. A locking segment 28 of the papillary anchor 9 is held open by the cutting piece (not shown). The locking segment 28 is a band of the papillary anchor 9 that can be flexed to open a gap for the artificial chordae line 14 to pass through. In the natural shape of the papillary anchor 9, when no force is applied, this locking segment 28 fits closely with the remainder of the anchor 9 and so it will hold the artificial chordae line 14 in place. The locking segment 28 is held open until the artificial chordae line 14 is the correct length. The cutting piece cuts the artificial chordae line 14, which is pulled against the blade when the adjustment process is completed.

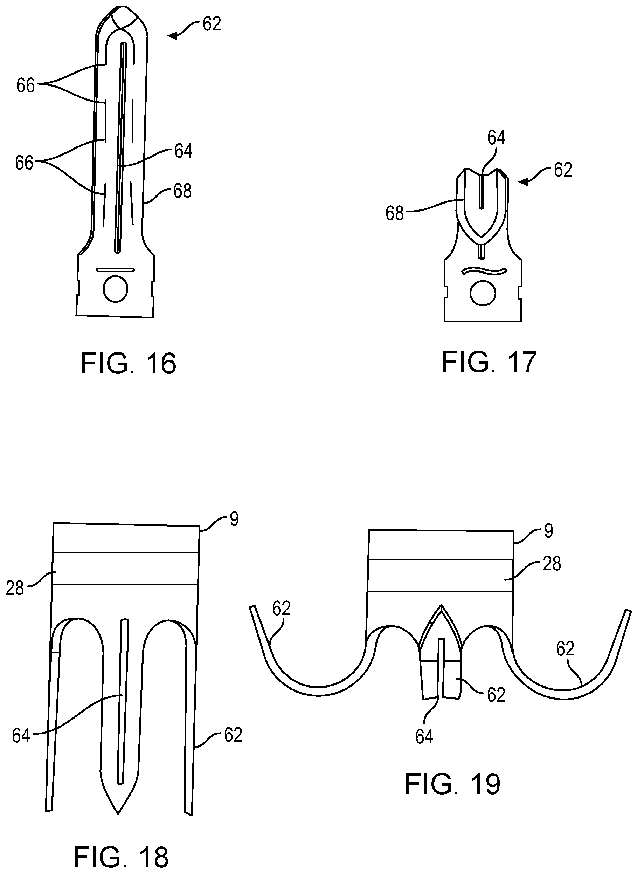

Figures 16 to 19 include more details of the papillary anchor 9, including its hooks 62 which are formed by curving pins 62. Figures 8 and 9 show one possible form for the hooks 62, with a central slit 64 and a series of holes 66 threaded with a suture 68. As discussed above, this suture 68 and the holes 66 can allow the hooks 62 to better engage with body tissue during healing, as well as keeping the material of the hooks 62 connected to the main body of the papillary anchor 9 in the event of a breakage. Figure 16 shows the folded/constrained shape of the hook 62, which is also the shape of a tine formed in a tubular section during manufacture of the anchor 9, prior to heat setting to form the curve. Figure 17 shows the curved form of the hook 62, i.e. the unfolded/unconstrained form.

Figures 18 and 19 show an example of an entire papillary anchor 9, again illustrating the folded (figure 18) and unfolded (figure 19) configurations. This papillary anchor 9 includes hooks 62 with an opening in the form of a slit 64, which may result in better engagement with the body during healing as well as increased surface area without loss of flexibility.

The catheter device 2 disclosed in each of WO2016/042022 and WO2020/109596 implants the leaflet anchor 10 from a ventricular side of the leaflet 12. As shown in figure 15, the artificial chordae line 14 therefore descends from the leaflet 12 from a ventricular surface of the leaflet 12 to the papillary muscle 26. A number of benefits are associated with implanting the leaflet anchor 10 or the soft tissue anchor system discussed below, and hence the artificial chordae line 14, in a ventricular side of the leaflet 12, as discussed in each of WO2016/042022 and WO2020/109596.

However, there may be situations in which it is advantageous to implant a leaflet anchor 110 or the soft tissue anchor system discussed below, and hence an artificial chordae line 114, from an atrial side of the leaflet 12. For example, as can be seen in figure 15 the artificial chordae line 14 descends to the papillary muscle 26 without providing any additional support to an edge 13 of the leaflet 12. When implanted from a ventricular side of the leaflet 12, the artificial chordae line 14 does not provide additional support to the edge 13 of the leaflet 12. The implanted artificial chordae line 14 may therefore not replicate the action of chordae tendineae located towards the edge 13 of the leaflet 12 as accurately as desired.

Implanting the leaflet anchor 10 or the soft tissue anchor system discussed below from a ventricular side of the leaflet 12 also requires a more precise placement of the leaflet anchor 10. As there is no additional support provided to the edge 13 of the leaflet 12, the placement of the leaflet anchor 10 will determine to what extent the edge 13 of the leaflet 12 is supported and/or secured by the leaflet anchor 12. In contrast, the placement of a leaflet anchor 110 or the soft tissue anchor system discussed below implanted from the atrial side of the leaflet 12 can be less precise, since the artificial chordae line 114 will provide additional support to the edge 13 of the leaflet 12 as it passes into the ventricle from the atrium of the heart.

Figure 20 shows the withdrawal of a guide catheter 122 and a distal part 108 of a catheter device 102 once an artificial chordae line 114 has been implanted in the papillary muscle 26 using a papillary anchor 109, and once the artificial chordae line 114 has also been implanted in the leaflet 12 of the heart using a leaflet anchor 110 or the soft tissue anchor system discussed below. The adjustment catheter 121 is shown in place before its withdrawal. The length of the artificial chordae line 114 can be adjusted as necessary in the illustrated configuration.

Figure 20 is similar to the arrangement shown in figure 15, but shows the leaflet anchor 110 implanted from an atrial side of the leaflet 12 rather than a ventricular side. As can be seen in figure 2, the artificial chordae line 114 extends from a base of the leaflet anchor 110 where it is attached towards the leaflet edge 13. As the artificial chordae line 114, when under tension or otherwise, will take the shortest possible path to the papillary muscle 26 where the other end is implanted, the artificial chordae line 114 will be in contact with the atrial side surface of the leaflet 12 and will descend, in contact, over the edge 13 of the leaflet 12. As such, the artificial chordae line 114, implanted in an atrial side of the leaflet 12, will provide additional support to the edge 13 of the leaflet 12.

The artificial chordae line 114 can comprise regions of varying cross-sectional area along its length. By increasing the cross-sectional area of the artificial chordae line 114 in certain sections, the artificial chordae line 114 can have an increased area of contact with the leaflet 12 of the heart. As such the force applied by the artificial chordae line 114 to the leaflet 12 may be more evenly distributed, and any pinching of the leaflet 12 which the artificial chordae line 114 may cause can be avoided.

The artificial chordae line 114 comprises a flattened cross-section proximal to the leaflet 12, i.e. such that a major axis of the cross-sectional area of the artificial chordae line 114 lies parallel to the surface of the leaflet 12. In alternative arrangements, the artificial chordae line 114 can be formed of a plurality of sutures, such that an area of contact between the artificial chordae line 114 and the atrial surface of the leaflet 12 is increased.

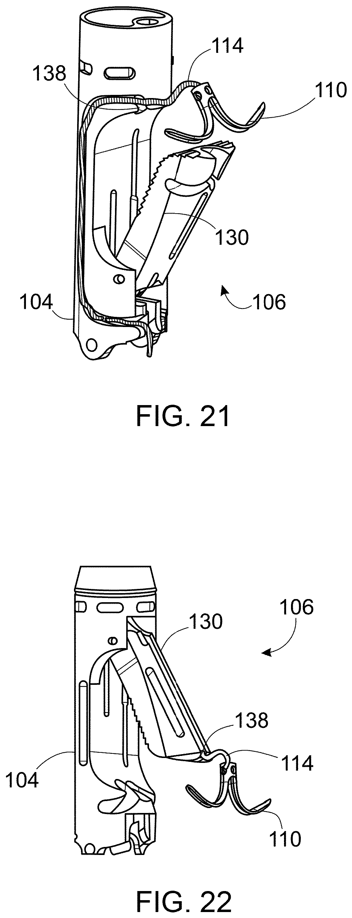

To implant the leaflet anchor 110 in the leaflet 12 of the heart from an atrial side, a leaflet anchor deployment mechanism and a gripper housing 106 of the catheter device 102 are arranged as shown in either figure 21 or figure 22.

Figure 21 shows the catheter device 102 comprising a gripper housing 106, a gripper arm 130 and the artificial chordae line 114 attached to the leaflet anchor 110 or the soft tissue anchor system discussed below and routed through the main body of the catheter device 102. In the example of these figures the leaflet anchor 110 is housed in the main body of the catheter device 102, and is deployed by pushing it out of a leaflet anchor tube 138 located in the main body of the catheter device 102. The leaflet anchor tube 138 is similar in function to the leaflet anchor tube 38 described above. The leaflet anchor tube 138 is located in the main body of the catheter device 102 such that when the leaflet 12 is grasped between the gripper arm 130 and the main body of the catheter device 102 (similarly to as shown in figure 7), the leaflet anchor 110 can be deployed from the anchor tube 138 into an atrial side of the leaflet 12. The leaflet anchor 110 will hence be deployed in the leaflet 12 as shown in figure 20. With the use of the soft tissue anchor system discussed below then the catheter device 102 can be adapted to replace the leaflet anchor tube 138 of these figures with an alternative anchor deployment mechanism, such as tubes of different design for holding and guiding the arm portion(s) of the fabric body as they are implanted into the leaflet.

Figure 22 shows an alternative arrangement of the catheter device 102 comprising a gripper housing 106, a gripper arm 130 and the artificial chordae line 114 attached to the leaflet anchor 110. Whilst not shown, the artificial chordae line will be routed through the gripper arm 130 and the main body of the catheter device 102 such that the artificial chordae line 114 can be deployed from the catheter device 102, once the line 114 is adjusted following implantation. The leaflet anchor 110 is housed in a leaflet anchor tube 110 located in the gripper arm 130, with an opening of the leaflet anchor tube 138 located at a distal end of the gripper arm 130. So that the leaflet anchor 110 can be deployed in an atrial side of the leaflet 12 as shown in figure 20, the gripper arm 130 is rotated from a proximal end of the catheter device 102, so that the opening of the leaflet anchor tube 138 will be located adjacent to the atrial side surface of the leaflet 12. This can also be adapted for use with a deployment arrangement for the soft tissue anchor system discussed below.

In both of the arrangements as shown in figures 21 and 22, the components of the catheter device 102 may function similarly to those described in relation to figures 1 to 19. The leaflet anchor tube 138 will generally operate similarly to the leaflet anchor tube 38 discussed in relation to figures 6 to 12. The gripper device 106 will function similarly to the gripper device 6 as discussed in relation to figures 2 to 6. The papillary anchor 109 will be similar to the leaflet anchor 9 discussed above.

In both of the arrangements shown in figures 21 and 22 and as discussed above, the leaflet anchor tube 138 extends in a direction along the main body of the catheter device 102 or the gripper arm 130 such that the opening of the leaflet anchor tube 138 opens towards a distal end of the catheter device 102. As the catheter device 102 approaches the leaflet 12 and the papillary muscle 26 from above the leaflet 12 and the papillary muscle, i.e. from the left atrium as discussed above, the opening of the leaflet anchor tube 138 is therefore arranged to meet the atrial surface of the leaflet 12, such that the leaflet anchor 110 can be implanted in the atrial surface of the leaflet 12.

The catheter devices taught in each of WO2016/042022 and WO2020/109596 used a U-rod to deploy the leaflet anchor. However, the catheter device 102 employs a linear rod to deploy the leaflet anchor 110. This arrangement can also be utilised for deployment of the soft tissue anchor system discussed below. The linear rod will extend from a proximal end of the catheter device 102 and into the leaflet anchor tube 138, such that the leaflet anchor 110 or the soft tissue anchor system discussed below can be deployed into the atrial side of the leaflet 12. A linear rod may deploy an anchor system by pushing it out of the distally-facing opening of the leaflet anchor tube 138, using the end of the linear rod located in the leaflet anchor tube 138. The linear rod is flexible so that it can curve or bend, e.g. from the main body of the catheter device 102 shown in figure 22 and into the gripper arm 130 when flexed, and is tensile so that it can be pushed into and retracted from the leaflet anchor tube 138 without elongating. When the leaflet anchor tube 138 is located in the gripper arm 130 as shown in figure 22, the elastic properties of the linear rod can help restore the gripper arm 130 to the closed position, i.e. flush to the main body of the catheter device. The linear rod is made of a material with the ability to deform elastically to a high degree in order to allow for the bending of the bendable section. Suitable materials include shape memory materials, for example shape memory metals such as nitinol. Using a shape memory metal also means that the linear rod can be made to be stiff, which makes the transfer of force with the linear rod more efficient. Alternatively, the linear rod could be made of several types of materials to achieve the required properties.

Whilst the following features will be discussed in relation to the catheter device 102 as discussed in relation to figures 20 to 22, it will be appreciated that the following features are similarly compatible with the catheter device 2 as discussed in relation to figures 1 to 19 and as disclosed in each of WO2016/042022 and WO2020/109596.

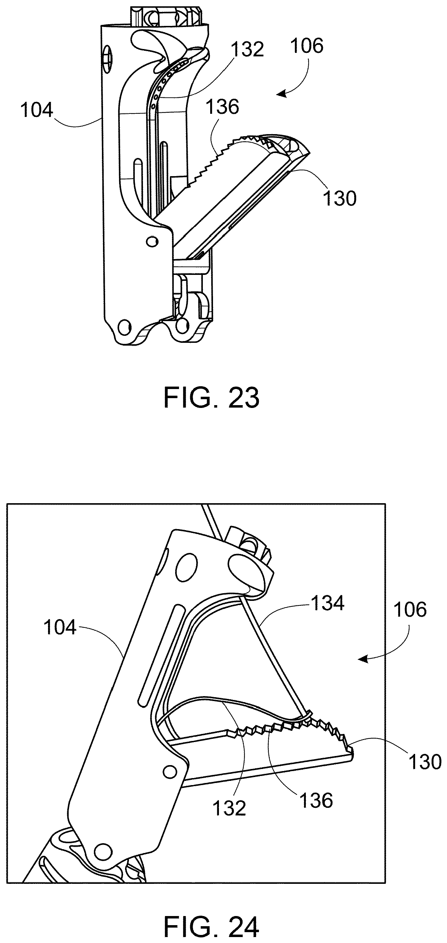

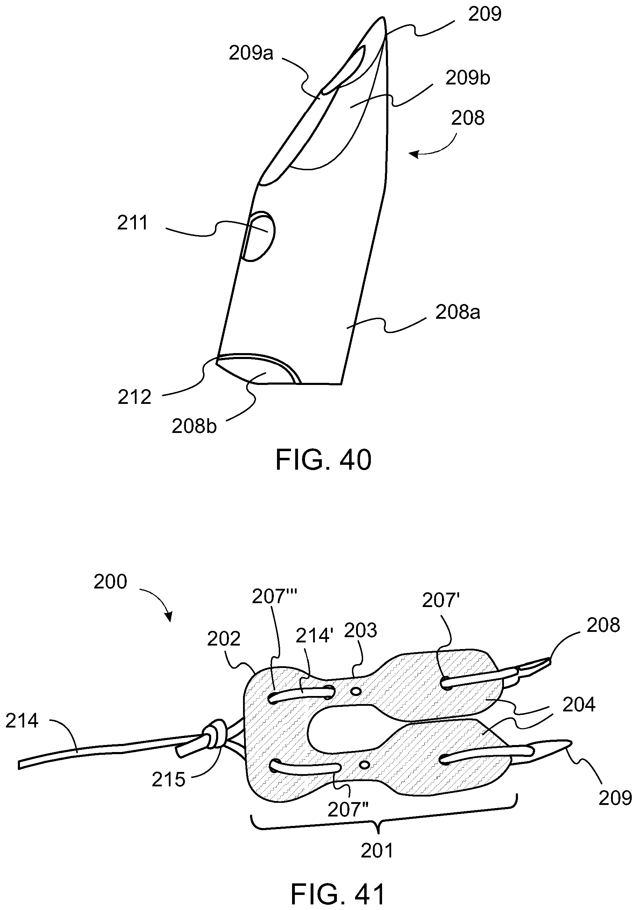

Figures 23 and 24 show an alternative arrangement for the gripper device 106. The gripper device 106 comprises the gripper arm 130 in combination with a gripper lever 132. The gripper lever 132 is biased such that at rest, i.e. in an undeployed configuration, the gripper lever will sit flush to the main housing of the catheter device 102 as shown in Figure 41. The gripper lever 132 is located between the gripper arm 130 and the main body of the catheter device 102, such that the gripper lever 132 cannot be actuated away from the main body of the catheter device 102 without first opening the gripper arm 130 as shown in figure 24.

The gripper lever 132 is fixed at an end adjacent to where the gripper arm 130 is rotated from. The other end of the gripper lever 132 is free to move relative to the main body of the catheter device 102. The free end of the gripper lever 132 is attached to a wire or rod 134 which runs through the main body of the catheter device 102 and pushes the free end of the gripper lever 132. When pushed, the rod 134 therefore actuates the gripper lever 132 such that it is in a deployed configuration. In the deployed configuration, free end of the gripper lever 132 is arranged to meet the gripper arm 130. Thus, when in use, the leaflet 12 can be grasped between the gripper arm 130 and the gripper lever 132.

The combined grasping action of the gripper arm 130 and the gripper lever 132 can help ensure that the leaflet 12 is correctly grasped. The gripper arm 130 can be opened so that a gripping surface 136 of the gripper arm 130 meets the leaflet 12. Without additional support the leaflet 12 may move away from the gripper arm 130 as the gripper arm 130 is closed, due to the motion of the leaflet 12 during the cardiac cycle. However, the present arrangement of the gripper device 106 deploys the gripper lever 132 before the gripper arm 130 is closed. The leaflet 12 is therefore secured between the gripper lever 132 and the gripper arm 130 before the gripper arm 130 is closed. Finally, the gripper lever 132 and the gripper arm 130 can be withdrawn, with the leaflet 12 still secured, so that the leaflet 12 is secured in the desired position between the gripper arm 130 and the main body of the catheter device 102 when the gripper arm 130 is in the closed position. The leaflet anchor 110 can then be deployed in the desired location. Thus, the provision of the gripper lever 132 may help increase the likelihood of successfully grasping the leaflet 12, and may ensure the correct positioning of the leaflet anchor 110 or the soft tissue anchor system discussed below in the leaflet 12 during implantation of the leaflet anchor 110 or the soft tissue anchor system discussed further below.

The gripper lever 132 can include a number of indentations or teeth along its length, which may assist in grasping the leaflet 12. The indentations or teeth increase the frictional hold of the gripper lever 132, such that the leaflet 12 is less likely to accidentally release from the gripper arm 130 and the gripper lever 132 when grasped. The gripper lever 132 is generally flexible so that it can be held at the end at which the gripper arm 130 is rotated from and can be pushed at the free end by the rod 134 to meet the gripper arm 130. The gripper lever 132 and the rod 134 may each be formed of a suitable elastic, yet tensile, material such as nitinol or stainless steel. The gripper lever 132 can be secured to the main body of the catheter device 102 and the rod 134 by welding or gluing the components together.

Whilst the gripper lever 132 has been described herein as being flush to the main body of the catheter device 102 when unconstrained, and contacting the gripping surface 136 when in the rod 134 is actuated, the gripper lever 132 could alternatively be flush to the gripping surface 136 of the gripper arm 130 when unconstrained. Accordingly, the gripper lever 132 will be opened away from the gripper arm 130 when the rod 134 pulls the free end of the gripper lever 132. The leaflet 12 can then be grasped between the open gripper lever 132 and the open gripper arm 130. Releasing the rod 134 will therefore cause the gripper lever 132 to return to its unconstrained position, and hence grasp the leaflet 12 between itself and the gripper arm 130. The gripper lever 132 being biased to grasp the leaflet 12 when no forces are applied may result in a more secure and/or reliable hold of the leaflet 12.

As briefly mentioned above, embodiments of the present invention pertain to soft tissue anchor systems as now described herein. The soft tissue anchor systems described herein can be used in the catheter device 2 of the type described above in connection with figures 1 to 24, for which purpose the leaflet anchor 10, 110 shown in those figures may be replaced by the soft tissue anchor system described below and discussed in relation to the remaining figures, with other modifications being apparent to the person skilled in the art.

The soft tissue anchor systems illustrated in, or discussed in relation to, the remaining figures is for implantation in soft body tissue and, more specifically, can be used as a leaflet anchor system for use in the surgical repair of mitral valve leaflets, the leaflet anchor system being used to attach an artificial line to a leaflet of the heart during the repair. This type of repair is discussed in both WO2016/042022 and WO2020/109588, and as described above in relation to figures 1 to 24.

In contrast to the concepts disclosed in WO'022 and WO'588, however, in some embodiments the present anchor system uses a fabric-type arrangement in which the two arms of a U-shaped fabric body pass through the body tissue, before collapsing in a concertina-like manner to sandwich the body tissue between the base portion of the U-shaped fabric body and each arm portion of the U-shaped fabric body.

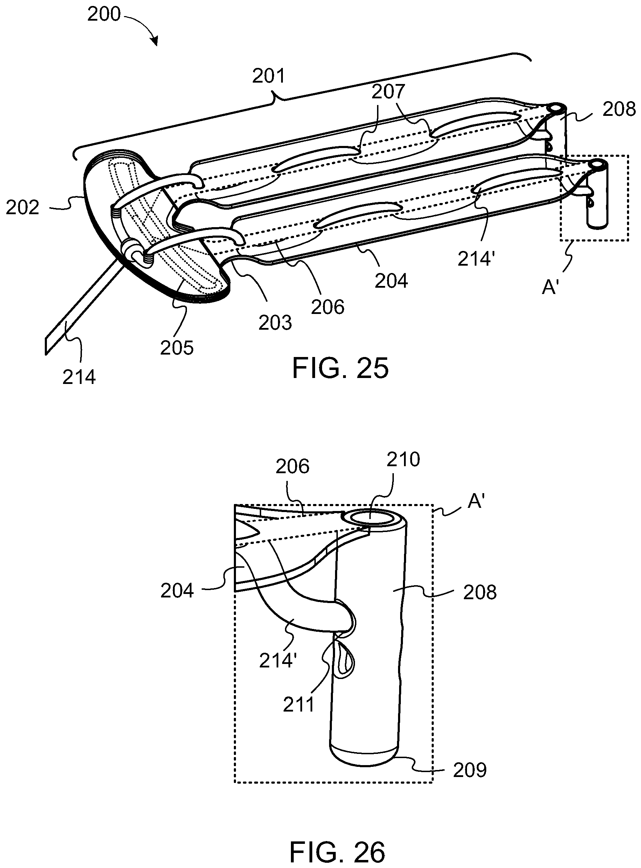

Figure 25 illustrates a soft tissue anchor system 200 comprising a U-shaped fabric body 201, in accordance with the present invention. The U-shaped fabric body 201 comprises a base portion 202 and two arm portions 204 extending from the base portion 202. A narrow waist portion 203 extends between the base portion 202 and each respective arm portion 204. Soft tissue anchor systems 200 comprising a plurality of arm portions 204 connected by a single base portion 202 may increase a surface area of the anchor system 200 when engaged with body tissue, on both sides of the body tissue. This may improve the stability of the anchor system 200 when implanted in body tissue.

The U-shaped fabric body 201 is used to anchor an artificial chordae line 214, located towards the base portion 202, to body tissue. Being a fabric body, it will be understood that the U-shaped fabric body 201 is primarily formed of a soft material. The U-shaped fabric body 201 may be comparable in function to a pledget. In some embodiments, and as illustrated in figure 25, the base portion 202 comprises a shape retention feature 205 embedded therein, such as a nitinol wire frame or the like. The shape retention feature 205 may help the base portion 202 retain its shape over time and/or provide additional lateral support to the body tissue when implanted. The arm portions 204 can also optionally be provided with stiffening elements 206.

The U-shaped fabric body 201 also comprises a tensile line 214'. Each arm portion 204 comprises a portion of tensile line 214' threaded through the arm 204 and the base 202, the threading running from an end of the arm portion 204 distal to the base portion 202, to the base portion 102. Each portion of tensile line 214' is fixed towards the distal ends of the arm portions 204, but is otherwise free to move with respect to the threaded holes 207 formed along the arm portions 204 and in the base portion 202.

Each arm portion 204 is configured to collapse in folds towards the base portion 202, by action of the tensile line 214' threaded through the arm portions 204. That is, the tensile line 214' can collapse the arm portions 204 in folds towards the base portion 202 when a tension force is applied to the tensile line 214'. In other words, the tensile line 214', threaded through the arm portions 204, is an example of a means for collapsing each arm portion 204 in folds towards the base portion 202 such that, in use, the body tissue is sandwiched between the base portion 202 and each of the arm portions 204. As the U-shaped fabric body 201 is formed of a primarily soft material, actuation of the tensile line 214' from an end distal to where the portions of line 214' are fixed to the arm portions 204 results in the arm portions 204 collapsing in a concertina fashion (i.e. collapsing in folds towards the base portion 202).

Also located at the ends of the arm portions 204 distal to the base portion 202 are end caps 208. The end caps 208 are rigid structures which aid implantation of the U-shaped fabric body 201 in the body tissue, and also assist in maintaining engagement of the U-shaped fabric body 201 to the body tissue.

Figure 26 shows detail A' of figure 25 from a closer perspective. The portions of line 214' threaded through the arm portions 204 are fixed to the distal ends of the arm portions 214' via the end caps 208. In the embodiment illustrated in figures 25 and 26, each respective end of the tension line 214' is received by an opening 211 of each respective end cap 208, and is fixed therein. Each end cap 208 also comprises an opening 210 for receiving a wire guide member 218, as discussed in further detail below. The use of end caps 208 which receive wire guide members 218 for implanting the anchor system 200 may facilitate ease of implantation, because the fabric body 201 need not be implanted from within a hollow needle. Accordingly, trauma to the site of implantation will be reduced, as a narrower piercing member may be used to deploy the soft tissue anchor system 200.

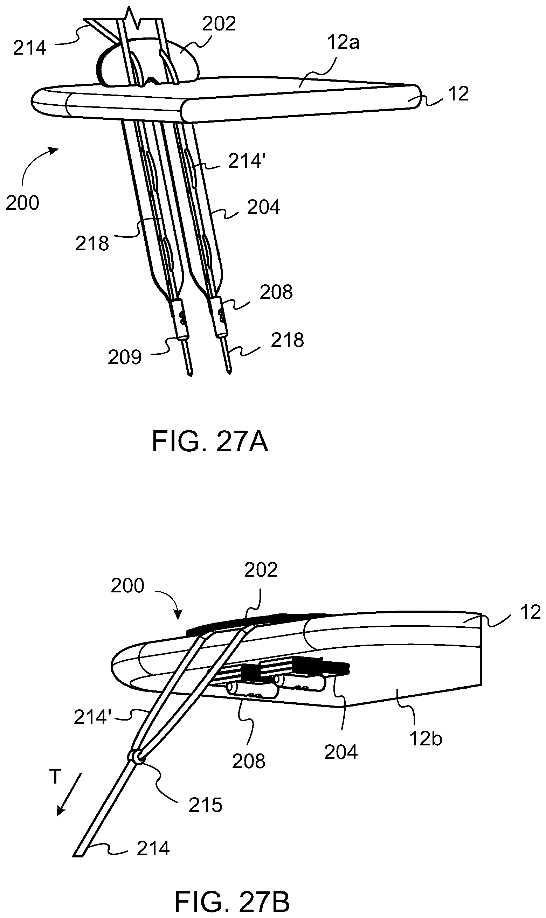

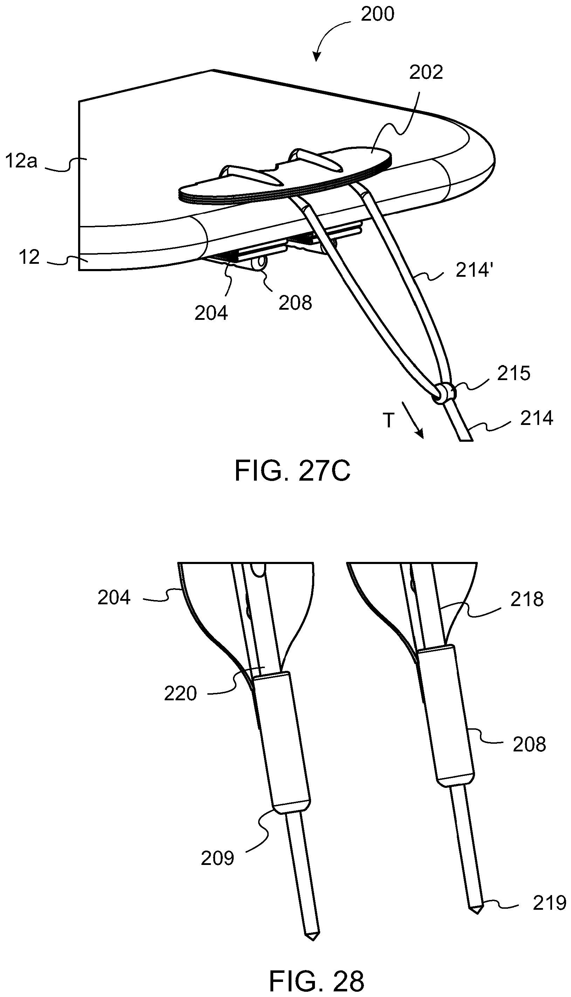

Figures 27A, 27B and 27C show the soft tissue anchor system 200 implanted in a mitral valve leaflet 12 of the heart. The base portion 202 is arranged to contact an atrial surface 12a of the leaflet 12, and the concertinaed arm portions 204 (as in figures 27B and 27C) are arranged to contact a ventricular surface 12b of the leaflet 12. The leaflet 12 is therefore sandwiched between the base portion 202 and the arm portions 204, in use.

As can be seen in figures 27B and 27C, the artificial chordae line 214 provides a tensile force as indicated by arrow T to the tensile line 214', thereby drawing the fixed ends thereof towards the base portion 202. This results in the arm portions 204 collapsing in folds into the concertinaed position.

The artificial chordae line 214 can be joined to the tensile line 214' via any suitable fastening means, for example a knot or an eyelet. In the embodiment shown in figure 27B, the artificial line 214 is joined to the tensile line 214' via a knot 215.

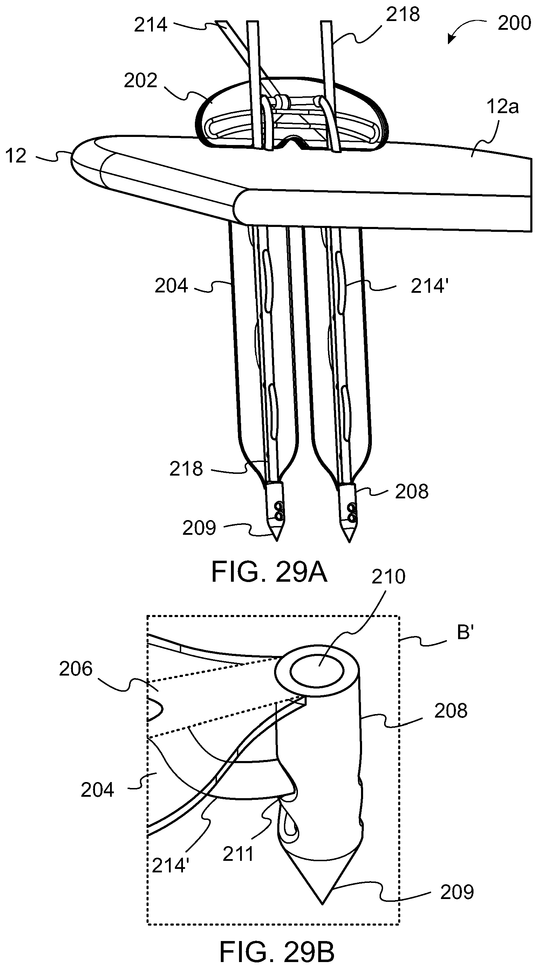

Referring now to figure 27A and also figures 29A and 29B, to implant the U-shaped fabric body 201, a pair of wire guide members 218 are employed. The wire guide members 218 are each received by the opening 210 of the end cap 208. The wire guide members 218 are used to push the end caps 208 into a piercing engagement with the leaflet 12, and through the leaflet 12, such that the U-shaped fabric body 201 engages the leaflet 12. In this configuration, the end caps 208 extend collinearly with the arm portions 204. The wire guide members 218 can then be retracted once the U-shaped fabric body 201 is implanted in the leaflet 12. Withdrawal of the wire guide members 218 may result in the end caps 208 returning to an orientation perpendicular with the arm portions 204 at rest, or parallel to a plane of the folds of the arm portions 204 when the arm portions 204 are collapsed (as in figures 27B and 27C, for example).

Figure 28 shows the end caps 208 when engaged with the wire guide members 218, in further detail. The end caps 208 have a tip 209 which, in each of figures 25 to 28, is a dulled or rounded tip 209. Providing a dulled or rounded tip 209 may reduce a risk of laceration or abrasion of the leaflet 12 by the end cap 208 once implanted in the leaflet 12.

The wire guide member 218 illustrated in figure 27A and figure 28 passes through the end cap 208 and is itself used to pierce the leaflet 12, whilst simultaneously pushing the end caps 208 through the leaflet 12. In the arrangement shown in figures 27A and 28, the wire guide member 218 comprises a piercing section 219. The piercing section 219 comprises a distal tip that is configured to pierce the leaflet 12 during implantation of the soft tissue anchor system 200. Thus, in the arrangement shown in figures 27A and 28, the wire guide member 218 may be regarded as a piercing wire guide member 218. The opening 210 of the end cap 208 appreciably extends along the entire length of the end cap 208, to enable passage of the piercing wire guide member 218 therethrough. To aid with manipulation of the end caps 208, the wire guide member 218 comprises a thicker control section 220. A shoulder portion 221 is located between the thinner piercing section 219 and the thicker control section 220, and is configured to engage a complementary portion of the end cap 208. Transmitting a force to the end caps 208 in this manner results in the arm portions 204 being pulled through the implantation site created by the respective piercing sections 219 of the wire guide members 218.

In other arrangements not illustrated, however, the wire guide member 218 may comprise a first hollow shaft used to push the end caps 208, with a second piercing wire passing through the hollow shaft for piercing the tissue. This configuration would give more degrees of freedom, but also comprises more components.

As shown in figures 29A and 29B, in some embodiments the tip 209 of the end cap 208 distal to the arm portions 204 is pointed, such that the end cap 208 is capable of piercing the leaflet 12. Where the tip 209 of the end cap 208 is pointed, the wire guide members 218 are used to transmit a force to the end caps 208 to both pierce the leaflet 12 and to pull the arm portions 204 through the implantation sites created in the leaflet 12 by the pointed tips 209 of the end caps 208.

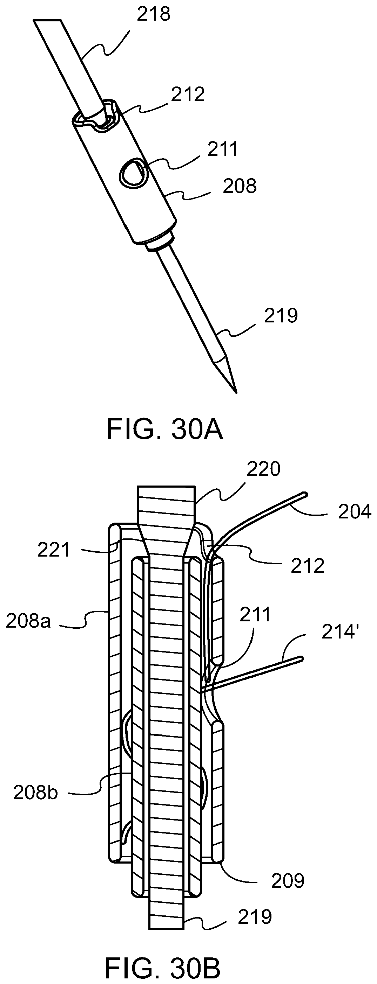

Figures 30 to 32 illustrate various arrangements of an end cap 208 having a dulled or rounded tip 209. In each arrangement, the end cap 208 comprises an outer tubular member 208a and an inner tubular member 208b. The inner tubular member 208b is received by the outer tubular member 208a, such that the inner tubular member 208b is concentrically nested within the outer tubular member 208a. The outer tubular member 208a and the inner tubular member 208b each define openings extending along their length, such that the resulting end cap 208 can receive the wire guide member 218 and, in each of the arrangements illustrated in figures 30 to 32, such that the piercing section 219 can pass completely through the end cap 208. The inner tubular member 208b defines the opening 210 for receiving the wire guide member 218.

The outer tubular member 208a defines the opening 211 for receiving the tension line 214', as well as a notch or groove 212 for facilitating a connection of the arm portion 204 of the U-shaped fabric body 201 to the end cap 208. The notch 212 can assist with positioning the arm portion 204 relative to the end cap 208, such that the opening 210 of the end cap 208 is not obscured or blocked by the end cap 208.

As shown in each of figures 30 to 32, the tension line 214' and the arm portion 204 is received between the outer tubular member 208a and the inner tubular member 208b. The arm portion 204 extends along the inner tubular member 208b, whilst the tension line 2124' is wrapped around the inner tubular member 208a. The tension line 214' and the arm portion 204 are fixed between the outer tubular member 208a and the inner tubular member 208b by crimping the outer tubular member 208a. Accordingly, the outer tubular member 208a is made of a crimpable material such as stainless steel, titanium or the like. The inner tubular member 208b is formed of a shape retention material, such as nitinol, stainless steel, titanium or the like. In the presently illustrated embodiments, the outer tubular member 208a has an outer diameter of 1.0 to 1.1mm, and a length of 3mm.

In each of figures 30 to 32, the shape of the wire guide member 218 is illustrated in further detail. As described above, the wire guide member 218 comprises a control section 220, for transmitting a motive force to the end cap 208, a piercing section 219 for piercing the leaflet 12, and a shoulder portion 221 extending therebetween. In the presently illustrated embodiments the control section 220 has a diameter of 0.4mm and the piercing section 219 has a diameter of 0.3mm. The shoulder portion 221 is sloped, and accordingly defines the transition between the control section 220 and the piercing section 221.

Turning to specifically the first arrangement illustrated in figures 30A and 30B, the end cap 208 has a dulled tip 209. The inner tubular member 208b and the outer tubular member 208a are misaligned, such that the inner tubular member 208b extends out of the tip 209 of the outer tubular member 208a, and sits below the opening of the outer tubular member 208a. Accordingly, a step transition is defined between the inner tubular member 208b and the outer tubular member 208a at the opening 210 of the end cap 208. This transition provides a surface for the shoulder portion 221 of the wire guide member 218 to dock with, and thereby transmit a motive, or pushing force, to the end cap 208.

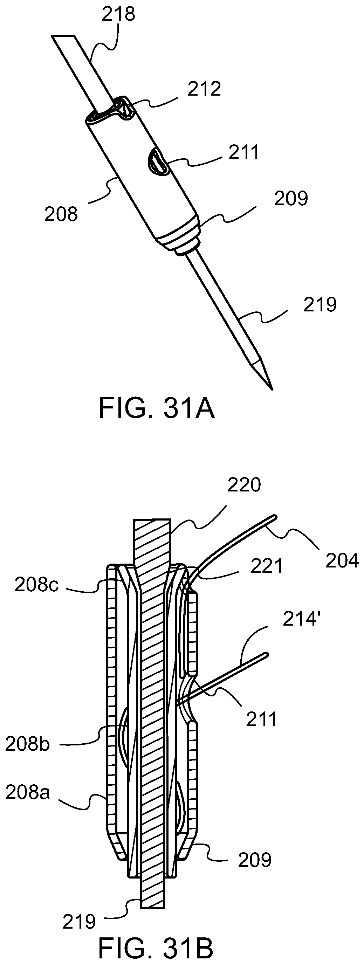

In a second arrangement illustrated in figures 31A and 31B, the end cap 208 comprises a rounded tip 209. Accordingly, the tip 209 of the outer tubular member 208a is itself tapered, or rounded. Providing a taper, or flare, to the tip 209 of the outer tubular member 208a may help provide an interference fit for maintaining the inner tubular member 208b within the outer tubular member 208a. Additionally, the inner tubular member 208a comprises a flared inlet 208c defining the opening 210, and sitting flush with the opening of the outer tubular member 208a. The flared inlet 208c has a shape complementary to the shoulder portion 221 of the wire guide member 218, to facilitate docking of the wire guide member 218 with the end cap 208.

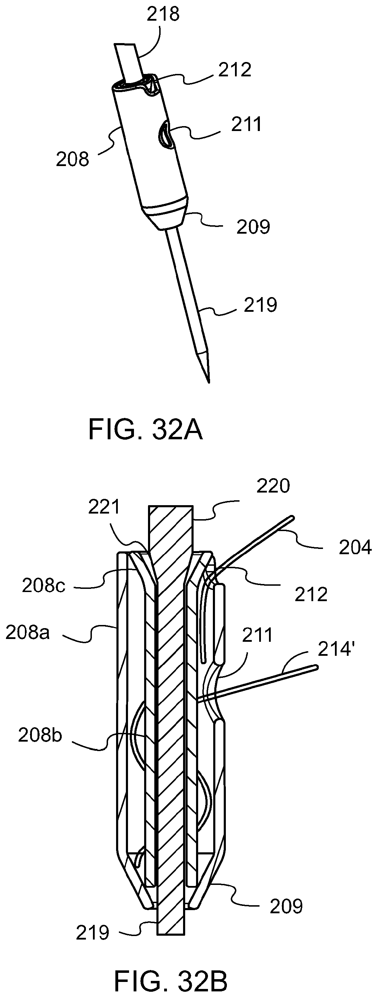

In a third arrangement illustrated in figures 32A and 32B, the inner tubular member 208b also comprises a flared inlet 208c sitting flush with the opening of the outer tubular member 208a. However, in the third arrangement the inner tubular member 208a does not extend out of the tip 209 of the outer tubular member 208a. Instead, the inner tubular member 208b abuts an internal surface of the tip 209 of the outer tubular member 209a. Accordingly, the tip of the end cap 208 is defined completely by the tip 209 of the outer tubular member 208a. The end cap 208 thus has a smooth tip 209 with which to pass through the leaflet 12 during implantation.

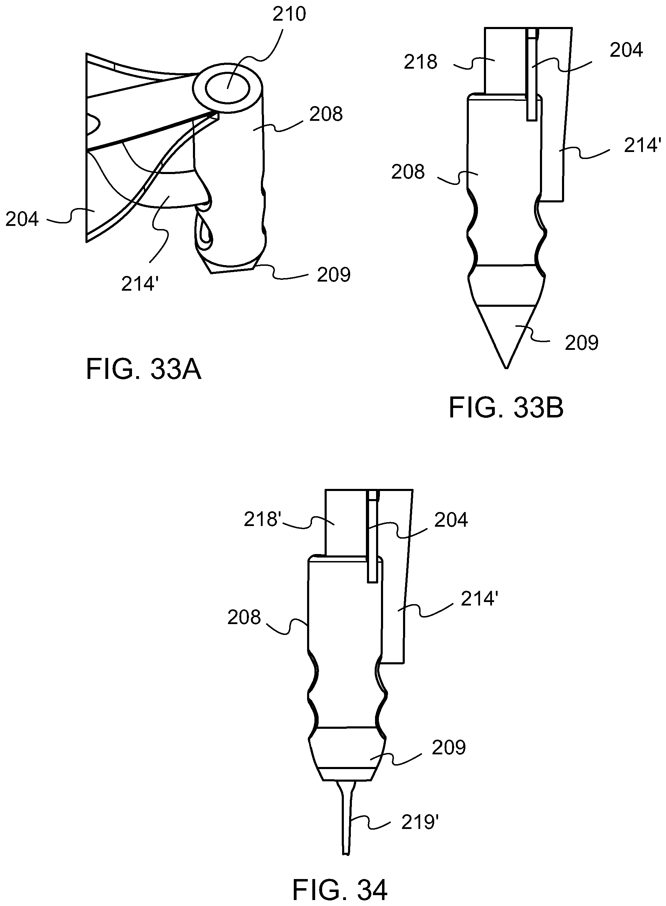

Figures 33A and 33B illustrate another alternative arrangement for the end cap 208. Figure 33A shows the end cap 208 in a first configuration, in which the wire guide member 218 is not docked or engaged with the end cap 208. In the first configuration, the tip 209 of the end cap 208 is dulled, or rounded. However, when the wire guide member 218 engages the end cap 208 via the opening 208, the tip 209 of the end cap 208 is pointed, such that it can pierce the leaflet 12. Accordingly, in the arrangement illustrated in figures 33A and 33B, the tip 209 of the end cap 208 is a retractable pointed tip 209.

Figure 34 shows another embodiment, where a wire guide member 218' is separate from a piercing wire member 219'. The piercing wire member 219' is arranged to pass through both the end cap 208 and the wire guide member 218'. The piercing wire member 219' is pushed through the leaflet 12 so as to pierce it, and acts as a guide for the end cap 208 and the wire guide member 218' engaging the end cap 208. Accordingly, the wire guide member 218' acts to push the end cap 208 over the thinner piercing wire member 219', such that the end cap 208, and the arm portion 204 attached to it, is passed through the leaflet 12 and hence implanted.

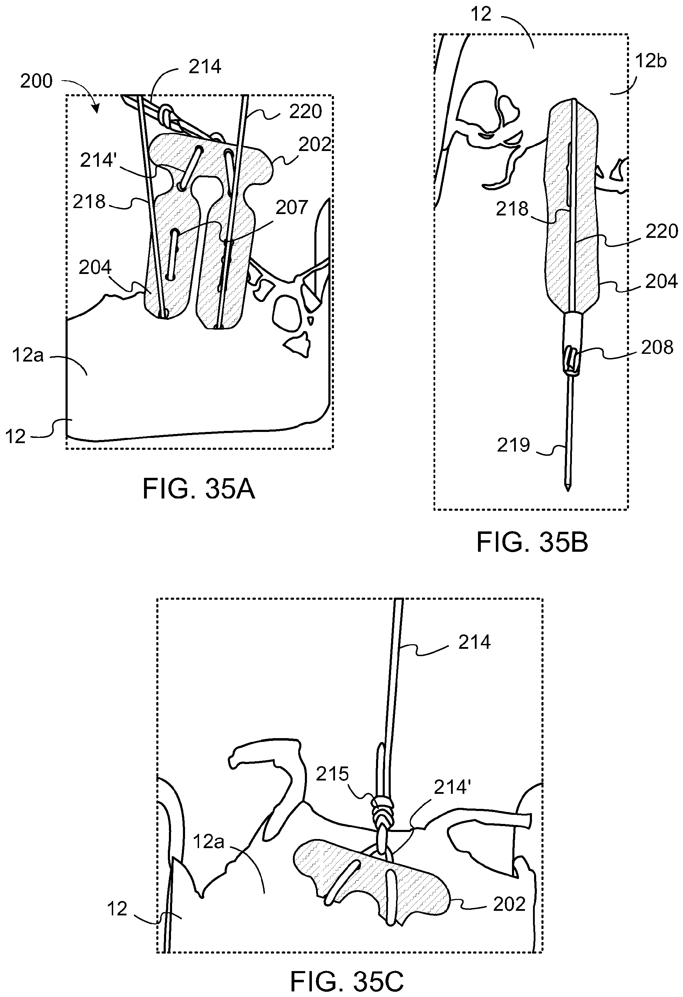

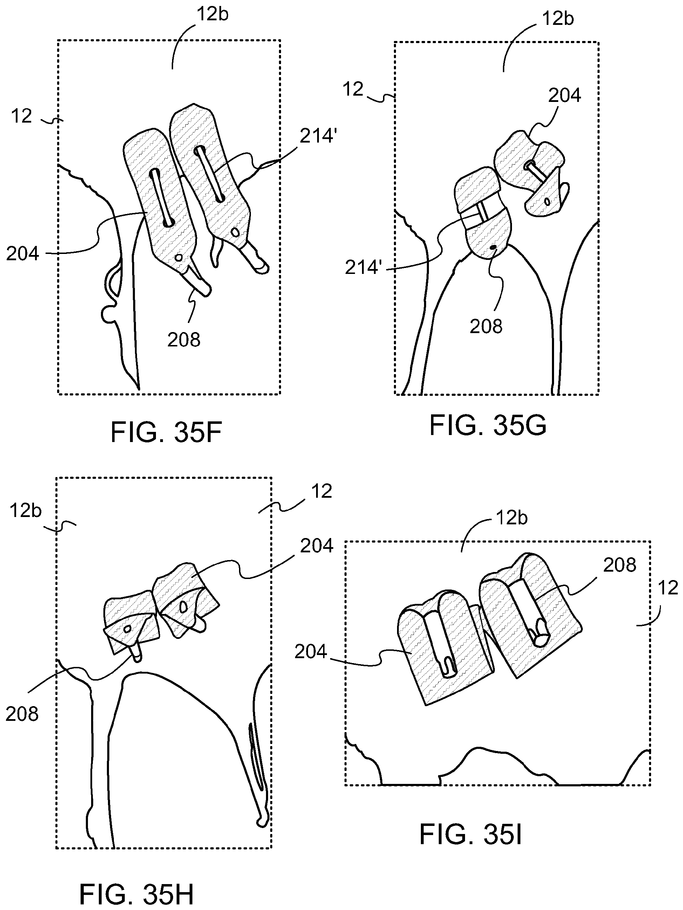

Figures 35A to 35I illustrate a soft tissue anchor system 200 at various stages during its implantation in a leaflet 12. Whilst the soft tissue anchor system 200 illustrated comprises an end cap 208 having a wire guide member 218 comprising a piercing section 219 pass therethrough, it will be readily appreciated that the general stages of implantation discussed herein are applicable to various embodiments of a soft tissue anchor system 200 described herein.

Firstly, a wire guide member 218 engages each end cap 208 of the soft tissue anchor system 200. The wire guide member 218, by application of a force, results in piercing of the leaflet 12, and the pulling of the arm portions 204 through the leaflet 12. In the present embodiment, the piercing section 219 of the wire guide member 218 initially pierces the leaflet 12, and the end caps 208 are pushed through the implantation sites by action of the control section 220. Figure 35A illustrates the arm portions 204 of the fabric anchor body 201 initially passing through the leaflet 1 from the atrial side.

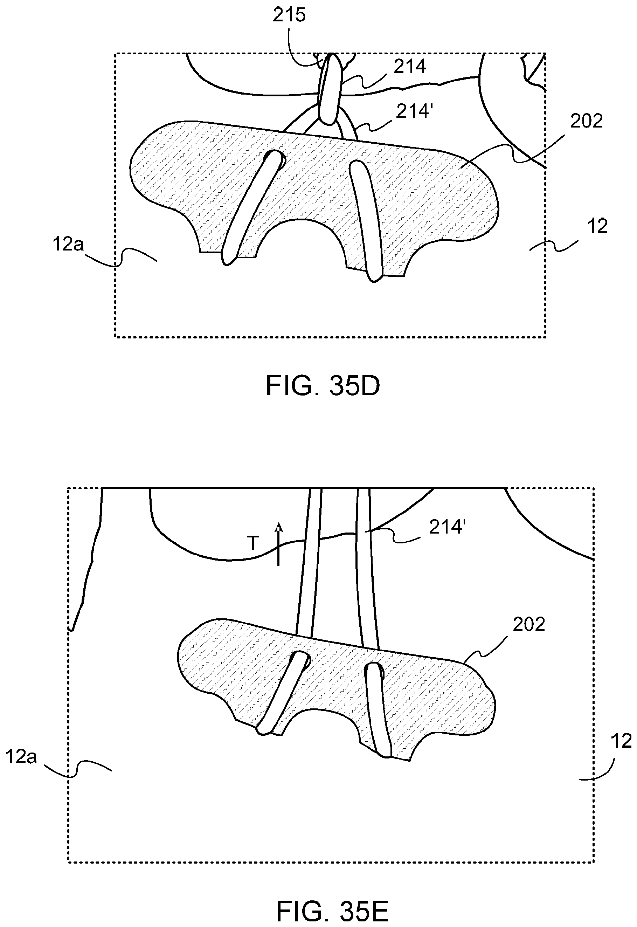

The wire guide members 218 are then manipulated such that the arm portions 204 are completely passed through the leaflet 12, as shown in figure 35B. The arm portions 204 are completely passed through the leaflet 12 when the base portion 202 sits adjacent the leaflet 12, or is in contact with the leaflet 12, as shown in figures 35C and 35D. The narrow waist portions 203 of the arm portions 204 are aligned with the leaflet 12 when the arm portions 204 are fully implanted in the leaflet 12.

Once the arm portions 204 are implanted in the leaflet 12, the wire guide members 218 are withdrawn such that they no longer engage the end caps 208, and pass back through the leaflet 12 via the implantation sites of the arm portions 204.

With the wire guide members 218 withdrawn, a tension force T is then applied to the tension line 214' by pulling the artificial line 214. As shown in figures 35C and 35D, the artificial line 214 is fixed to the tension line 214' via a bridle knot 215. A bridle knot allows relative movement between the artificial line 214 and the tension line 214'. The tension force T is applied in a direction away from the surface of the leaflet 12.

Application of the tension force T causes the tension line 214' to be withdrawn back through the leaflet 12. The position of the knot 215 to the leaflet 12 will change accordingly change, such that the knot 215 moves away from the base portion 202 (as seen in figure 35E).

As the tension line 214' withdraws, it collapses the arm portions 204 in folds towards the base portion 202, such that the leaflet 12 is sandwiched between the folded arm portions 204 and the base portion 202. The concertina motion of the arm portions 204 is illustrated in figures 35F to 35H. Since the tension line 214' is attached to the end caps 208 via the central openings 211 formed in a wall of the end caps 208, and since the arm portions 204 are joined to the end caps 208 towards the opening 210 for receiving the wire guide member 218, the end caps 208 are motivated into a plane parallel to the folds/the surface 12b of the leaflet 12, further assisting with the collapsing of the arm portions 204 in folds.

Finally, as shown in figure 35I, the folds of the arm portions 204 and the end caps 208 themselves line in a plane parallel to the surface 12b of the leaflet 12.

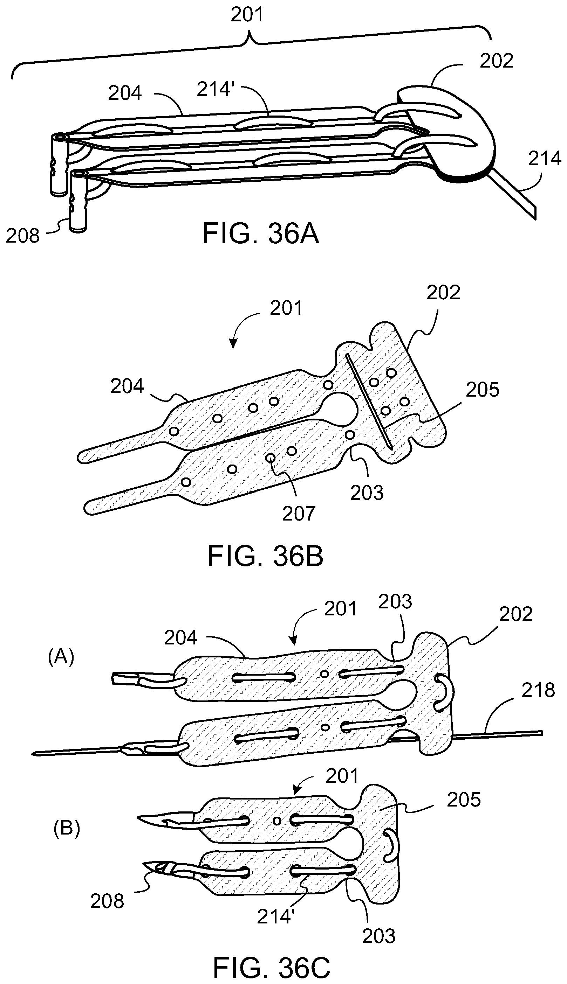

Figures 36A to 36C are provided to illustrate the various constructions of a soft tissue anchor system 200 comprising a U-shaped fabric body 201. Figure 36A is a schematic representation of the soft tissue anchor system 200, and is provided as a useful juxtaposition with figures 36B and 36C, which each show prototypes of the soft tissue anchor system 200 comprising a U-shaped fabric body 201.

The U-shaped fabric body 201, as shown in figure 36B, can be formed from a single piece of fabric defining the arm portions 204, the narrow waist portions 203 and the base portion 202. To form the base portion 202, a portion of the fabric body 201 may be folded back over on itself, and can optionally sandwich a shape retention member 205 therebetween, as shown in figure 36B.

Figure 36C shows two prototype soft tissue anchor systems 200, according to embodiments of the present invention. One anchor system 200 comprises a shape retention member 205 (see prototype A), whilst the other does not (see prototype B). Prototype B is of a shorter length of around 16mm, whilst prototype A is longer. The length of the arm portions 204 can accordingly be varied during manufacture of the U-shaped fabric body 201.

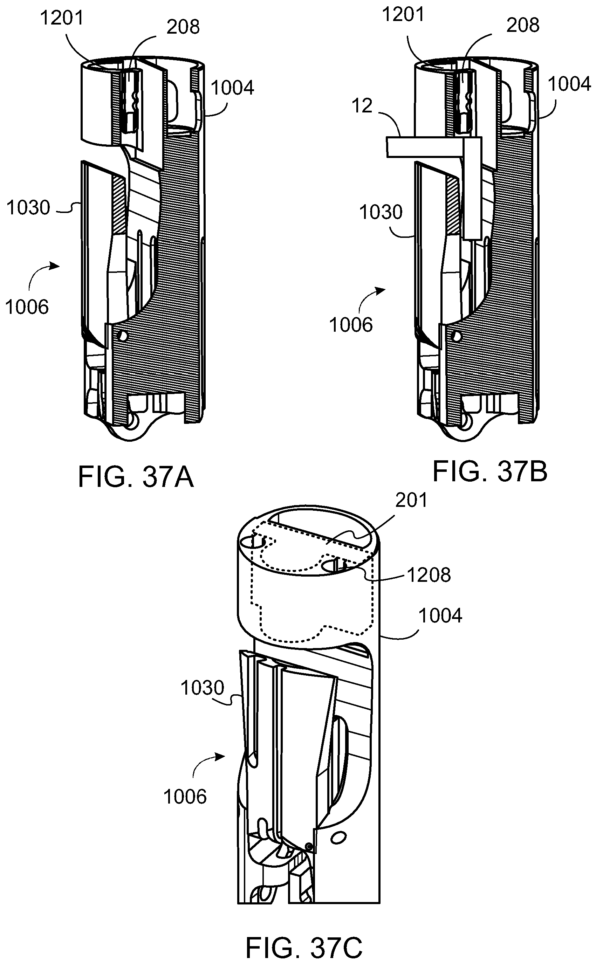

Figures 37A to 37C schematically illustrate how soft tissue anchor systems 200 according to embodiments of the invention may be housed by, and deployed from, a catheter device. Each of figures 37A to 37C show only a proximal part 1004 of a catheter device, but it will readily be appreciated that such a proximal part 1004 could be implemented in a catheter device as discussed in relation to any of figures 1 to 24.

Figure 37A shows an end cap 208 of a soft tissue anchor system 200 housed in a channel or groove 1208 for deployment from the proximal part 1004 of a catheter device. The proximal part 1004 functions as a gripper housing 1006, and accordingly the channel 1208 faces the gripper arm 1030 such that when a leaflet 12 is grasped by the gripper arm 1030, as illustrated in figure 37B, the end cap 208 can be deployed from the channel 1208 by actuation of a respective wire guide member 218.

The proximal part 1004 of the catheter device also provides a housing 1201 for the U-shaped fabric body 201. Figure 37C illustrates the U-shaped fabric body 201 when stowed in the housing 1201. The U-shaped fabric body housing 1201 also faces the gripper arm 1030, such that the U-shaped fabric body 201 can be implanted into a grasped leaflet 12.

The U-shaped fabric body 201 is placed in a thin tubular sheath, or curtain, in the housing 1201. The sheath acts to reduce friction experienced by the U-shaped fabric body 201 from the housing 1201 during deployment of the soft tissue anchor system 200 from the catheter device. The sheath can deflect, or crumple, during deployment to aid deployment of the fabric body 201.

The U-shaped fabric body housing 1201 is open to the channels 1208 for the end caps 208, such that the arm portions 204 can extend between the end cap channels 1208 and the U-shaped fabric body housing 1201. This arrangement may accordingly facilitate smooth deployment of the soft tissue anchor system 200 from the proximal part 1004 of the catheter device, into a grasped leaflet 12.

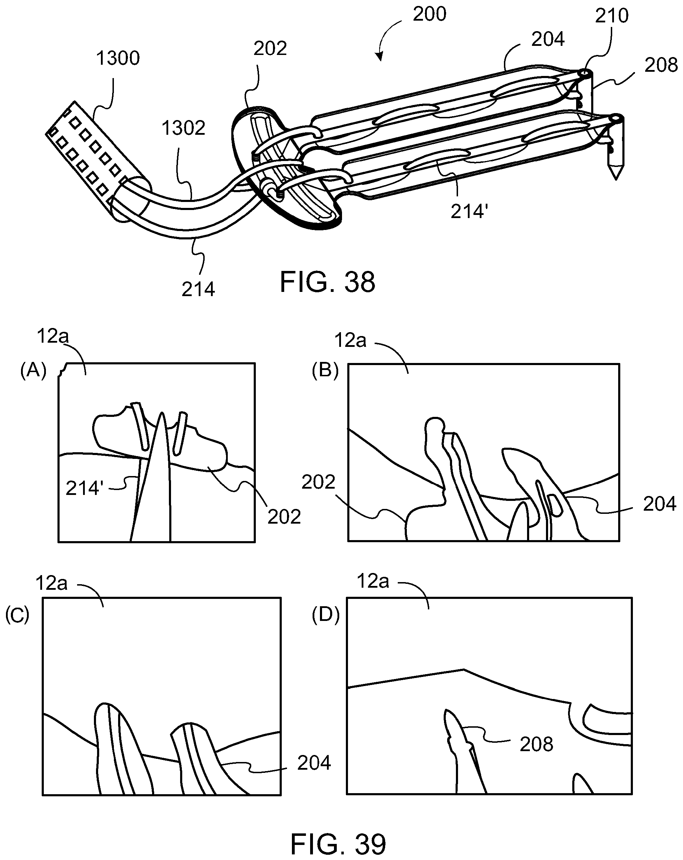

Figure 38 shows a retrieval mechanism for a soft tissue anchor 200 comprising a U-shaped fabric body 201. The retrieval mechanism comprises a retrieval shaft 1300 and a snare 1302. The retrieval shaft is threaded over the artificial line 214, and the snare 1302 is used to capture the fabric body 201 by ensnaring the base portion 202. The snare 1302 can be custom-made, or an off-the-shelf component. A fluorescent marker (not shown) is used to position the snare 1302. Once the base portion 202 is captured, tension in the tension line 214' is relaxed, such that the soft tissue anchor system 200 can be retrieved by pulling the base portion 202, thereby unfolding the arm portions 204 as they are pulled back through the leaflet 12.

Figures 39A to 39D illustrate the steps of retrieval of a prototype soft tissue anchor system 20. When no tension is applied via the tension line 214', the base portion 202 can be pulled away from the leaflet 12. The arm portions 204 are pulled back through the leaflet 12 as a result, and unfold as they do. The straightening out of the arm portions 204 results in the end caps 208 self-righting, such that they extend collinearly with the arm portions 204 and preferably perpendicular to the surface 12b of the leaflet 12. Finally, the end caps 208 pass back through the leaflet 12.

Figure 40 shows an alternative arrangement of an end cap 208 comprising a pointed tip 209. The end cap 208 comprises an outer tubular member 208a comprising an opening 211 formed in a wall thereof for receiving a tension line 214' and a notch 212 for facilitating attachment of the end cap 208 to an arm portion 204 of a fabric body 201. An inner tubular member 208b, defining an opening for receiving a wire guide member 218, is nested within the outer tubular member 208a.

The pointed tip 209 comprises a bevel plane 209a. The bevel plane 209a defines a face in which a bevel of the pointed tip 209 is formed. The pointed tip 209 also comprises two lancet planes 209b located symmetrically on either side of the bevel plane 209a. Such a configuration of the pointed tip 209 may reduce a force required to pierce the leaflet 12 during implantation of the soft tissue anchor system 200, thereby reducing trauma experienced during implantation.

Figure 41 shows a prototype soft tissue anchor system 200 in plan view. The end caps 208 comprise pointed tips 209, and the knot 215 used to fix the tension line 214' to the artificial line 215 is a fixed knot 215, preventing the tension line 214' from moving relative to the artificial line 215. The soft tissue anchor system 200 according to this embodiment is provided without a shape retention member 205 and a stiffening element 206, although in various embodiments such a soft tissue anchor system 200 may be provided with these features.

The tension line 214'is threaded through a hole 207' formed in the arm portion 204, a hole 207" formed in the narrow waist portion 203, and a hole 207‴ formed in the base portion, per arm portion 204. This arrangement may improve compression of the fabric body 201 around soft body tissue, since the placement of a hole 207" in the narrow waist portion 203, this hole 207" located towards the base portion 202, may encourage the collapse of the base portion 202 and the arm portions 204 towards the soft body tissue, thereby improving the stability of the soft tissue anchor system 200 upon implantation.

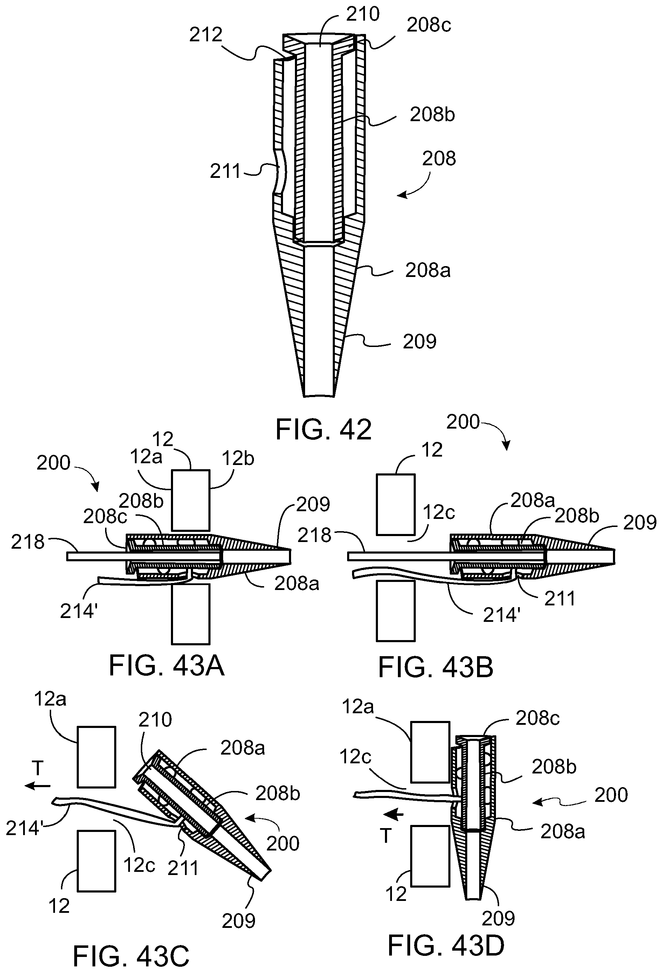

Figure 42 illustrates an alternative arrangement of an end cap 208. In this arrangement, the end cap 208 comprises an outer tubular member 208a comprising a pointed tip 209, an opening 211 for receiving a tension line 214', and a notch 212 for receiving an arm portion 204 of a fabric body 201. The end cap 208 also comprises an inner tubular member 208b nested within the outer tubular member 208a. The inner tubular member 208b comprises a flared inlet 208c defining an opening 210 for receiving a wire guide member 218.

The outer tubular member 208a is configured to retain the inner tubular member 208b via a press fit, in the present embodiment. An end of the inner tubular member 208b distal to the flared inlet 208c is configured to mate with a complementary notch of the outer tubular member 208a, such that the inner tubular member 208b is retained via an interference fit. Additionally, the flared inlet 208c of the inner tubular member 208b is arranged to abut an inner wall or surface of the outer tubular member 208a, thereby again reinforcing the press fit mating.

According to embodiments of the present invention, the aforementioned end caps 208 may be used as part of a soft tissue anchor system 200 in their own right. Where an end cap 208 is used as an anchor member in its own right, it may be regarded as a tubular cap member 208. Accordingly, soft tissue anchor systems 200 according to the present invention may not necessarily be provided with a U-shaped fabric body 201, or a fabric body at all.

Figures 43A to 43D illustrate a soft tissue anchor system 200 comprising a tubular cap member 208 and a tension line 214' during various steps of its implantation in a leaflet 12. Whilst the tubular cap member 208 illustrated in figures 43A to 43D, and as described herein, has a structure as described in relation to figure 42, it will be readily appreciated that the general principles and teachings of the implantation of the tubular cap member 208 could be applied to any of the aforementioned end caps 208 described above.

In the present embodiment, the soft tissue anchor system 200 comprises only a single tubular cap member 208 and a tension line 214', in combination with the tubular cap member 208. However, in other embodiments the tubular cap member 208 may be provided with a fabric body comprising an arm portion and a base portion. A plurality of tubular cap members 208 may also be used, as part of the same soft tissue anchor system, without deviating from the installation steps discussed in relation to figures 43A to 43D.

To implant the soft tissue anchor system 200 in the leaflet 12, a wire guide member 218 engages the tubular cap member 208 via the opening 210. The wire guide member 218 is then used to push the tubular cap member 208 into the leaflet 12, such that the tubular cap member 208 pierces the leaflet 12, as shown in figure 43A.

The wire guide member 218 continues to apply a motive force to the tubular cap member 208, such that the tubular cap member 208 completely passes through the leaflet 12, as shown in figure 43B.

The wire guide member 218 is subsequently withdrawn, leaving the tubular cap member 208 suspended on the opposite side of the leaflet 12 from where it was implanted (see figure 43C). The tension line 214' is left extending through the implantation site 12c.

Finally, a tension force T is applied to the tension line 214', this tension force applied in a direction away from the surface 12a into which the tubular cap member 208 was implanted. The application of this tension force T results in the tubular cap member 208 extending in a plane parallel to a surface of the leaflet 12b and, in the present embodiment, directly abutting this surface 12b, as shown in figure 43D.

As shown in figure 42 and figures 43A to 43D, the opening 211 from which the tension line 214' extends is located closer towards the opening 210 for receiving the wire guide member 218, rather than towards the tip 209 of the tubular cap member 208. Towards the tip 209, the tubular cap member 208 has a smaller projected area due to being narrower. Accordingly, by placing the tension line 214' towards the opening 210 (where the tubular cap member 208 has a larger projected area due to being wider), the tubular cap member 208 may be able to contact the surface 12b of the leaflet 12 with an equal projected area on either side of the tension line 214', resulting in a more stable abutment of the tubular cap member 208 with the leaflet surface 12b.

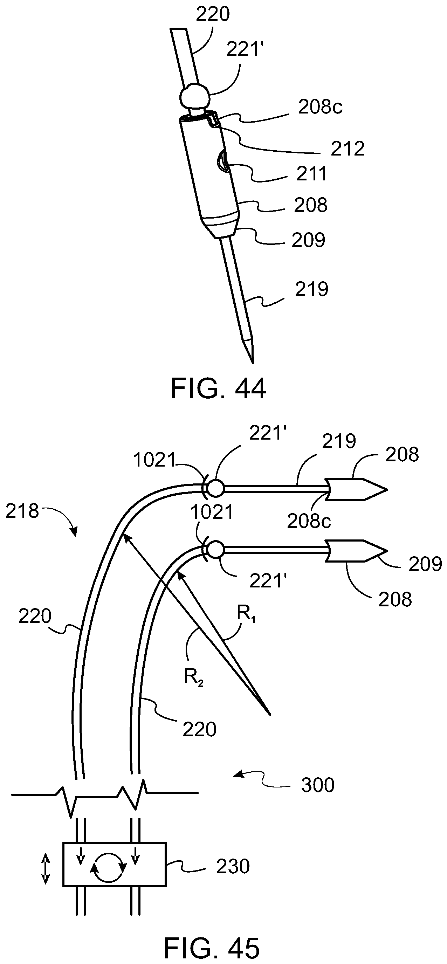

Figure 44 shows a wire guide member 218 for implanting an anchor member, according to embodiments of the invention, being received by an anchor member. In the illustrated embodiment, the anchor member is a tubular cap member 208. However, the anchor member could alternatively be an end cap 208 of an arm portion 204. The wire guide member 218 comprises a guide section which, in the presently illustrated embodiment is a piercing section 219, and a control section 220. In other embodiments, however, the guide section could be simply a thinner wire section configured to be received by a tubular cap member 208 having a pointed tip 209 for piercing soft tissue. The control section 220 is thicker than the piercing section 219. A bulge portion 221' is provided between the control section 220 and the piercing section 219. The bulge portion 221' is wider in diameter than both the control section 220 and the piercing section 219.

The bulge portion 221' is configured to engage the flared inlet 208c of the tubular cap member 208, such that the wire guide member 218 can provide a motive force to the tubular cap member 208 during implantation of the soft tissue anchor system 200. The bulge portion 221' is also configured to engage a complementary abutment portion 1021 located in a catheter device for implanting the soft tissue anchor system 200, as discussed below in relation to figure 45.

Figure 45 schematically represents a deployment system 300 for implanting a soft tissue anchor system 200 in a leaflet 12. The deployment system 300 belongs to a catheter device 2, such as a catheter device of the form discussed in relation to any of figures 1 to 24. The soft tissue anchor system 200 comprises multiple anchor members which, in the presently illustrated embodiment, are end caps 208 of a soft tissue anchor system 200 comprising a U-shaped fabric body 201. In other embodiments, however, they could be tubular cap members 208 as described in relation to figures 43A to 43D.

During delivery of the catheter device to the implantation site, such as a leaflet 12, the wire guide members 218 may experience a curvature due to curvature of the delivery shaft of the catheter device. A first wire guide member 218 located towards the inside of this curvature will experience a first radius of curvature R₁. A second wire guide member 218 located towards the outside of this curvature will experience a second, different, radius of curvature R₂. The second radius of curvature R₂ is greater than the first radius of curvature R₁. Accordingly, the second wire guide member 218 has to traverse a greater distance than the first wire guide member 218 to reach its respective end cap 208.

In preferred embodiments of the disclosure each end cap 208 of a single fabric body 201 will be simultaneously implanted in the soft body tissue 12. However, if one wire guide member 218 traverses a greater distance than the other, the end caps 208 may not necessarily be implanted in the soft body tissue 12 at the same time. Thus, in the presently illustrated embodiment, the catheter device comprises a deployment system 300 configured to simultaneously deploy each of the end caps 208 in the leaflet 12.

The deployment system 300 comprises the plurality of wire guide members 218 and the abutment portion 1021. The abutment portion 1021 acts to prevent each wire guide member 218 from being withdrawn into the catheter device past a certain distance, and therefore acts to provide an obstruction limiting a translation of the wire guide members 218 in a proximal direction, by abutting the bulge portion 221' when the wire guide members 218 are withdrawn proximally (i.e. into the delivery shaft).

The abutment portions 1021 are located in the proximal part 1004 of the housing of the catheter device. Accordingly, they are located after any possible deflection of the wire guide members 218 within the delivery shaft and are therefore capable of restoring a coplanar alignment of the wire guide members 218.

The deployment system 300 of the present embodiment also comprises a spring arrangement 230. The spring arrangement 230 is located in a delivery handle of the catheter device in the present embodiment, although it could be located elsewhere in other embodiments. The spring arrangement 230 is configured to bias the wire guide members 218 towards the delivery shaft/in a proximal direction. Accordingly, the spring arrangement 230 biases the bulge portions 221' towards the abutment portions 1021.

With the wire guide members 218 in coplanar alignment at a distal end (i.e. an end engaging the end cap members 208) regardless of a deflection of the wire guide members 218 within the delivery shaft, the wire guide members 218 can be simultaneously operated by the same translational input to achieve simultaneous implantation of the end caps 208 they respectively engage.

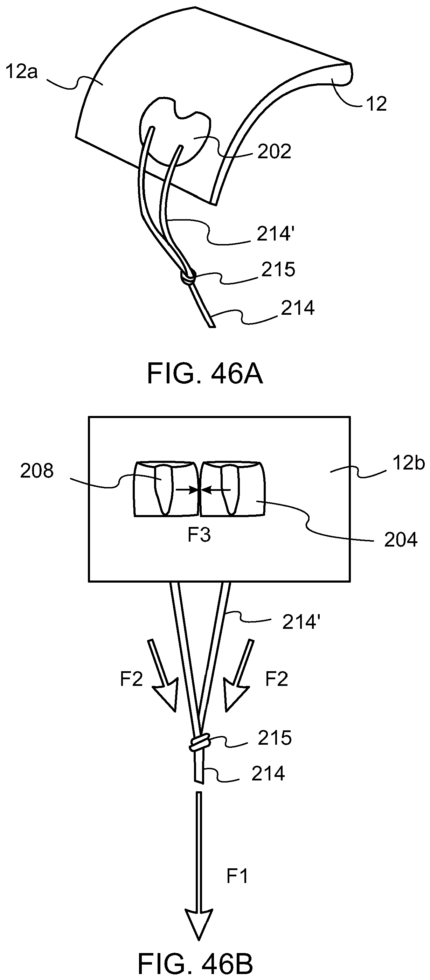

Figure 46A shows a soft tissue anchor system 200 implanted in a mitral valve leaflet 12 of the heart. The soft tissue anchor system 200 is implanted from the atrial side, such that the base portion 202 of the soft tissue anchor system 200 is adjacent an atrial surface of the leaflet 12. Accordingly, the line 214, 214' of the soft tissue anchor system 200 descends over the free edge of the leaflet 12. The line 214, 214 can provide support to the leaflet edge in this configuration.

Figure 46B shows the soft tissue anchor system 200 from the ventricular side of the leaflet 12. The arm portions 204 are adjacent to the ventricular surface 12b of the leaflet 12, with the end caps 208 lying in a plane parallel to the ventricular surface 12b due to the line 214' being placed under tension.

Figure 46B illustrates the tensile forces acting on the line 214, 214' in further detail. The tensile line 214' can be regarded to comprise a plurality of bridle lines. Each bridle line 214' is threaded through a respective arm portion 204, and is connected to a respective end cap 208. Each bridle line 214' is then connected in a fixed manner at a common bridle point 215 which, as illustrated in figure 46B, is the fixed knot 215. The artificial line 214 is also connected to the bridle point 215, and extends in a direction away from the leaflet 12.

A single tensile force F1 is applied to the artificial line 214. For ease of explanation, the tensile force F1 can be regarded as applying a tensile force in a single vertical direction. Application of the tensile force F1 places the bridle point 215 under tension. Since the bridle point 215 is connected to a plurality of bridle lines 214', this tensile force is also applied to each of the bridle lines 214'. However, since the bridle point 215 provides a common location from which each of the bridle lines 214' fork or bifurcate, the resulting tensile force F2 acting on each of the bridle lines 214' has a horizontal component as well as a vertical component. This is the result of the bridle point 215 distributing the tensile force F1 across each of the bridle lines 214', and as a result of the overall Y-shaped configuration of the artificial line 214 and the tensile line 214'.

As a result of the tensile force F2 having a tensile force component in a direction perpendicular to the single tensile force F1 applied to the bridle point 215, the arm portions 204 and end caps 208 experience a tensile force F3 acting in a horizontal direction. This tensile force F3 acts to draw the arm portions 204 and the end caps 208 towards the plane intersecting both the bridle point 215 and the direction in which the single tensile force F1 is applied. Accordingly, the arm portions 204 and the end caps 208 may act to draw the tissue of the leaflet 12 together in a pinched manner. The overall action of tensile forces F3 may accordingly 'capture' excess tissue of the leaflet 12 located between the arm portions 204 and end caps 208, thereby restoring the shape of the leaflet 12 and/or providing additional structural support to the leaflet structure 12.

The reshaping of the tissue of the leaflet 12 resulting from capturing excess leaflet tissue as described above has been found to provide an outcome similar to resection of the leaflet. Resection is a common surgical step in existing methods of heart valve repair, in which a surgeon resects a damaged section of the heart valve and stitches the edges of the remaining tissue together. Resection is often performed where there is excessive leaflet tissue present. However, by providing a line arrangement comprising a bridle point 215 and a plurality of bridle lines 214', the need for a resection step may be obviated. This could simplify the overall surgical procedure.

The design of the fabric body 201 of the soft tissue anchor system 200 can be altered prior to fabrication, e.g. by adjusting the dimensions of the base portion 202 and the arm portions 204, or by adjusting the number of arm portions 204 and respective end caps 208 included in the design, to facilitate the capture of a desired amount of leaflet tissue and hence provide a suitable 'resection' effect.

## Claims

1. A soft tissue anchor system (200) for implantation in soft body tissue (12) to hold an artificial line (214), the anchor comprising:
a fabric body (201) comprising a base portion (202) and at least two arm portions (204) extending from the base portion (202);
**characterised in that**:
the fabric body is a U-shaped fabric body (201); and
each arm portion (204) is configured to collapse in folds towards the base portion (202) such that, in use, the body tissue (12) is sandwiched between the base portion (202) and each of the arm portions (204).

2. A soft tissue anchor system (200) as claimed in claim 1, comprising a tension line (214') threaded through the arm portions (204) and the base portion (202);
wherein the tension line (214') is configured to collapse each arm portion (204) in folds towards the base portion (202) when a tensile force is applied to the tension line (214').

3. A soft tissue anchor system (200) as claimed in claim 2, wherein at least one of:
the tension line (214') is fixed to each arm portion (204) at an end of the arm portion (204) distal to the base portion (202); and/or
the tension line (214') is threaded no more than four times through the arm portion (204) and the base portion (202); and/or
the tension line (214') comprises a plurality of bridle lines, wherein each bridle line is associated with a respective arm portion (204), wherein each bridle line is connected to a common bridle point (215), and wherein the bridle point (215) is configured to place each bridle line under tension when a tensile force is applied to the bridle point (215).

4. A soft tissue anchor system (200) as claimed in claim 2 or 3, comprising:
the artificial line (214);
wherein the artificial line (214) is configured to apply the tensile force.

5. A soft tissue anchor system (200) as claimed in any preceding claim, wherein each arm portion (204) comprises an end cap (208) fixed at an end of each arm portion (204) distal to the base portion (202);
wherein each end cap (208) comprises an opening (210) configured to engage a wire guide member (218) for implanting the U-shaped fabric body (201) in the body tissue (12).

6. A soft tissue anchor system (200) as claimed in claim 5, wherein each end cap (208) comprises an outer tubular member (208a) and an inner tubular member (208b), wherein the outer tubular member (208a) is configured to receive the inner tubular member (208b); and
wherein the inner tubular member (208b) defines the opening (210) configured to engage the wire guide member (210).

7. A soft tissue anchor system (200) as claimed in claim 5 or 6, wherein the inner tubular member (208b) comprises a flared inlet (208c) defining the opening (210) configured to engage the wire guide member (210);
optionally wherein the flared inlet (208c) is configured to mate with a corresponding portion (221) of the wire guide member (218).

8. A soft tissue anchor system (200) as claimed in any of claims 5, 6 or 7, wherein the outer tubular member (208a) comprise a side wall and an aperture (211) formed in the side wall, wherein the aperture (211) receives the tension line (214');
and/or
wherein an end of the tension line (214') is fixed to each end cap (208) between the outer tubular member (208a) and the inner tubular member (208b).

9. A soft tissue anchor system (200) as claimed in any of claims 5 to 8, wherein a tip (209) of the end cap (208) distal to the base portion (202) is a pointed tip configured to pierce the body tissue (12);
optionally wherein the pointed tip comprises a bevel plane (209a).

10. A soft tissue anchor system (200) as claimed in any of claims 5 to 9, wherein each end cap (208) is configured to extend collinearly with a respective arm portion (204) during implantation and/or retrieval of the U-shaped fabric body (201); and
wherein each end cap (208) is configured to extend parallel to a plane of each fold of the respective arm portion (204) when a tensile force is applied to the tension line (214').

11. A soft tissue anchor system (200) as claimed in any preceding claim, wherein the base portion (202) defines a maximum width of the U-shaped fabric body (201).

12. A soft tissue anchor system (200) as claimed in any preceding claim, wherein the U-shaped fabric body (201) comprises a narrow waist portion (203) extending between the base portion (202) and each arm portion (204).

13. A soft tissue anchor system (200) as claimed in any preceding claim, wherein the soft tissue anchor system is a leaflet anchor system for implantation in a heart valve leaflet (12) to hold an artificial chordae line (214).

14. A catheter device (2) for implanting a soft tissue anchor system (200) in heart tissue (12), the catheter device (2) comprising:
a housing section, wherein the housing section extends from a distal end of the catheter device along the length of the catheter device toward a proximal end of the catheter device; and
a soft tissue anchor system (200) as claimed in any preceding claim located within the housing section.

15. A catheter device (2) as claimed in claim 14, wherein each arm portion (204) comprises an end cap (208) fixed at an end of each arm portion (204) distal to the base portion (202), and each end cap (208) comprises an opening (210) configured to engage a wire guide member (218) for implanting the U-shaped fabric body (201) in the body tissue (12), the catheter device (2) comprising:
a deployment system (300) configured to simultaneously implant each of the end caps (208) in the heart tissue (12);
wherein the deployment system (300) comprises a plurality of wire guide members (218), wherein each wire guide member (218) comprises a guide portion (219) located at a distal end of the wire guide members (218) and configured to engage a respective end cap (208); and
wherein the deployment system (300) is configured to maintain a coplanar alignment between the guide portions (219) of the plurality of wire guide members (218) during implantation of the end caps (208).

16. A catheter device (2) as claimed in claim 15, wherein each wire guide member (218) comprises a bulge portion (221'); and
wherein each wire guide member (218) is movable between a first configuration and a second configuration, wherein in the first configuration the bulge portion (221') is configured to engage a first obstruction (1021) configured to limit a translation of the wire guide member (218) in the proximal direction, and wherein in the second configuration the wire guide member (218) is configured to engage a respective end cap (208).

17. A catheter device (2) as claimed in claim 16, wherein the first obstruction (1021) is an abutment portion located in a proximal part of the housing section; and/or
wherein in the second configuration the bulge portion (221') is configured to abut a complementary portion of the end cap (208); and/or
wherein the deployment system (300) comprises a spring arrangement (230) configured to bias each wire guide member (218) towards being in the first configuration.

18. A catheter device (2) as claimed in any of claims 14 to 17, comprising:
a leaflet anchor for placement in a leaflet (12) of a heart valve, wherein the leaflet anchor is the soft tissue anchor system (200), wherein the leaflet anchor is arranged to be coupled to the artificial line (215); and
a leaflet anchor deployment mechanism for deploying the leaflet anchor to attach it to the leaflet of the heart.

19. A catheter device (2) as claimed in claim 18, comprising:
a mechanical gripper device (1006) for grasping the leaflet (12) of the heart valve; and
a leaflet anchor tube for housing the leaflet anchor (200) before deployment into the body tissue (12);
wherein the gripper device (1006) and the leaflet anchor (200) are arranged such that when, in use, the gripper device (1006) grasps the leaflet (12), the leaflet anchor system can be pushed out of the leaflet anchor tube to pierce the leaflet and deploy the anchor such that it is implanted in the leaflet;
optionally wherein the gripper device (1006) includes a gripper arm (1030) rotatably coupled to a main body of the catheter device (2) such that the gripper arm (1030) is configured to rotate relative to the catheter device (2) to move an outer end of the gripper arm (1030) away from the main body of the catheter device (2).

20. A method of manufacturing a soft tissue anchor system (200) as claimed in any of claims 1 to 13, the method comprising:
fabricating the U-shaped fabric body (201); and
configuring each arm portion (204) to collapse in folds;
optionally wherein the step of fabricating the U-shaped fabric body (201) comprises laser cutting a fabric.

## Patentansprüche

1. Weichgewebeankersystem (200) zur Implantation in weiches Körpergewebe (12), um eine künstliche Leine (214) zu halten, wobei der Anker Folgendes umfasst:
einen Gewebekörper (201) umfassend einen Basisteil (202) und mindestens zwei Armteile (204), die sich von dem Basisteil (202) aus erstrecken;
**dadurch gekennzeichnet, dass**:
der Gewebekörper ein U-förmiger Gewebekörper (201) ist; und
jeder Armteil (204) so konfiguriert ist, dass er in Falten zu dem Basisteil (202) hin zusammenfällt so dass das Körpergewebe (12) im Gebrauch zwischen dem Basisteil (202) und jedem der Armteile (204) eingeklemmt ist.

2. Weichgewebeankersystem (200) nach Anspruch 1, umfassend eine Spannleine (214'), die durch die Armteile (204) und den Basisteil (202) gefädelt ist;
wobei die Spannleine (214') so konfiguriert ist, dass sie jeden Armteil (204) in Falten zu dem Basisteil (202) hin zusammenfallen lässt, wenn eine Zugkraft auf die Spannleine (214') ausgeübt wird.

3. Weichgewebeankersystem (200) nach Anspruch 2, wobei mindestens eines der folgenden Merkmale vorliegt:
die Spannleine (214') ist an jedem Armteil (204) an einem Ende des Armteils (204) distal zu dem Basisteil (202) befestigt; und/oder
die Spannleine (214') ist nicht mehr als viermal durch den Armteil (204) und den Basisteil (202) gefädelt; und/oder
die Spannleine (214') umfasst eine Vielzahl von Verbindungsleinen, wobei jede Verbindungsleine mit einem entsprechenden Armteil (204) verbunden ist, wobei jede Verbindungsleine mit einem gemeinsamen Verbindungspunkt (215) verbunden ist, und wobei der Verbindungspunkt (215) so konfiguriert ist, dass jede Verbindungsleine unter Spannung gesetzt wird, wenn eine Zugkraft auf den Verbindungspunkt (215) ausgeübt wird.

4. Weichgewebeankersystem (200) nach Anspruch 2 oder 3, umfassend:
die künstliche Leine (214);
wobei die künstliche Leine (214) so konfiguriert ist, dass sie die Zugkraft aufbringt.

5. Weichgewebeankersystem (200) nach einem vorstehenden Anspruch, wobei jeder Armteil (204) eine Endkappe (208) umfasst, die an einem Ende jedes Armteils (204) distal zu dem Basisteil (202) befestigt ist;
wobei jede Endkappe (208) eine Öffnung (210) umfasst, die so konfiguriert ist, dass sie mit einem Drahtführungselement (218) in Eingriff kommt, um den U-förmigen Gewebekörper (201) in das Körpergewebe (12) zu implantieren.

6. Weichgewebeankersystem (200) nach Anspruch 5, wobei jede Endkappe (208) ein äußeres rohrförmiges Element (208a) und ein inneres rohrförmiges Element (208b) umfasst, wobei das äußere rohrförmige Element (208a) so konfiguriert ist, dass es das innere rohrförmige Element (208b) aufnimmt; und
wobei das innere rohrförmige Element (208b) die Öffnung (210) umgrenzt, die so konfiguriert ist, dass sie mit dem Drahtführungselement (210) in Eingriff kommt.

7. Weichgewebeankersystem (200) nach Anspruch 5 oder 6, wobei das innere rohrförmige Element (208b) einen aufgeweiteten Einlass (208c) umfasst, der die Öffnung (210) umgrenzt, die so konfiguriert ist, dass sie mit dem Drahtführungselement (210) in Eingriff kommt;
optional, wobei der aufgeweitete Einlass (208c) so konfiguriert ist, dass er mit einem entsprechenden Abschnitt (221) des Drahtführungselements (218) zusammenpasst.

8. Weichgewebeankersystem (200) nach einem der Ansprüche 5, 6 oder 7, wobei das äußere rohrförmige Element (208a) eine Seitenwand und eine in der Seitenwand ausgebildete Apertur (211) umfasst, wobei die Apertur (211) die Spannleine (214') aufnimmt;
und/oder
wobei ein Ende der Spannleine (214') an jeder Endkappe (208) zwischen dem äußeren rohrförmigen Element (208a) und dem inneren rohrförmigen Element (208b) befestigt ist.

9. Weichgewebeankersystem (200) nach einem der Ansprüche 5 bis 8, wobei eine Spitze (209) der Endkappe (208) distal zum Basisteil (202) eine spitze Spitze ist, die zum Durchstechen des Körpergewebes (12) konfiguriert ist;
optional, wobei die spitze Spitze eine schräge Ebene (209a) umfasst.

10. Weichgewebeankersystem (200) nach einem der Ansprüche 5 bis 9, wobei jede Endkappe (208) so konfiguriert ist, dass sie sich während der Implantation und/oder Entnahme des U-förmigen Gewebekörpers (201) kollinear mit einem entsprechenden Armteil (204) erstreckt; und
wobei jede Endkappe (208) so konfiguriert ist, dass sie sich parallel zu einer Ebene jeder Falte des jeweiligen Armteils (204) erstreckt, wenn eine Zugkraft auf die Spannleine (214') ausgeübt wird.

11. Weichgewebeankersystem (200) nach einem vorstehenden Anspruch, wobei der Basisteil (202) eine maximale Breite des U-förmigen Gewebekörpers (201) definiert.

12. Weichgewebeankersystem (200) nach einem vorstehenden Anspruch, wobei der U-förmige Gewebekörper (201) einen schmalen Taillenteil (203) umfasst, der sich zwischen dem Basisteil (202) und jedem Armteil (204) erstreckt.

13. Weichgewebeankersystem (200) nach einem vorstehenden Anspruch, wobei das Weichgewebeankersystem ein Segelankersystem zur Implantation in ein Herzklappensegel (12) ist, um eine künstliche Chordae-Leine (214) zu halten.

14. Kathetervorrichtung (2) zum Implantieren eines Weichgewebeankersystems (200) in Herzgewebe (12), wobei die Kathetervorrichtung (2) Folgendes umfasst:
einen Gehäuseabschnitt, wobei sich der Gehäuseabschnitt von einem distalen Ende der Kathetervorrichtung entlang der Länge der Kathetervorrichtung zu einem proximalen Ende der Kathetervorrichtung erstreckt; und
ein Weichgewebeankersystem (200) nach einem der vorstehenden Ansprüche, das sich in dem Gehäuseabschnitt befindet.

15. Kathetervorrichtung (2) nach Anspruch 14, wobei jeder Armteil (204) eine Endkappe (208) umfasst, die an einem Ende jedes Armteils (204) distal zu dem Basisteil(202) befestigt ist, und jede Endkappe (208) eine Öffnung (210) umfasst, die so konfiguriert ist, dass sie mit einem Drahtführungselement (218) in Eingriff kommt, um den U-förmigen Gewebekörper (201) in das Körpergewebe (12) zu implantieren, wobei die Kathetervorrichtung (2) Folgendes umfasst:
ein Entfaltungssystem (300), das zum gleichzeitigen Implantieren jeder der Endkappen (208) in das Herzgewebe (12) konfiguriert ist;
wobei das Entfaltungssystem (300) eine Vielzahl von Drahtführungselementen (218) umfasst, wobei jedes Drahtführungselement (218) einen Führungsabschnitt (219) umfasst, der sich an einem distalen Ende der Drahtführungselemente (218) befindet und so konfiguriert ist, dass er mit einer entsprechenden Endkappe (208) in Eingriff kommt; und
wobei das Entfaltungssystem (300) so konfiguriert ist, dass es während der Implantation der Endkappen (208) eine koplanare Ausrichtung zwischen den Führungsabschnitten (219) der Vielzahl von Drahtführungselementen (218) aufrechterhält.

16. Kathetervorrichtung (2) nach Anspruch 15, wobei jedes Drahtführungselement (218) einen Wölbungsabschnitt (221') umfasst; und
wobei jedes Drahtführungselement (218) zwischen einer ersten Konfiguration und einer zweiten Konfiguration beweglich ist, wobei in der ersten Konfiguration der Wölbungsabschnitt (221') so konfiguriert ist, dass er in ein erstes Hindernis (1021) eingreift, das so konfiguriert ist, dass es eine Translation des Drahtführungselements (218) in der proximalen Richtung begrenzt, und wobei in der zweiten Konfiguration das Drahtführungselement (218) so konfiguriert ist, dass es mit einer entsprechenden Endkappe (208) in Eingriff kommt.

17. Kathetervorrichtung (2) nach Anspruch 16, wobei das erste Hindernis (1021) ein Anschlagteil ist, das sich in einem proximalen Teil des Gehäuseabschnitts befindet; und/oder
wobei in der zweiten Konfiguration der Wölbungsabschnitt (221') so konfiguriert ist, dass er an einem komplementären Abschnitt der Endkappe (208) anliegt; und/oder
wobei das Entfaltungssystem (300) eine Federanordnung (230) umfasst, die so konfiguriert ist, dass jedes Drahtführungselement (218) in Richtung der ersten Konfiguration vorgespannt ist.

18. Kathetervorrichtung (2) nach einem der Ansprüche 14 bis 17, umfassend:
einen Segelanker zur Platzierung in einem Segel (12) einer Herzklappe, wobei es sich bei dem Segelanker um das Weichgewebeankersystem (200) handelt, wobei der Segelanker so angeordnet ist, dass er mit der künstlichen Leine (215) gekoppelt werden kann; und
einen Segelanker-Entfaltungsmechanismus zum Entfalten des Segelankers, um ihn an dem Segel des Herzes zu befestigen.

19. Kathetervorrichtung (2) nach Anspruch 18, umfassend:
eine mechanische Greifvorrichtung (1006) zum Ergreifen des Segels (12) der Herzklappe; und ein Segelankerrohr zur Aufnahme des Segelankers (200) vor dem Entfalten in das Körpergewebe (12);
wobei die Greifvorrichtung (1006) und der Segelanker (200) so angeordnet sind, dass, wenn die Greifvorrichtung (1006) bei der Verwendung das Segel (12) ergreift, das Segelankersystem aus dem Segelankerrohr herausgeschoben werden kann, um das Segel zu durchstechen und den Anker zu entfalten so dass es in dem Segel implantiert wird;
optional, wobei die Greifvorrichtung (1006) einen Greifarm (1030) einschließt, der drehbar mit einem Hauptkörper der Kathetervorrichtung (2) gekoppelt ist, so dass der Greifarm (1030) so konfiguriert ist, dass er sich relativ zu der Kathetervorrichtung (2) dreht, um ein äußeres Ende des Greifarms (1030) von dem Hauptkörper der Kathetervorrichtung (2) weg zu bewegen.

20. Verfahren zur Herstellung eines Weichgewebeankersystems (200) nach einem der Ansprüche 1 bis 13, wobei das Verfahren Folgendes umfasst:
Herstellen des U-förmigen Gewebekörpers (201); und
Konfigurieren jedes Armteil (204), so dass es in Falten zusammenfällt;
optional, wobei der Schritt der Herstellung des U-förmigen Gewebekörpers (201) das Laserschneiden eines Gewebes umfasst.

## Revendications

1. Système (200) d'ancrage de tissu mou pour implantation dans un tissu (12) corporel mou de façon à maintenir une ligne artificielle (214), l'ancrage comprenant :
un corps (201) en étoffe comprenant une partie de base (202) et au moins deux parties bras (204) s'étendant à partir de la partie de base (202) ;
**caractérisé en ce que** :
le corps en étoffe est un corps (201) en étoffe en forme de U ; et
chaque partie bras (204) est configurée de façon à se replier vers la partie de base (202) en formant des plis, de sorte que, lors de l'utilisation, le tissu (12) corporel est intercalé entre la partie de base (202) et chacune des parties bras (204).

2. Système (200) d'ancrage de tissu mou selon la revendication 1, comprenant une ligne de tension (214') enfilée à travers les parties bras (204) et la partie de base (202) ;
dans lequel la ligne de tension (214') est configurée pour replier chaque partie bras (204) en plis vers la partie de base (202) lorsqu'une force de traction est appliquée à la ligne de tension (214').

3. Système (200) d'ancrage de tissu mou selon la revendication 2, dans lequel au moins un parmi :
la ligne de tension (214') est fixée à chaque partie bras (204) au niveau d'une extrémité de la partie bras (204) distale à la partie de base (202) ; et/ou
la ligne de tension (214') n'est pas enfilée plus de quatre fois à travers la partie bras (204) et la partie de base (202) ; et/ou
la ligne de tension (214') comprend une pluralité de lignes de bride, dans lequel chaque ligne de bride est associée à une partie bras (204) respective, dans lequel chaque ligne de bride est reliée à un point de bride (215) commun, et dans lequel le point de bride (215) est configuré pour mettre chaque ligne de bride sous tension lorsqu'une force de traction est appliquée au point de bride (215).

4. Système (200) d'ancrage de tissu mou selon la revendication 2 ou la revendication 3, comprenant :
la ligne artificielle (214) ;
dans lequel la ligne artificielle (214) est configurée pour appliquer la force de traction.

5. Système (200) d'ancrage de tissu mou selon une quelconque revendication précédente, dans lequel chaque partie bras (204) comprend un capuchon d'extrémité (208) fixé au niveau d'une extrémité de chaque partie bras (204) distale à la partie de base (202) ;
dans lequel chaque capuchon d'extrémité (208) comprend une ouverture (210) configurée pour recevoir un organe (218) de guidage de fil pour implanter le corps (201) en étoffe en forme de U dans le tissu (12) corporel.

6. Système (200) d'ancrage de tissu mou selon la revendication 5, dans lequel chaque capuchon d'extrémité (208) comprend un organe tubulaire externe (208a) et un organe tubulaire interne (208b), dans lequel l'organe tubulaire externe (208a) est configuré pour recevoir l'organe tubulaire interne (208b) ; et
dans lequel l'organe tubulaire interne (208b) définit l'ouverture (210) configurée pour recevoir l'organe (210) de guidage de fil.

7. Système (200) d'ancrage de tissu mou selon la revendication 5 ou la revendication 6, dans lequel l'organe tubulaire interne (208b) comprend une entrée évasée (208c) définissant l'ouverture (210) configurée pour recevoir l'organe (210) de guidage de fil ;
facultativement dans lequel l'entrée évasée (208c) est configurée pour s'accoupler à une partie (221) correspondante de l'organe (218) de guidage de fil.

8. Système (200) d'ancrage de tissu mou selon l'une quelconque des revendications 5, 6 ou 7, dans lequel l'organe tubulaire externe (208a) comprend une paroi latérale et un orifice (211) formé dans la paroi latérale, dans lequel l'orifice (211) reçoit la ligne de tension (214') ;
et/ou
dans lequel une extrémité de la ligne de tension (214') est fixée à chaque capuchon d'extrémité (208) entre l'organe tubulaire externe (208a) et l'organe tubulaire interne (208b).

9. Système (200) d'ancrage de tissu mou selon l'une quelconque des revendications 5 à 8, dans lequel un embout (209) du capuchon d'extrémité (208) distal à la partie de base (202) est un embout pointu configuré pour percer le tissu (12) corporel ;
facultativement dans lequel l'embout pointu comprend un plan biseauté (209a).

10. Système (200) d'ancrage de tissu mou selon l'une quelconque des revendications 5 à 9, dans lequel chaque capuchon d'extrémité (208) est configuré pour s'étendre de manière colinéaire avec une partie bras (204) respective pendant l'implantation et/ou le retrait du corps (201) en étoffe en forme de U ; et
dans lequel chaque capuchon d'extrémité (208) est configuré pour s'étendre parallèlement à un plan de chaque pli de la partie bras (204) respective lorsqu'une force de traction est appliquée à la ligne de tension (214').

11. Système (200) d'ancrage de tissu mou selon une quelconque revendication précédente, dans lequel la partie de base (202) définit une largeur maximale du corps (201) en étoffe en forme de U.

12. Système (200) d'ancrage de tissu mou selon une quelconque revendication précédente, dans lequel le corps (201) en étoffe en forme de U comprend une partie de taille étroite (203) s'étendant entre la partie de base (202) et chaque partie bras (204).

13. Système (200) d'ancrage de tissu mou selon une quelconque revendication précédente, dans lequel le système d'ancrage de tissu mou est un système d'ancrage de feuillet pour implantation dans un feuillet (12) de valvule cardiaque pour maintenir une ligne de cordage artificielle (214).

14. Dispositif cathéter (2) pour implanter un système (200) d'ancrage de tissu mou dans du tissu (12) cardiaque, le dispositif cathéter (2) comprenant :
une section de logement, dans lequel la section de logement s'étend d'une extrémité distale du dispositif cathéter selon la longueur du dispositif cathéter vers une extrémité proximale du dispositif cathéter ; et
un système (200) d'ancrage de tissu mou selon une quelconque revendication précédente situé à l'intérieur de la section de logement.

15. Dispositif cathéter (2) selon la revendication 14, dans lequel chaque partie bras (204) comprend un capuchon d'extrémité (208) fixé au niveau d'une extrémité de chaque partie bras (204) distale à la partie de base (202), et chaque capuchon d'extrémité (208) comprend une ouverture (210) configurée pour recevoir un organe (218) de guidage de fil pour implanter le corps (201) en étoffe en forme de U dans le tissu (12) corporel, le dispositif cathéter (2) comprenant :
un système de déploiement (300) configuré pour implanter simultanément chacun des capuchons d'extrémité (208) dans le tissu (12) cardiaque ;
dans lequel le système de déploiement (300) comprend une pluralité d'organes (218) de guidage de fil, dans lequel chaque organe (218) de guidage de fil comprend une partie de guidage (219) située au niveau d'une extrémité distale des organes (218) de guidage de fil et configurée pour recevoir un capuchon d'extrémité (208) respectif ; et
dans lequel le système de déploiement (300) est configuré pour maintenir un alignement coplanaire entre les parties de guidage (219) de la pluralité d'organes (218) de guidage de fil pendant l'implantation des capuchons d'extrémité (208).

16. Dispositif cathéter (2) selon la revendication 15, dans lequel chaque organe (218) de guidage de fil comprend une partie de renflement (221') ; et
dans lequel chaque organe (218) de guidage de fil est mobile entre une première configuration et une deuxième configuration, dans lequel, dans la première configuration, la partie de renflement (221') est configurée pour venir en prise avec une première obstruction (1021) configurée pour limiter une translation de l'organe (218) de guidage de fil dans la direction proximale, et dans lequel, dans la deuxième configuration, l'organe (218) de guidage de fil est configuré pour recevoir un capuchon d'extrémité (208) respectif.

17. Dispositif cathéter (2) selon la revendication 16, dans lequel la première obstruction (1021) est une partie de butée située dans une partie proximale de la section de logement ; et/ou
dans lequel, dans la deuxième configuration, la partie de renflement (221') est configurée pour venir en butée contre une partie complémentaire du capuchon d'extrémité (208) ; et/ou
dans lequel le système de déploiement (300) comprend un agencement de ressort (230) configuré pour solliciter chaque organe (218) de guidage de fil pour qu'il soit dans la première configuration.

18. Dispositif cathéter (2) selon l'une quelconque des revendications 14 à 17, comprenant :
un ancrage de feuillet destiné à être placé dans un feuillet (12) d'une valvule cardiaque, dans lequel l'ancrage de feuillet est le système (200) d'ancrage de tissu mou, dans lequel l'ancrage de feuillet est agencé pour être couplé à la ligne artificielle (215) ; et
un mécanisme de déploiement d'ancrage de feuillet pour déployer l'ancrage de feuillet pour l'attacher au feuillet du cœur.

19. Dispositif cathéter (2) selon la revendication 18, comprenant :
un dispositif de préhension (1006) mécanique pour saisir le feuillet (12) de la valvule cardiaque ; et un tube d'ancrage de feuillet pour loger l'ancrage (200) de feuillet avant le déploiement dans le tissu (12) corporel ;
dans lequel le dispositif de préhension (1006) et l'ancrage (200) de feuillet sont agencés de sorte que, lors de l'utilisation, le dispositif de préhension (1006) saisit le feuillet (12), le système d'ancrage de feuillet peut être poussé hors du tube d'ancrage de feuillet pour percer le feuillet et déployer l'ancrage pour qu'il soit implanté dans le feuillet ;
facultativement dans lequel le dispositif de préhension (1006) inclut un bras de préhension (1030) couplé en rotation à un corps principal du dispositif cathéter (2) de sorte que le bras de préhension (1030) est configuré pour tourner par rapport au dispositif cathéter (2) pour mettre en mouvement une extrémité externe du bras de préhension (1030) afin de l'éloigner du corps principal du dispositif cathéter (2).

20. Procédé de fabrication d'un système (200) d'ancrage de tissu mou selon l'une quelconque des revendications 1 à 13, le procédé comprenant :
le fait de fabriquer le corps (201) en étoffe en forme de U ; et
le fait de configurer chaque partie bras (204) de façon qu'elle se replie en formant des plis ;
facultativement dans lequel l'étape de fabrication du corps (201) en étoffe en forme de U comprend le fait de découper une étoffe au laser.
